# EUROPÄISCHE PATENTSCHRIFT

(11) **EP 2 524 959 B1**
(45) Veröffentlichungstag und Bekanntmachung des Hinweises auf die Patenterteilung: **22.01.2014**
(21) Anmeldenummer: 11166440.5
(22) Anmeldetag: 17.05.2011
(51) Int. Cl.: C07D 317/12, C11B 9/00, A23L 1/226, A61K 8/49, A61Q 13/00, C07D 317/10, C07D 317/72, C11D 3/50, C11D 3/20

(54) **Riech- und/oder Aromastoffkompositionen enthaltend Dioxolane**
Dioxolanes containing olfactory and/or aromatic substances
Compositions d'odeurs et/ou d'arômes comprenant du dioxolane

(43) Veröffentlichungstag der Anmeldung: 21.11.2012
(73) Patentinhaber: Symrise AG, 37603 Holzminden (DE)
(72) Erfinder: Hölscher, Bernd, 37620, Halle (DE); Singer, Dr. Emilie, 37603, Holzminden (DE)
(74) Vertreter: Eisenführ Speiser

(56) Entgegenhaltungen:
- EP-A2- 0 033 928
- WO-A2-02/057227
- DE-A1- 2 541 438
- JP-A- 2004 176 026
- US-A- 3 055 766
- US-A- 4 381 243

## Beschreibung

Die vorliegende Erfindung betrifft primär die Verwendung von Dioxolanen der nachfolgend definierten Formel (I) als Riech- und/oder Aromastoff, bestimmte Riechstoff- und/oder Aromastoffkompositionen umfassend diese Dioxolane sowie entsprechende parfümierte und/oder aromatisierte Artikel.

Wegen der im Allgemeinen unzureichenden Verfügbarkeit vieler natürlicher Riechstoffkomponenten, der notwendigen Anpassung an wechselnde modische Geschmacksrichtungen sowie dem ständig steigenden Bedarf an neuen Riechstoffen, die allein oder in Form von Kompositionen wertvolle Duftstoffe bzw. Parfüms mit interessanten Duftnoten darstellen, besteht auch weiterhin ein Bedürfnis nach Verbindungen mit interessanten Riechstoffqualitäten.

Wegen der steigenden Nachfrage der Verbraucher nach neuen Duftnoten besteht der Parfümindustrie ein ständiger Bedarf an Riechstoffen, mit denen sich in Parfüms neuartige Effekte erzielen und auf diese Art neue Modetrends kreieren lassen.

Trotz einer Vielzahl bereits vorhandener Riechstoffe besteht in der Parfümindustrie auch weiterhin ein genereller Bedarf an neuen Riechstoffen, insbesondere an solchen, die über Ihre primären, nämlich geruchlichen, Eigenschaften hinaus zusätzliche positive Sekundäreigenschaften besitzen, wie z.B. eine höhere Stabilität unter bestimmten Anwendungsbedingungen, eine hohe Ausgiebigkeit, eine große Strahlungskraft, gute Diffusivität (d.h. eine gute Raumwirkung), Fülle, Kraft und/oder Natürlichkeit, geruchsverstärkende Eigenschaften oder aber auch bessere dermatologische Eigenschaften gegenüber Riechstoffen mit vergleichbaren primären geruchlichen Eigenschaften.

Es besteht daher in der Parfümindustrie grundsätzlich ein Bedarf an weiteren Riechstoffen, die sich zur Herstellung von Riechstoffkompositionen oder parfümierten Artikeln eignen. Insbesondere besteht ein Bedarf an Riechstoffen, die durch die oben erwähnten technischen Eigenschaften zu einem erhöhten Nutzen von Riechstoffkompositionen und Parfümölen führen.

WO 02/085294 beschreibt diverse Acetale zur Blockierung bzw. Reduzierung des unangenehmen Geruchs bestimmter Säuren. Die dort offenbarten Acetale können gemäß WO 02/085294 in verschiedene Zusammensetzungen eingesetzt werden, unter anderem im Bereich der Körperpflege und in Reinigungsmitteln.

In DE 25 41 438 sind Dioxolanaldehyd-Riechstoffe beschrieben, die dort durch Hydroformylierung der entsprechenden Dioxolane erhalten wurden. Die diversen in DE 25 41 438 offenbarten Dioxolane dienten dort als Edukte zur Herstellung der Dioxolanaldehyd-Riechstoffe. Das einzige in DE 25 41 438 geruchlich beschriebene Dioxolan ist 2-Hexyl-4-vinyl-1,3-dioxolan, welches gemäß DE 25 41 438 einen fruchtigen Geruch von Ananas aufweist.

EP 1 992 606 A1 offenbart bestimmte Alkenacetale als Riechstoffe. Die in EP 1 992 606 A1 offenbarten Riechstoffe leiten sich von 4-Pentenalen ab, und weisen insbesondere eine 2,3,3-Trimethyl-4-pentenyl-Strukturelement auf.

Die primäre Aufgabe bestand nun darin, Riech- und/oder Aromastoffe zu finden, die ein interessantes sensorisches Profil aufweisen und sich als Riechstoffe für den Einsatz in der Parfümerie bzw. als Aromastoffe zur Aromatisierung von verzehrbaren Zubereitungen eignen, ohne dabei selbst eine Ananas-Note zu vermitteln. Es werden folglich im Rahmen der vorliegenden Erfindung keine Riechstoffe gesucht, die ausschließlich oder sehr stark nach Ananas riechen bzw. eine Ananas-Note vermitteln.

Darüberhinaus sollten diese Riech- und/oder Aromastoffe vorzugsweise positive Sekundäreigenschaften besitzen, vorzugsweise geruchsverstärkende Eigenschaften und/oder die Fähigkeit besitzen, geruchs- und/oder geschmacksverstärkende Eigenschaften aufzuweisen und/oder in Kombination mit anderen Riech- und/oder Aromastoffen deren Natürlichkeit, Frische, (Mund)Fülle, (Strahl)Kraft und/oder Ausstrahlung abzurunden bzw. zu erhöhen und/oder die Diffusivität einer Riechstoffmischung zu erhöhen.

Diese Aufgabe wurde gelöst durch die Verwendung einer Verbindung gemäß Formel (I) 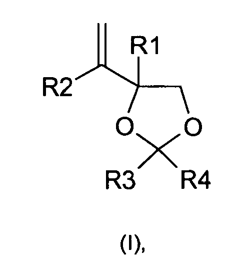 einschließlich der Stereoisomeren, wobei
R1 und R2 jeweils unabhängig voneinander entweder Wasserstoff oder Methyl, vorzugsweise Wasserstoff ist, und
a) R3 und R4 - zusammen mit dem Ketal- Kohlenstoff-Atom des Dioxolanringes - einen Ring mit insgesamt 5, 6, 7 oder 8 Ring-Kohlenstoff-Atomen bilden, wobei der Ring optional eine oder zwei Doppelbindungen und/oder optional 1 bis maximal 3 Sauerstoffatome enthält, und wobei der Ring optional substituiert ist mit einer oder mehreren verzweigten oder unverzweigten, verbrückten oder unverbrückten, Alkylgruppen, Alkenylgruppen, Cycloalkylgruppen, Cycloalkenylgruppen, Arylgruppen, Arylalkylgruppen, Alkoxyalkylgruppen oder Alkoxyarylgruppen, und wobei R3 und R4 zusammen insgesamt 3 bis 30 Kohlenstoff-Atome umfassen,
   oder
b) R3 entweder Wasserstoff oder ein organischer Rest mit 1 bis 15 Kohlenstoff-Atomen ist, wobei der organische Rest optional 1 bis maximal 3 Sauerstoff-Atome enthält, vorzugsweise R3 entweder Wasserstoff oder ein gesättigtes oder ungesättigtes, aromatisches oder aliphatisches, verzweigtes oder unverzweigtes, cyclisches oder lineares, verbrücktes oder unverbrücktes Strukturelement mit 1 bis 15 Kohlenstoff-Atomen ist, wobei das Strukturelement optional 1 bis maximal 3 Sauerstoff-Atome enthält und das Strukturelement optional substituiert ist mit 1 bis maximal 3 Alkyl- und/oder Alkenylgruppen und/oder mit einer oder zwei Hydroxygruppen,
   und
R4 ein organischer Rest mit 2 bis 15 Kohlenstoff-Atomen ist, wobei der organische Rest optional 1 bis maximal 3 Sauerstoff-Atome enthält, vorzugsweise R4 ein gesättigtes oder ungesättigtes, aromatisches oder aliphatisches, verzweigtes oder unverzweigtes, cyclisches oder lineares, verbrücktes oder unverbrücktes Strukturelement mit 2 bis 15 Kohlenstoff-Atomen ist, wobei das Strukturelement optional 1 bis maximal 3 Sauerstoff-Atome enthält und das Strukturelement optional substituiert ist mit 1 bis maximal 3 Alkyl- und/oder Alkenylgruppen und/oder mit einer oder zwei Hydroxygruppen
und wobei wenigstens eine der folgenden Bedingungen gilt:
i. sofern R4 ein verzweigter oder unverzweigter Alkyl-Rest mit 4 bis 15 Kohlenstoff-Atomen mit optional ein, zwei oder mehreren Doppelbindungen ist, an Position 3 zu Position 4 von R4 eine Kohlenstoff-Kohlenstoff-Einfachbindung vorliegt
   oder
ii. sofern R4 ein verzweigter oder unverzweigter Alkyl-Rest mit 2 bis 15 Kohlenstoff-Atomen mit optional ein, zwei oder mehreren Doppelbindungen ist, R3 nicht Wasserstoff ist,
als Riech- und/oder Aromastoff zum Vermitteln, Verstärken und/oder Modifizieren einer, zweier oder mehrerer Geruchs- und/oder Geschmacksnoten, wobei keine Geruchs- und/oder Geschmacksnote Ananas vermittelt wird.

Zur Klarstellung: die Bedingung i. bedeutet, dass zwischen den beiden Kohlenstoffatomen an den Positionen 3 und 4 von R4 insbesondere keine Kohlenstoff-Kohlenstoff-Doppelbindung vorliegt.

Bevorzugt sind Verbindungen der Formel (I), wobei
R1 und R2 jeweils unabhängig voneinander entweder Wasserstoff oder Methyl, vorzugsweise Wasserstoff ist, und
a) R3 und R4 zusammen ein cyclisches Alkylsystem mit 5, 6, 7 oder 8 Kohlenstoff-Atomen ergeben, das optional substituiert ist mit einem oder mehreren verzweigten oder unverzweigten Alkyl-Resten ausgewählt aus der Gruppe bestehend aus Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, und wobei das Alkylsystem optional eine oder zwei Doppelbindungen enthält,
   oder
b) R3 entweder Wasserstoff oder ein verzweigter oder unverzweigter Alkyl-Rest mit 1 bis 15 Kohlenstoff-Atomen und optional ein, zwei oder mehreren Doppelbindungen oder ein cyclisches Alkylsystem mit 5, 6 oder 7 Kohlenstoff-Atomen mit optional ein, zwei oder mehreren Doppelbindungen ist, wobei der Alkyl-Rest vorzugsweise ausgewählt ist aus der Gruppe bestehend aus Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl,
   und
   1. R4 ein Aromat ist, wobei der Aromat optional mit einem, zwei oder mehreren Alkyl-Resten mit 1 bis 3 Kohlenstoff-Atomen und/oder einem, zwei oder mehreren Hydroxy-Resten und/oder einem, zwei oder mehreren Alkoxy-Resten substituiert ist,
      oder
   2. R4 ein mono-, bi- oder tricyclisches Alkylsystem ist, das optional mit einem, zwei oder mehreren Alkyl-Resten, Hydroxy-Resten oder Alkoxy-Resten substituiert ist, wobei die Alkyl-Reste vorzugsweise ausgewählt sind aus der Gruppe bestehend aus Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl,
      oder
   3. R4 ein verzweigter oder unverzweigter Alkyl-Rest mit 2 bis 15 Kohlenstoff-Atomen mit ein, zwei oder mehreren Doppelbindungen oder ein Ringsystem mit 5, 6 oder 7 Kohlenstoff-Atomen mit ein, zwei oder mehreren Doppelbindungen, ist,
      und wobei wenigstens eine der folgenden Bedingungen gilt:
      i. sofern R4 ein verzweigter oder unverzweigter Alkyl-Rest mit 4 bis 15 Kohlenstoff-Atomen mit optional ein, zwei oder mehreren Doppelbindungen ist, an Position 3 zu Position 4 von R4 eine Kohlenstoff-Kohlenstoff-Einfachbindung vorliegt
         oder
      ii. sofern R4 ein verzweigter oder unverzweigter Alkyl-Rest mit 2 bis 15 Kohlenstoff-Atomen mit optional ein, zwei oder mehreren Doppelbindungen ist, R3 ein verzweigter oder unverzweigter Alkyl-Rest mit 1 bis 15 Kohlenstoff-Atomen und optional ein, zwei oder mehreren Doppelbindungen ist.

Weiter bevorzugte Verbindungen der Formel (I) sind solche, in denen R1 = H und R2 = H ist. Weiter bevorzugte Verbindungen der Formel (I) entsprechen damit der Formel (I-A): 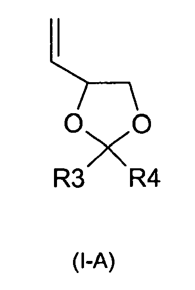 wobei in der Formel (I-A) R3 und R4 die oben bezüglich Formel (I) genannte (bevorzugte) Bedeutung haben.

Erfindungsgemäß bevorzugt ist eine Verwendung, wobei für die Verbindung gemäß Formel (I) bzw. (I-A) nur die Bedingung i. vorliegt.

Erfindungsgemäß bevorzugt ist eine Verwendung, wobei für die Verbindung gemäß Formel (I) bzw. (I-A) nur die Bedingung ii. vorliegt.

Erfindungsgemäß bevorzugt ist eine Verwendung, wobei für die Verbindung gemäß Formel (I) bzw. (I-A) beide Bedingungen i. und ii. vorliegen.

Erfindungsgemäß bevorzugte Verbindungen der Formel (I) bzw. (I-A) sind die Verbindungen der Formel (II): 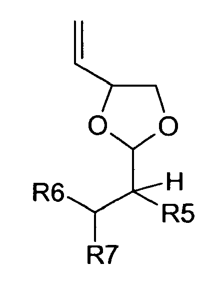 (II), einschließlich der Stereoisomeren,
wobei in der Formel (II):
R5 und R6 jeweils unabhängig voneinander entweder Wasserstoff oder Methyl ist,
R7 ein organischer Rest mit 1 bis 10 Kohlenstoff-Atomen ist, wobei der organische Rest optional 1 bis maximal 3 Sauerstoff-Atome enthält, vorzugsweise R7 ein gesättigtes oder ungesättigtes, aromatisches oder aliphatisches, verzweigtes oder unverzweigtes, cyclisches oder lineares, verbrücktes oder unverbrücktes Strukturelement mit 1 bis 10 Kohlenstoff-Atomen ist, wobei das Strukturelement optional 1 bis maximal 3 Sauerstoff-Atome enthält und das Strukturelement optional substituiert ist mit 1 bis maximal 3 Alkyl- und/oder Alkenylgruppen und/oder mit einer Hydroxygruppe, und
wobei sofern R7 ein verzweigter oder unverzweigter Alkyl-Rest mit 1 bis 10 Kohlenstoff-Atomen ist, an Position 1 zu Position 2 von R7 eine Kohlenstoff-Kohlenstoff-Einfachbindung vorliegt.

Erfindungsgemäß bevorzugte Verbindungen der Formel (II) sind solche, in denen
R5 und R6 jeweils unabhängig voneinander entweder Wasserstoff oder Methyl ist, und
R7 ein verzweigter oder unverzweigter Alkyl-Rest mit 1 bis 10 Kohlenstoff-Atomen, vorzugsweise ausgewählt aus der Gruppe bestehend aus Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, ist,
oder
R7 ein Cyloalkyl-Rest mit 5, 6 oder 7 Kohlenstoff-Atomen ist, der optional substituiert ist mit einem oder mehreren Alkyl-Resten, ausgewählt aus der Gruppe bestehend aus Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, einem Hydroxy-Rest oder einem Alkoxy-Rest,
und
wobei sofern R7 ein verzweigter oder unverzweigter Alkyl-Rest mit 1 bis 10 Kohlenstoff-Atomen ist, an Position 1 zu Position 2 von R7 eine Kohlenstoff-Kohlenstoff-Einfachbindung vorliegt.

Zur Klarstellung: die Maßgabe bezüglich R7 bedeutet, dass zwischen den beiden Kohlenstoffatomen an den Positionen 1 und 2 von R7 insbesondere keine Kohlenstoff-Kohlenstoff-Doppelbindung vorliegt.

Die erfindungsgemäßen bzw. erfindungsgemäß einzusetzenden Verbindungen der Formel (I) können dabei, je nach Substitutionsmuster, als beliebiges Gemisch als beliebiges Gemisch verschiedener Stereoisomeren, vorliegen, insbesondere als beliebiges Gemisch von Diastereomeren, dabei insbesondere als cis- oder trans-Isomere, oder Enantiomeren, dabei insbesondere als Racemat.

Die Gesamtzahl der Kohlenstoffatome der Verbindungen der Formeln (I), (I-A) bzw. (II) beträgt jeweils vorzugsweise maximal 22, bevorzugt maximal 18, weiter bevorzugt maximal 16 und insbesondere bevorzugt maximal 14.

Die Verbindungen der Formel (I), (I-A) bzw. (II) eignen sich wegen ihrer olfaktorischen Eigenschaften vorzüglich für den Einsatz in Riech- und Aromastoffkompositionen. Die Verbindung kann mit einer Vielzahl weiterer Riech- oder Aromastoffe kombiniert in zahlreichen Produkten verwendet werden. Besonders vorteilhaft lässt sich die Verbindung mit anderen Riech- oder Aromastoffen in verschiedenen, unterschiedlichen Mengenverhältnissen zu neuartigen Riechoder Aromastoffkompositionen kombinieren.

Die erfindungsgemäßen bzw. erfindungsgemäß einzusetzenden Verbindungen der Formel (I) sind insbesondere zur Verwendung als Riech- und/oder Aromastoffe geeignet, die in Parfüms und/oder Aromen eingesetzt werden können. Zudem weisen die erfindungsgemäßen bzw. erfindungsgemäß einzusetzenden Verbindungen insbesondere in alkalischen und in oxidierenden Medien eine hohe, ganz hervorragende Stabilität auf. Insbesondere wegen dieser Eigenschaften eignen sich die Verbindungen der Formel (I) in hervorragendem Maße für die Verwendung als Riech- und/oder Aromastoffe, und zwar insbesondere, wenn sie in parfümierten oder aromatisierten Artikeln oder Zubereitungen eingesetzt werden, die einen pH > 7 besitzen und/oder oxidierend wirken.

Ferner zeigen die Verbindungen der Formel (I), (I-A) bzw. (II) bzw. Riechstoffmischungen umfassend diese Verbindungen eine ausgeprägte Diffusivität (Raumwirkung), wodurch sie besonders zur Einarbeitung in Produkte zur Raumluftverbesserung geeignet sind (vorzugsweise in flüssiger, gelartiger oder auf einem festen Träger aufgebrachter Form sowie Pumpsprays oder Aerosolsprays).

Ein weiterer Aspekt der vorliegenden Erfindung betrifft die Verwendung von Verbindungen der Formel (I), (I-A) bzw. (II) als Booster für Riech- oder Aromastoffe, insbesondere für Riech- oder Aromastoffe mit blumigen (dabei insbesondere rosig, indolig), ledrigen, fruchtigen, frischen, cremigen, holzigen, moosigen und/oder grünen Noten.

Als "Booster" (Enhancer) verstärken die Verbindungen der Formel (I), (I-A) bzw. (II) die Geruchs- oder Geschmacksnoten von Riech- oder Aromastoffen. Besonders ausgeprägt ist der sogenannte "Boost-Effekt" in Parfümölen, insbesondere Parfümölen mit blumigen (dabei insbesondere rosig, indolig), ledrigen, fruchtigen, frischen, cremigen, holzigen, moosigen und/oder grünen Noten.

Ein entsprechendes Verfahren zum Modifizieren und/oder Verstärken (Boosten) eines Geruchs und/oder Geschmacks mit einer, mehreren oder sämtlichen der Noten insbesondere Noten blumig (dabei insbesondere rosig, indolig), ledrig, fruchtig, frisch, cremig, holzig, moosig und/oder grün, umfasst den folgenden Schritt:
- Vermischen eines oder mehrerer Riech- oder Aromastoffe mit einer, mehreren oder sämtlichen der Noten blumig (dabei insbesondere rosig, indolig), ledrig, fruchtig, frisch, cremig, holzig, moosig und/oder grün, mit einer Menge an Verbindungen der Formel (I), (I-A) bzw. (II), die ausreicht, den Geruchs- und/oder Geschmackseindruck des bzw. der Riech- oder Aromastoffe, die eine oder mehrere der Noten blumig (dabei insbesondere rosig, indolig), ledrig, fruchtig, frisch, cremig, holzig, moosig und/oder grün verursachen, sensorisch zu modifizieren und/oder zu verstärken.

Die erfindungsgemäßen bzw. erfindungsgemäß zu verwendenden Verbindungen der Formel (I), (I-A) bzw. (II) können zudem bereits in geringen Dosierungen die Intensität der Riech- bzw. Aromastoffmischung verstärken und das Gesamtbild der Mischung geruchlich abrunden und können eingesetzt werden, um einer Riech- oder Aromastoffkomposition mehr Fülle oder Mundfülle, Frische, (Strahl)Kraft, Ausstrahlung, Glanz, Abrundung und/oder Natürlichkeit zu verleihen.

Sofern in Rahmen dieses Textes ein Widerspruch zwischen dem chemischen Namen und der dargestellten Strukturformel der Verbindungen der Formeln (I), (I-A) oder (II) auftreten sollten, gilt die dargestellte Strukturformel.

Besonders bevorzugte Verbindungen der Formel (I) sind
2-Isobutyl-4-vinyl-[1,3]dioxolan,
2-secButyl-4-vinyl-[1,3]dioxolan,
2-Isopropyl-4-vinyl-[1,3]dioxolan,
2-(3,5-Dimethylcyclohex-3-enyl)-4-vinyl-[1,3]dioxolan,
2-(2,4-Dimethylcyclohex-3-enyl)-4-vinyl-[1,3]dioxolan,
2-(2,6-Dimethylhept-5-enyl)-4-vinyl-[1,3]dioxolan,
2-Ethyl-2-(2-methylbutyl)-4-vinyl-[1,3]dioxolan,
2-(2,4,4-Trimethylpentyl)-4-vinyl-[1,3]dioxolan,
2-(3,5-Dimethylhex-4-enyl)-2-methyl-4-vinyl-[1,3]dioxolan,
2-Methyl-2-(4-methylpent-3-enyl)-4-vinyl-[1,3]dioxolan,
8-tertButyl-2-vinyl-1,4-dioxaspiro-[4.5]-decan,
2-(2,6-Dimethylhepta-1,5-dienyl-4-vinyl-[1,3]dioxolan,
2-[2-(4-Methylcyclohex-3-enyl)-propyl]-4-vinyl-[1,3]dioxolan,
2-Phenyl-4-vinyl-[1,3]dioxolan,
2-Cyclohexyl-4-vinyl-[1,3]dioxolan,
2-(2,2,3-Trimethylcyclopent-3-enylmethyl)-4-vinyl-[1,3]dioxolan,
2-Vinyl-1,4-dioxaspiro[4.5]decan,
7-Methyl-2-vinyl-1,4-dioxaspiro[4.5]decan,
2-(2-Methylpropenyl)-4-vinyl-[1,3]dioxolan,
2-Methyl-2-(3-methylbutyl)-4-vinyl-[1,3]dioxolan.

Ganz besonders bevorzugte Verbindungen der Formel (I) sind
2-Isobutyl-4-vinyl-[1,3]dioxolan,
2-(3,5-Dimethylcyclohex-3-enyl)-4-vinyl-[1,3]dioxolan,
2-(2,4-Dimethylcyclohex-3-enyl)-4-vinyl-[1,3]dioxolan,
2-(2,6-Dimethylhept-5-enyl)-4-vinyl-[1,3]dioxolan,
2-Vinyl-1,4-dioxaspiro[4.5]decan,
7-Methyl-2-vinyl-1,4-dioxaspiro[4.5]decan,
2-(2-Methylpropenyl)-4-vinyl-[1,3]dioxolan, und
2-Methyl-2-(3-methylbutyl)-4-vinyl-[1,3]dioxolan.

Die am meisten bevorzugte Verbindung der Formel (I) ist 2-Isobutyl-4-vinyl-[1,3]dioxolan, da diese Verbindung ein bislang wohl einzigartiges Geruchsprofil aufweist und darüberhinaus die im Rahmen der vorliegenden Erfindung beschriebenen Effekte in besonders ausgeprägtem Maße zeigt.

Die erfindungsgemäßen bzw. erfindungsgemäß einzusetzenden Verbindungen der Formel (I), (I-A) und (II) liegen -je nach konkreter Bedeutung der Reste R1 bis R4 bzw. R5 bis R7 - können als cis- oder trans-Isomere vorliegen. Auch wenn sich in eigenen Untersuchungen im Rahmen der vorliegenden Erfindung gezeigt hat, dass sich die jeweiligen cis- bzw. trans-Isomere geruchlich etwas unterscheiden, ist es bezüglich der sensorischen Eigenschaften im Allgemeinen nicht nachteilig, Isomerengemische der Verbindungen der Formel (I), (I-A) und (II) einzusetzen.

Bei der Herstellung fallen die Verbindungen der Formel (I), (I-A) bzw. (II) häufig als Mischung verschiedener Stereoisomere an. Auch aus diesem Grund sind Gemische erfindungsgemäßer bzw. erfindungsgemäß einzusetzender Verbindungen der Formel (I), (I-A) bzw. (II) im Zusammenhang mit der vorliegenden Erfindung regelmäßig bevorzugt.

Sofern eine Verbindung der Formel (I), (I-A) bzw. (II) als Gemisch ihrer cis- und trans-Isomeren eingesetzt wird, liegt das Massenverhältnis von cis : trans - Isomeren vorzugsweise im Bereich von 4 : 1 bis 1 : 4, bevorzugt im Bereich von 3 : 1 bis 1 : 3, weiter bevorzugt im Bereich von 2 : 1 bis 1 : 2, insbesondere bevorzugt im Bereich von 3 : 2 bis 2 : 3.

So wurde beispielsweisegefunden, dass sich das cis-Isomer und das trans-Isomer von 2-Isobutyl-4-vinyl-[1,3]dioxolan geruchlich etwas unterscheiden. Während cis-2-Isobutyl-4-vinyl-[1,3]dioxolan einen Geruch von natürlichen Geruch von Olive, insbesondere von grüner Olive, gepaart mit rosigen Aspekten, aufweist, zeigt trans-2-Isobutyl-4-vinyl-[1,3]dioxolan hingegen einen Geruch von Olive, insbesondere von grüner Olive, und Knoblauch, gepaart mit einer fettigen Note.

Cis-2-Isobutyl-4-vinyl-[1,3]dioxolan eignet sich insbesondere als Riechstoff. Trans-2-Isobutyl-4-vinyl-[1,3]dioxolan ist wegen seiner fettigen Note insbesondere als Aromastoff geeignet.

Die nachfolgende Tabelle enthält die sensorischen, insbesondere die geruchlichen, Beschreibungen von besonders bevorzugten erfindungsgemäß einzusetzenden Verbindungen der Formel (I).

| **Eintrag** | **Verbindung** | **Struktur** | **Sensorische Beschreibung** |
|---|---|---|---|
| 1 | 2-Isobutyl-4-vinyl-[1,3]dioxolan | 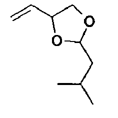 | Stark, grüne Oliven, natürlich |
| 2 | 2-secButyl-4-vinyl-[1,3]dioxolan | 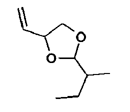 | Frucht |
| 3 | 2-Isopropyl-4-vinyl-[1,3]dioxolan | 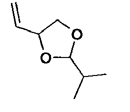 | Metallisch, Grün |
| 4 | 2-(2,4-Dimethylcyclohex-3-enyl)-4-vinyl-[1,3]dioxolan | 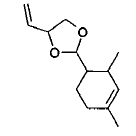 | Frucht, Blüte, Rose |
| 5 | 2-(2,6-Dimethylhept-5-enyl)-4-vinyl-[1,3]dioxolan | 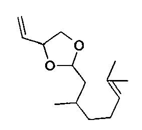 | Frisch, Myrrhe, Frucht, Birne, Mango, Blüte, Grün |
| 6 | 2-Ethyl-2-(2-methylbutyl)-4-vinyl-[1,3]dioxolan | 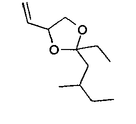 | Frucht, Grapefruit |
| 7 | 2-(2,4,4-Trimethylpentyl)-4-vinyl-[1,3]dioxolan | 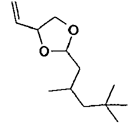 | Wässrig, Frucht, Melone |
| 8 | 2-(3,5-Dimethylhex-4-enyl)-2-methyl-4-vinyl-[1,3]dioxolan | 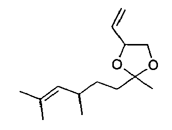 | Frucht, Grün |
| 9 | 2-Methyl-2-(4-methylpent-3-enyl)-4-vinyl-[1,3]dioxolan | 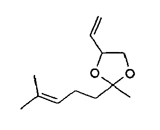 | Grün, Blüte, Aromatisch, Zitrone, Linalool |
| 10 | 8-tertButyl-2-vinyl-1,4-dioxaspiro-[4.5]-decan | 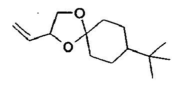 | Frucht, Melone |
| 11 | 2-(2,6-Dimethylhepta-1,5-dienyl-4-vinyl-[1,3]dioxolan | 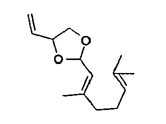 | Frisch, Zitrone, Wässrig |
| 12 | 2-[2-(4-Methylcyclohex-3-enyl)-propyl]-4-vinyl-[1,3]dioxolan | 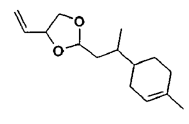 | Frucht, Mirabelle |
| 13 | 2-Phenyl-4-vinyl-[1,3]dioxolan | 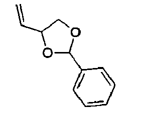 | Kraut, Krauseminz, Phenolisch, Pilz, Metallisch |
| 14 | 2-Cyclohexyl-4-vinyl-[1,3]dioxolan | 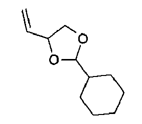 | Frucht, Kamille, Apfel, Aromatisch |
| 15 | 2-(2,2,3-Trimethylcyclopent-3-enylmethyl)-4-vinyl-[1,3]dioxolan | 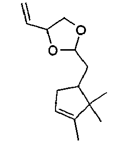 | Frucht, Apfel, Blüte, Carbinol |
| 16 | 2-Vinyl-1,4-dioxas-piro[4.5]decan | 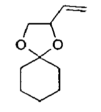 | Frucht, Zitrone |
| 17 | 7-Methyl-2-vinyl-1,4-dioxas-piro[4.5]decan | 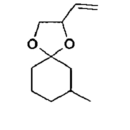 | Frisch, Agrumen, Zitrone |
| 18 | 2-(2-Methylpropenyl)-4-vinyl-[1,3]dioxolan | 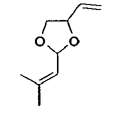 | Kraut, Fettig |
| 19 | 2-Methyl-2-(3-methylbutyl)-4-vinyl-[1,3]dioxolan | 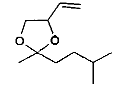 | Frisch, Grapefruit |

Erfindungsgemäß bevorzugt ist die Verwendung einer Verbindung der Formel (I), (I-A) bzw. (II), vorzugsweise in einer der als bevorzugt bzw. besonders bevorzugt gekennzeichneten Ausgestaltungen, zum Vermitteln, Verstärken und/oder Modifizieren einer, zweier, dreier oder mehrerer der Geruchs- und/oder Geschmacksnoten blumig, fettig, grüne Oliven, ledrig, Frucht, Grün, Blüte, Rose, Myrrhe, Birne, Mango, Grapefruit, Melone, Zitrone, Linalool, Mirabelle, Krauseminz, Phenolisch, Pilz, Metallisch, Kamille, Apfel, Carbinol, Aromatisch, Wässrig, Frisch, Kraut.

Entsprechend betrifft die Erfindung auch ein Verfahren zum Vermitteln, Modifizieren und/oder Verstärken eines Geruchs und/oder Geschmacks ohne Vermittlung des Geruchs und Geschmacks der Note Ananas, wobei eine sensorisch wirksame Menge
- einer erfindungsgemäßen Riechstoff- und/oder Aromastoffkomposition (wie nachfolgend definiert), vorzugsweise in einer der als bevorzugt oder besonders bevorzugt gekennzeichneten Ausgestaltungen,
   oder
- einer erfindungsgemäßen bzw. erfindungsgemäß einzusetzenden Verbindung der Formel (I), (I-A) oder (II), vorzugsweise in einer der als bevorzugt oder besonders bevorzugt gekennzeichneten Ausgestaltungen,
   mit einem Erzeugnis in Kontakt gebracht oder gemischt wird,
   insbesondere zum Vermitteln, Modifizieren und/oder Verstärken einer, zweier oder mehrerer der Geruchs- und/oder Geschmacksnoten blumig, fettig, grüne Oliven, ledrig, Frucht, Grün, Blüte, Rose, Myrrhe, Birne, Mango, Grapefruit, Melone, Zitrone, Linalool, Mirabelle, Krauseminz, Phenolisch, Pilz, Metallisch, Kamille, Apfel, Carbinol, Aromatisch, Wässrig, Frisch, Kraut.

Die vorliegende Erfindung betrifft auch die neue Verbindung 2-(2,6-Dimethylhept-5-enyl)-4-vinyl-[1,3]dioxolan.

Die Verbindungen der Formel (I) können beispielsweise durch Umsetzung von 1-Buten-3,4-diol und den entsprechenden Aldehyden (wenn nur einer der beiden Gruppe R3 oder R4 = H ist) bzw. Ketonen (wenn R3 und R4 nicht H bedeuten) erhalten werden, wie nachfolgend anhand des Aldehyds Isovaleraldehyd exemplarisch gezeigt:

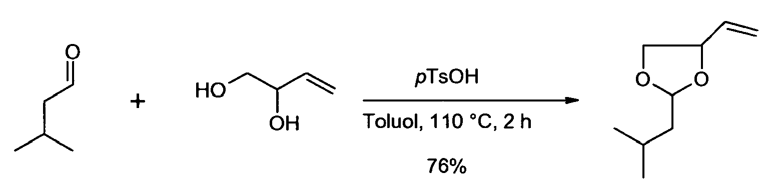

Wird beispielsweise Isovaleraldehyd in Toluol in Gegenwart von p-TsOH (para-Toluolsulfonsäure) mit 1-Buten-3,4-diol bei 110°C umgesetzt, wird nach 2 Stunden das entsprechende Dioxolan in 76%iger Ausbeute erhalten.

Derzeit ist 1-Buten-3,4-diol jedoch nicht in großer Menge kommerziell verfügbar.

1-Buten-3,4-diol kann beispielsweise ausgehend von 2-Buten-1,4-diol hergestellt werden, wie in DE 3334589 beschrieben:

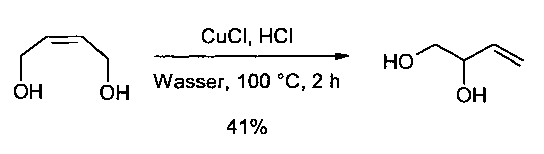

Aufgrund der niedrigen Gesamtausbeute an Dioxolanen wurde im Rahmen der vorliegenden Erfindung ein neuer Syntheseweg entwickelt, um zu Verbindungen der Formel (I) zu gelangen.

Das nachfolgende Reaktionsschema verdeutlicht exemplarisch die Umsetzung von Isovaleraldehyd mit dem kommerziell gut verfügbaren 2-Buten-1,4-diol in einem erfindungsgemäßen Verfahren:

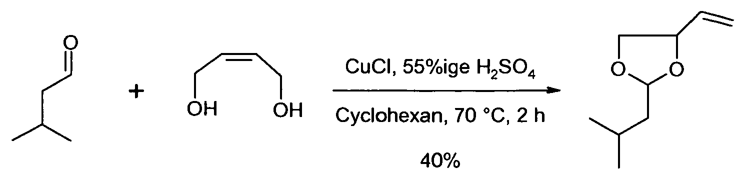

Mit dem Verfahren sind die Verbindungen der Formel (I) in einer Stufe und in einer Eintopfreaktion ausgehend von 2-Buten-1,4-diol erhältlich.

Ein Verfahren zur Herstellung einer Verbindung der Formel (I) umfassend den folgenden Schritt:
Reaktion einer Verbindung der Formel (III) mit einer Verbindung der Formel (IV) zu der Verbindung der Formel (I) gemäß dem Schema 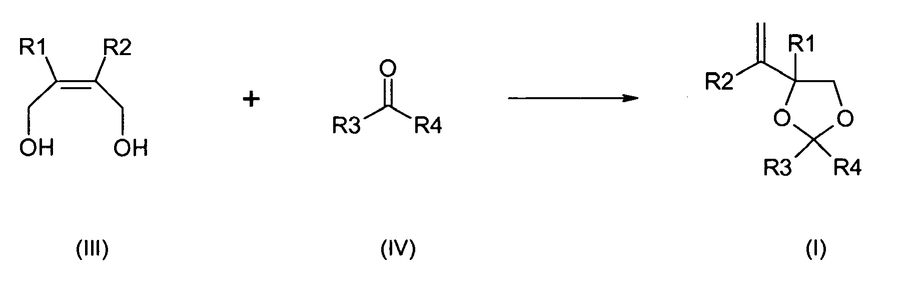 wobei die Reste R1, R2, R3 und R4 der Formeln (III) und (IV) wie die entsprechenden Reste für Formel (I) definiert sind.

Die Reste R1, R2, R3 und R4 haben dabei vorzugsweise jeweils die oben angegebene bevorzugte bzw. besonders bevorzugte Bedeutung.

Im Ergebnis entsprechen die Verbindungen der Formel (I) den Verbindungen der folgenden Strukturformel, in welcher im Vergleich zu der Formel (I) die Reste R1 und R2 bezüglich ihrer Positionen vertauscht sind

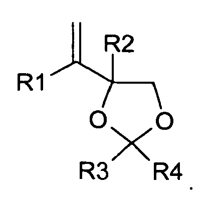

Die oben hinsichtlich der Verwendung als bevorzugt bzw. besonders bevorzugt gekennzeichneten Verbindungen der Formel (I), (I-A) und (II) gelten für das Herstellverfahren entsprechend.

Das Verfahren ist vorzugsweise dadurch gekennzeichnet, dass die Reaktion unter Zusatz von Kupfer(I)chlorid abläuft, vorzugsweise unter Zusatz von Kupfer(I)chlorid in wässriger Schwefelsäure, vorzugsweise 55%iger Schwefelsäure, und Cyclohexan.

Ein bevorzugtes Verfahren umfasst den Schritt:
Erhitzen einer Mischung aus einer Verbindung der Formel (III), einer Verbindung der Formel (IV) und einer katalytisch wirksamen Menge Kupfer(I)chlorid, vorzugsweise in einem 2-Phasen-System, bevorzugt einem 2-Phasen-System aus wässriger Schwefelsäure, besonders bevorzugt 55%iger Schwefelsäure, und Cyclohexan.

In einem bevorzugten Verfahren werden 0,8 bis 1,2 molare Äquivalente einer Verbindung der Formel (III), 0,8 bis 1,2 molare Äquivalente einer Verbindung der Formel (IV) und 0,02 bis 0,07 molare Äquivalente Kupfer(I)chlorid eingesetzt.

Das Verfahren wird vorzugsweise in der Weise durchgeführt, dass die Reaktionszeit 60 bis 240 Minuten, bevorzugt 80 bis 160 Minuten, weiter bevorzugt 100 bis 140 Minuten, besonders bevorzugt 110 bis 130 Minuten beträgt.

Das Verfahren wird vorzugsweise bei einer Reaktionstemperatur im Bereich von 40 bis 120°C, bevorzugt im Bereich von 50 bis 90°C, weiter bevorzugt im Bereich 60 bis 75°C, insbesondere bevorzugt im Bereich 65 bis 72°C durchgeführt. Das Verfahren wird vorzugsweise bei Umgebungsdruck durchgeführt, d.h. bei einem Druck von etwa 1013 mbar.

Ein besonders bevorzugtes Verfahren wird bei einer Reaktionstemperatur im Bereich von 60 bis 75°C für die Dauer von zwei Stunden durchgeführt.

Nachfolgende Arbeitsvorschrift kann dabei zur Herstellung der Verbindungen der Formel (I) benutzt werden: eine Mischung aus dem Diol der Formel (III) (1.00 Äquiv.), dem Aldehyd bzw. Keton der Formel (IV) (1.00 Äquiv.) und Kupfer(I)chlorid (0.04 Äquiv.) wird in 55%ige Schwefelsäure (44 g/mol) und Cyclohexan (22 g/mol) bei 70 °C für 2 Stunden erhitzt. Nach Abkühlung und Phasentrennung wird die organische Phase mit Wasser gewaschen und die wässrige Phase erneut abgetrennt. Nach Entfernung des Lösungsmittels kann das Rohprodukt weiter gereinigt werden, vorzugsweise durch eine Destillation über Natriumcarbonat.

Bevorzugt ist ein Verfahren, wobei für die Verbindung der Formel (I) und die Verbindung der Formel (IV) jeweils wenigstens eine der folgenden Bedingungen gilt:
i. sofern R4 ein verzweigter oder unverzweigter Alkyl-Rest mit 4 bis 15 Kohlenstoff-Atomen mit optional ein, zwei oder mehreren Doppelbindungen ist, an Position 3 zu Position 4 von R4 eine Kohlenstoff-Kohlenstoff-Einfachbindung vorliegt
   oder
ii. sofern R4 ein verzweigter oder unverzweigter Alkyl-Rest mit 2 bis 15 Kohlenstoff-Atomen mit optional ein, zwei oder mehreren Doppelbindungen ist, R3 nicht Wasserstoff ist, und vorzugsweise R3 ein verzweigter oder unverzweigter Alkyl-Rest mit 1 bis 15 KohlenstoffAtomen und optional ein, zwei oder mehreren Doppelbindungen ist.

Ein bevorzugtes Verfahren ist ein Verfahren, wobei für die Verbindung gemäß Formel (I) und die Verbindung der Formel (IV) jeweils nur die vorstehend genannte Bedingung i. vorliegt.

Ein bevorzugtes Verfahren ist ein Verfahren, wobei für die Verbindung gemäß Formel (I) und die Verbindung der Formel (IV) jeweils nur die vorstehend genannte Bedingung ii. vorliegt.

Ein bevorzugtes Verfahren ist ein Verfahren, wobei für die Verbindung gemäß Formel (I) und die Verbindung der Formel (IV)jeweils beide vorstehend genannte Bedingungen i. und ii. vorliegen.

Ein Verfahren ist besonders geeignet zur Herstellung der folgenden erfindungsgemäß bevorzugt einzusetzenden Verbindungen:
2-Isobutyl-4-vinyl-[1,3]dioxolan, 2-secButyl-4-vinyl-[1,3]dioxolan, 2-Isopropyl-4-vinyl-[1,3]dioxolan, 2-(3,5-Dimethylcyclohex-3-enyl)-4-vinyl-[1,3]dioxolan, 2-(2,4-Dimethylcyclohex-3-enyl)-4-vinyl-[1,3]dioxolan, 2-(2,6-Dimethylhept-5-enyl)-4-vinyl-[1,3]dioxolan, 2-Ethyl-2-(2-methylbutyl)-4-vinyl-[1,3]dioxolan, 2-(2,4,4-Trimethylpentyl)-4-vinyl-[1,3]dioxolan, 2-(3,5-Dimethylhex-4-enyl)-2-methyl-4-vinyl-[1,3]dioxolan, 2-Methyl-2-(4-methylpent-3-enyl)-4-vinyl-[1,3]dioxolan, 8-tertButyl-2-vinyl-1,4-dioxaspiro-[4.5]-decan, 2-(2,6-Dimethylhepta-1,5-dienyl-4-vinyl-[1,3]dioxolan, 2-[2-(4-Methylcyclohex-3-enyl)-propyl]-4-vinyl-[1,3]dioxolan, 2-Phenyl-4-vinyl-[1,3]dioxolan, 2-Cyclohexyl-4-vinyl-[1,3]dioxolan, 2-(2,2,3-Trimethylcyclopent-3-enylmethyl)-4-vinyl-[1,3]dioxolan, 2-Vinyl-1,4-dioxaspiro[4.5]decan, 7-Methyl-2-vinyl-1,4-dioxaspiro[4.5]decan, 2-(2-Methylpropenyl)-4-vinyl-[1,3]dioxolan, und 2-Methyl-2-(3-methylbutyl)-4-vinyl-[1,3]dioxolan.

Ein Verfahren ist besonders geeignet zur Herstellung der folgenden erfindungsgemäß besonders bevorzugt einzusetzenden Verbindungen:
2-Isobutyl-4-vinyl-[1,3]dioxolan, 2-(3,5-Dimethylcyclohex-3-enyl)-4-vinyl-[1,3]dioxolan, 2-(2,4-Dimethylcyclohex-3-enyl)-4-vinyl-[1,3]dioxolan, 2-(2,6-Dimethylhept-5-enyl)-4-vinyl-[1,3]dioxolan, 2-Vinyl-1,4-dioxaspiro[4.5]decan, 7-Methyl-2-vinyl-1,4-dioxaspiro[4.5]decan, 2-(2-Methylpropenyl)-4-vinyl-[1,3]dioxolan, und 2-Methyl-2-(3-methylbutyl)-4-vinyl-[1,3]dioxolan.

In einer besonders bevorzugten Ausgestaltung ist in einem Verfahren die Verbindung der Formel (III) 2-Buten-1,4-diol und die Verbindung der Formel (IV) Isovaleraldehyd.

Die Erfindung betrifft auch eine Riechstoff- und/oder Aromastoffkomposition umfassend eine, zwei, drei oder mehrere Verbindungen der Formel (I), 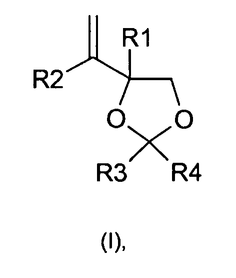 einschließlich der Stereoisomeren, wobei
R1 und R2 jeweils unabhängig voneinander entweder Wasserstoff oder Methyl, vorzugsweise Wasserstoff ist, und
a) R3 und R4 einen Ring mit insgesamt 5, 6, 7 oder 8 Ring-Kohlenstoff-Atomen bilden, wobei der Ring optional eine oder zwei Doppelbindungen und/oder optional 1 bis maximal 3 Sauerstoffatome enthält, und wobei der Ring optional substituiert ist mit einer oder mehreren verzweigten oder unverzweigten, verbrückten oder unverbrückten, Alkylgruppen, Alkenylgruppen, Cycloalkylgruppen, Cycloalkenylgruppen, Arylgruppen, Arylalkylgruppen, Alkoxyalkylgruppen oder Alkoxyarylgruppen, und wobei R3 und R4 zusammen insgesamt 3 bis 30 Kohlenstoff-Atome umfassen,
   oder
b) R3 entweder Wasserstoff oder ein organischer Rest mit 1 bis 15 Kohlenstoff-Atomen ist, wobei der organische Rest optional 1 bis maximal 3 Sauerstoff-Atome enthält, vorzugsweise R3 entweder Wasserstoff oder ein gesättigtes oder ungesättigtes, aromatisches oder aliphatisches, verzweigtes oder unverzweigtes, cyclisches oder lineares, verbrücktes oder unverbrücktes Strukturelement mit 1 bis 15 Kohlenstoff-Atomen ist, wobei das Strukturelement optional 1 bis maximal 3 Sauerstoff-Atome enthält und das Strukturelement optional substituiert ist mit 1 bis maximal 3 Alkyl- und/oder Alkenylgruppen und/oder mit einer oder zwei Hydroxygruppen,
   und
   R4 ein organischer Rest mit 2 bis 15 Kohlenstoff-Atomen ist, wobei der organische Rest optional 1 bis maximal 3 Sauerstoff-Atome enthält, vorzugsweise R4 ein gesättigtes oder ungesättigtes, aromatisches oder aliphatisches, verzweigtes oder unverzweigtes, cyclisches oder lineares, verbrücktes oder unverbrücktes Strukturelement mit 2 bis 15 Kohlenstoff-Atomen ist, wobei das Strukturelement optional 1 bis maximal 3 Sauerstoff-Atome enthält und das Strukturelement optional substituiert ist mit 1 bis maximal 3 Alkyl- und/oder Alkenylgruppen und/oder mit einer oder zwei Hydroxygruppen,
   und wobei wenigstens eine der folgenden Bedingungen gilt:
   i. sofern R4 ein verzweigter oder unverzweigter Alkyl-Rest mit 4 bis 15 Kohlenstoff-Atomen mit optional ein, zwei oder mehreren Doppelbindungen ist, an Position 3 zu Position 4 von R4 eine Kohlenstoff-Kohlenstoff-Einfachbindung vorliegt
      oder
   ii. sofern R4 ein verzweigter oder unverzweigter Alkyl-Rest mit 2 bis 15 Kohlenstoff-Atomen
      mit optional ein, zwei oder mehreren Doppelbindungen ist, R3 nicht Wasserstoff ist,
   und wobei die Riechstoff- und/oder Aromastoffkomposition zumindest 3, 4, 5, 6, 7, 8 oder mehr weitere Riech- und/oder Aromastoffe enthält, bei denen es sich jeweils nicht um eine Verbindung der Formel (I) handelt,
   und wobei die Riechstoff- und/oder Aromastoffkomposition nicht 2-Hexyl-4-vinyl-1,3-dioxolan enthält.
   Eine erfindungsgemäß bevorzugte Riechstoff- und/oder Aromastoffkomposition umfasst eine, zwei, drei oder mehrere Verbindungen der obigen Formel (I), wobei
   R1 und R2 jeweils unabhängig voneinander entweder Wasserstoff oder Methyl, jeweils vorzugsweise Wasserstoff ist,
   und
   a) R3 und R4 zusammen ein cyclisches Alkylsystem mit 5, 6, 7 oder 8 Kohlenstoff-Atomen ergeben, das optional substituiert ist mit einem oder mehreren verzweigten oder unverzweigten Alkyl-Resten ausgewählt aus der Gruppe bestehend aus Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, und wobei das Alkylsystem optional eine oder zwei Doppelbindungen enthält,
      oder
   b) R3 entweder Wasserstoff oder ein verzweigter oder unverzweigter Alkyl-Rest mit 1 bis 15 Kohlenstoff-Atomen und optional ein, zwei oder mehreren Doppelbindungen oder ein cyclisches Alkylsystem mit 5, 6 oder 7 Kohlenstoff-Atomen mit optional ein, zwei oder mehreren Doppelbindungen ist, wobei der Alkyl-Rest vorzugsweise ausgewählt ist aus der Gruppe bestehend aus Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl,
   und
   1. R4 ein Aromat ist, wobei der Aromat optional mit einem, zwei oder mehreren Alkyl-Resten mit 1 bis 3 Kohlenstoff-Atomen und/oder einem, zwei oder mehreren Hydroxy-Resten und/oder einem, zwei oder mehreren Alkoxy-Resten substituiert ist,
      oder
   2. R4 ein mono-, bi- oder tricyclisches Alkylsystem ist, das optional mit einem, zwei oder mehreren Alkyl-Resten, Hydroxy-Resten oder Alkoxy-Resten substituiert ist, wobei die Alkyl-Reste vorzugsweise ausgewählt sind aus der Gruppe bestehend aus Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl,
      oder
   3. R4 ein verzweigter oder unverzweigter Alkyl-Rest mit 2 bis 15 Kohlenstoff-Atomen mit optional ein, zwei oder mehreren Doppelbindungen oder ein Ringsystem mit 5, 6 oder 7 Kohlenstoff-Atomen mit optional ein, zwei oder mehreren Doppelbindungen, ist,
   und wobei die Riechstoff- und/oder Aromastoffkomposition zumindest 3, 4, 5, 6, 7, 8 oder mehr weitere Riech- und/oder Aromastoffe enthält, bei denen es sich jeweils nicht um eine Verbindung der Formel (I) handelt,
   und wobei die Riechstoff- und/oder Aromastoffkomposition nicht 2-Hexyl-4-vinyl-1,3-dioxolan enthält.

Eine erfindungsgemäß weiter bevorzugte Riechstoff- und/oder Aromastoffkomposition umfasst eine, zwei, drei oder mehrere Verbindungen der Formel (II), 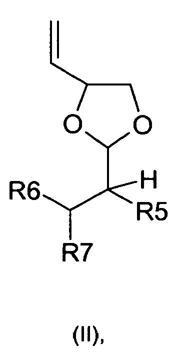 einschließlich der Stereoisomeren, umfasst, wobei
a) R5 und R6 jeweils unabhängig voneinander entweder Wasserstoff oder Methyl ist,
   und
b) R7 ein organischer Rest mit 1 bis 10 Kohlenstoff-Atomen ist, wobei der organische Rest optional 1 bis maximal 3 Sauerstoff-Atome enthält, vorzugsweise R7 ein gesättigtes oder ungesättigtes, aromatisches oder aliphatisches, verzweigtes oder unverzweigtes, cyclisches oder lineares, verbrücktes oder unverbrücktes Strukturelement mit 1 bis 10 Kohlenstoff-Atomen ist, wobei das Strukturelement optional 1 bis maximal 3 SauerstoffAtome enthält und das Strukturelement optional substituiert ist mit 1 bis maximal 3 Alkylund/oder Alkenylgruppen und/oder mit einer Hydroxygruppe, und
wobei sofern R7 ein verzweigter oder unverzweigter Alkyl-Rest mit 1 bis 10 Kohlenstoff-Atomen ist, an Position 1 zu Position 2 von R7 eine Kohlenstoff-Kohlenstoff-Einfachbindung vorliegt.

Eine erfindungsgemäß bevorzugte Riechstoff- und/oder Aromastoffkomposition umfasst eine, zwei, drei oder mehrere Verbindungen der obigen Formel (II), wobei
R5 und R6 jeweils unabhängig voneinander entweder Wasserstoff oder Methyl ist, und
R7 ein verzweigter oder unverzweigter Alkyl-Rest mit 1 bis 10 Kohlenstoff-Atomen, vorzugsweise ausgewählt aus der Gruppe bestehend aus Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, ist,
oder
R7 ein Cyloalkyl-Rest mit 5, 6 oder 7 Kohlenstoff-Atomen ist, der optional substituiert ist mit einem oder mehreren Alkyl-Resten, ausgewählt aus der Gruppe bestehend aus Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, einem Hydroxy-Rest oder einem Alkoxy-Rest,
und
wobei sofern R7 ein verzweigter oder unverzweigter Alkyl-Rest mit 1 bis 10 Kohlenstoff-Atomen ist, an Position 1 zu Position 2 von R7 eine Kohlenstoff-Kohlenstoff-Einfachbindung vorliegt.
Eine erfindungsgemäß weiter bevorzugte Riechstoff- und/oder Aromastoffkomposition umfasst eine, zwei, drei oder mehrere Verbindungen der obigen Formel (I), ausgewählt aus der Gruppe bestehend aus 2-Isobutyl-4-vinyl-[1,3]dioxolan, 2-secButyl-4-vinyl-[1,3]dioxolan, 2-Isopropyl-4-vinyl-[1,3]dioxolan, 2-(3,5-Dimethylcyclohex-3-enyl)-4-vinyl-[1,3]dioxolan, 2-(2,4-Dimethylcyclohex-3-enyl)-4-vinyl-[1,3]dioxolan, 2-(2,6-Dimethylhept-5-enyl)-4-vinyl-[1,3]dioxolan, 2-Ethyl-2-(2-methylbutyl)-4-vinyl-[1,3]dioxolan, 2-(2,4,4-Trimethylpentyl)-4-vinyl-[1,3]dioxolan, 2-(3,5-Dimethylhex-4-enyl)-2-methyl-4-vinyl-[1,3]dioxolan, 2-Methyl-2-(4-methylpent-3-enyl)-4-vinyl-[1,3]dioxolan, 8-tertButyl-2-vinyl-1,4-dioxaspiro-[4.5]-decan, 2-(2,6-Dimethylhepta-1,5-dienyl-4-vinyl-[1,3]dioxolan, 2-[2-(4-Methylcyclohex-3-enyl)-propyl]-4-vinyl-[1,3]dioxolan, 2-Phenyl-4-vinyl-[1,3]dioxolan, 2-Cyclohexyl-4-vinyl-[1,3]dioxolan, 2-(2,2,3-Trimethylcyclopent-3-enylmethyl)-4-vinyl-[1,3]dioxolan, 2-Vinyl-1,4-dioxaspiro[4.5]decan, 7-Methyl-2-vinyl-1,4-dioxaspiro[4.5]decan, 2-(2-Methylpropenyl)-4-vinyl-[1,3]dioxolan, und 2-Methyl-2-(3-Methylbutyl)-4-vinyl-[1,3]dioxolan.

Eine erfindungsgemäß besonders bevorzugte Riechstoff- und/oder Aromastoffkomposition umfasst eine, zwei, drei oder mehrere Verbindungen der obigen Formel (I), ausgewählt aus der Gruppe bestehend aus
2-Isobutyl-4-vinyl-[1,3]dioxolan, 2-(3,5-Dimethylcyclohex-3-enyl)-4-vinyl-[1,3]dioxolan, 2-(2,4-Dimethylcyclohex-3-enyl)-4-vinyl-[1,3]dioxolan, 2-(2,6-Dimethylhept-5-enyl)-4-vinyl-[1,3]dioxolan, 2-Vinyl-1,4-dioxaspiro[4.5]decan, 7-Methyl-2-vinyl-1,4-dioxaspiro[4.5]decan, 2-(2-Methylpropenyl)-4-vinyl-[1,3]dioxolan, und 2-Methyl-2-(3-methylbutyl)-4-vinyl-[1,3]dioxolan.

In einer erfindungsgemäßen Aromastoffkomposition (wie oben definiert) sind vorzugsweise einer, mehrere, besonders bevorzugt zwei, drei, vier, fünf, sechs, sieben, acht, neun oder zehn, oder sämtliche der weiteren Aromastoffe, ausgewählt aus der Gruppe bestehend aus:
Acetophenon, Allylcapronat, alpha-Ionon, beta-Ionon, Anisaldehyd, Anisylacetat, Anisylformiat, Benzaldehyd, Benzothiazol, Benzylacetat, Benzylalkohol, Benzylbenzoat, beta-Ionon, Buttersäure, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Essigsäure, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion^{®}), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cisdecadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie deren Stereoisomere, Enantiomere, Diastereomere sowie gegebenenfalls cis/trans-Isomere.

Beispiele für Riechstoffe, mit denen die erfindungsgemäßen bzw. erfindungsgemäß zu verwendenden Verbindungen der Formel (I) vorteilhaft kombiniert werden können und die demnach bevorzugt in einer erfindungsgemäßen Riechstoffkomposition (wie oben definiert) enthalten sind, finden sich z.B. in S. Arctander, Perfume and Flavor Materials, Vol. I und II, Montclair, N. J., 1969, Selbstverlag oder H. Surburg, J. Panten, Common Fragrance and Flavor Materials, 5th. Ed., Wiley-VCH, Weinheim 2006.

In einer erfindungsgemäßen Riechstoffkomposition (wie oben definiert) sind vorzugsweise einer, mehrere, besonders bevorzugt zwei, drei, vier, fünf, sechs, sieben, acht, neun oder zehn, oder sämtliche der weiteren Riechstoffe, ausgewählt aus der Gruppe bestehend aus:

Extrakte aus natürlichen Rohstoffen wie Etherische Öle, Concretes, Absolues, Resine, Resinoide, Balsame, Tinkturen wie z. B.

Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreumabsolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptuscitriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl, sowie Fraktionen davon, bzw. daraus isolierten Inhaltsstoffen;

Einzel-Riechstoffe aus der Gruppe
der Kohlenwasserstoffe, wie z. B. 3-Caren; α- Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z. B. Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale, die nicht den Formeln (I), (I-A) und (II) entsprechen, wie z. B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-5,9-undecadienal; Heptanaldiethylacetal; 1,1-Dimethoxy-2,2,5-trimethyl-4-hexen; Citronellyloxyacetaldehyd;
der aliphatischen Ketone und deren Oxime wie z. B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen wie z. B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Tridecensäurenitril; 2,12-Tridecadiensäurenitril; 3,7-Dimethyl-2,6-octadiensäurenitril; 3,7-Dimethyl-6-octensäurenitril;
der aliphatischen Carbonsäuren und deren Ester wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;
der acyclischen Terpenalkohole wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;
der acyclischen Terpenaldehyde und -ketone wie z. B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
der cyclischen Terpenaldehyde und -ketone wie z. B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; Nootkaton; Dihydronootkaton; alpha-Sinensal; beta-Sinensal; Acetyliertes Cedernholzöl (Methylcedrylketon);
der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 7-Cyclohexadecen-1-on; 8-Cyclohexadecen-1-on; 9-Cycloheptadecen-1-on; Cyclopentadecanon; Cyclohexadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert-Butylcyclohexylacetat; 2-tert-Pentylcyclohexylacetat; 4-tert-Pentylcyclohexylacetat; Decahydro-2-naphthylacetat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxane; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 5-Phenyl-3-methyl-2-pentensäurenitril; 5-Phenyl-3-methylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

In einer bevorzugten Ausgestaltung einer erfindungsgemäßen Riech- oder Aromastoffkomposition sind die erfindungsgemäßen bzw. erfindungsgemäß zu verwendenden Verbindungen der Formel (I) vorzugsweise mit einem oder mehreren, besonders bevorzugt mit zwei, drei, vier, fünf oder mehr, blumigen und/oder fruchtigen weiteren Riech- und/oder Aromastoffen kombiniert.

Entsprechend betrifft die vorliegende Erfindung auch eine Riechstoff- oder Aromastoffkomposition, die einen, zwei, drei, vier, fünf oder mehrere Riech- und/oder Aromastoffe umfasst, die eine blumige und/oder fruchtige Geruchs- oder Geschmacksnote vermitteln.

Dabei wird durch die erfindungsgemäße bzw. erfindungsgemäß zu verwendende Verbindung der Formel (I) vorteilhafterweise (zumindest teilweise) eine geruchliche Verstärkung der blumigen Riech- bzw. Aromastoffe erreicht.

Blumige Riech- oder Aromastoffe, mit denen erfindungsgemäßen bzw. erfindungsgemäß zu verwendenden Verbindungen der Formel (I) (insbesondere in erfindungsgemäßen Riech- oder Aromastoffkompositionen) vorteilhaft kombiniert werden können, werden vorzugsweise ausgewählt aus der Gruppe bestehend aus:

Hydroxycitronellal, Methoxycitronellal, Cyclamenaldehyd [2-Methyl-3-(4-isopropylphenyl)propanal], 1-(4-Isopropyl-cyclohexyl)ethanol (Mugetanol^{®}), 4-tert.-Butyl-α-methyldihydrozimtaldehyd (Lilial^{®}), cis-Hexahydrocuminylalkohol (Mayol^{®}), 3-[4-(1,1-Dimethylethyl)phenyl]propanal (Bourgeonal^{®}), 2,2-Dimethyl-3-(3-methylphenyl)propanol (Majantol^{®}), 3-Methyl-3-(3-methylbenzyl)-butan-2-ol, 2-Isobutyl-4-methyltetrahydro-2H-pyran-4-ol (Florosa^{®}), 2-Methyl-3-(3,4-methylendioxyphenyl)propanal (Heliofolal^{®}), 4-(4-Hydroxy-4-methylpentyl)-3-cyclohexencarbaldehyd (Lyral^{®}), 4-(Octahydro-4,7-methano-5H-inden-5-ylidenbutanal (Dupical^{®}), Vernaldehyd, 4-(4-Methyl-3-penten-1-yl)-3-cyclohexencarbaldehyd (Vertomugal^{®}), Octahydro-5-(4-methoxybutyliden)-4,7-methano-1H-inden (Mugoflor^{®}), 2,6-Dimethyl-2-heptanol (Freesiol^{®}), 1-Ethyl-1-methyl-3-phenylpropanol (Phemec^{®}), 2,2-Dimethyl-3-phenyl-1-propanol (Muguet alcohol), Profarnesol, Dihydrofarnesol, Farnesol, Nerolidol, Hydroxycitronellaldimethylacetal, Hexylbenzoat, Geraniol, Nerol, Linalool, Tetrahydrogeraniol, Tetrahydrolinalool, Ethyllinalool, Geranyltiglinat, Phenethylalkohol (2-Phenylethylalkohol), Citronellol, Rosenoxid, 2-Methyl-5-phenylpentanol (Rosaphen), 3-Methyl-5-phenylpentanol (Phenoxanol), Methyldihydrojasmonat (Hedion^{®}, Hedione^{®} high cis), 2-Heptylcyclopentanon (Projasmon P), cis-Jasmon, Dihydrojasmon, Zimtalkohol (3-Phenyl-2-propen-1-ol), Dihydrozimtalkohol (3-Phenylpropanol), 2-Methyl-4-phenyl-1,3-dioxolan (Jacinthaflor^{®}) und Dihydromyrcenol (2,6-Dimethyl-7-octen-2-ol).

Zudem sind erfindungsgemäße bzw. erfindungsgemäß zu verwendende Verbindungen der Formel (I) vorteilhafterweise dazu geeignet, fruchtige Riech- oder Aromastoffe, insbesondere fruchtige Riechstoffe, hinsichtlich ihres Geruchs zu verstärken.

Fruchtige Riech- oder Aromastoffe, mit denen die erfindungsgemäßen bzw. erfindungsgemäß zu verwendenden Verbindungen der Formel (I) vorteilhaft kombiniert werden können, und die demnach besonders bevorzugte (weitere) Riech- oder Aromastoffe einer erfindungsgemäßen Riech- oder Aromastoffkompositionen sind, sind vorzugsweise ausgewählt der Gruppe bestehend aus:

2-Methyl-buttersäureethylester, 4-(p-Hydroxyphenyl)-2-butanon, Ethyl-3-methyl-3-phenylglycidat, Buttersäureisoamylester, Essigsäureisoamylester, Essigsäure-n-butylester, Buttersäureethylester, 3-Methyl-buttersäureethylester, n-Hexansäureethylester, n-Hexansäureallylester, Ethyl-2-trans-4-cis-decadienoat, 1,1-Dimethoxy-2,2,5-trimethyl-4-hexan, 2,6-Dimethyl-5-hepten-1-al, gamma-Undecalacton, gamma-Nonalacton, Hexanal, 3Z-Hexenal, n-Decanal, n-Dodecanal, Citral, Vanillin, Ethylvanillin, Maltol, Ethylmaltol und deren Mischungen.

Bevorzugte Riechstoff- oder Aromastoffkomposition, umfassen eine Gesamtmenge an Verbindungen der Formel (I) und/oder (II) im Bereich von 0,0001 bis 70 Gew.-%, vorzugsweise im Bereich 0,001 bis 50 Gew.-% und besonders bevorzugt im Bereich 0,01 bis 20 Gew.-%, jeweils bezogen auf die Gesamtmenge an Riech- und/oder Aromastoffen.

Regelmäßig weiter bevorzugt sind erfindungsgemäße Riech- oder Aromastoffkompositionen, die eine Gesamtmenge an erfindungsgemäßen bzw. erfindungsgemäß zu verwendenden Verbindungen der Formeln (I), (I-A) und/oder (II) im Bereich von 0,0001 bis 15 Gew.-%, vorzugsweise 0,001 bis 10 Gew.-% und besonders bevorzugt 0,01 bis 5 Gew.-%, umfassen, bezogen auf die Gesamtmenge der Riech- oder Aromastoffkomposition.

Sofern die (eine oder mehrere) erfindungsgemäßen bzw. erfindungsgemäß zu verwendenden Verbindungen der Formel (I) hauptsächlich eingesetzt werden, um einer Riech- oder Aromastoffkomposition mehr Fülle oder Mundfülle, Frische, (Strahl)Kraft, Ausstrahlung, Glanz, Abrundung und/oder Natürlichkeit zu verleihen und/oder bestimmte Noten zu zu verstärken, insbesondere Noten der Richtungen blumig (dabei insbesondere rosig, indolig), ledrig, fruchtig, frisch, cremig, holzig, moosig und/oder grün, ist der Anteil an Verbindungen der Formel (I) vorzugsweise recht niedrig und vorzugsweise im Bereich von 0,0005 bis 2 Gew.-%, bevorzugt im Bereich von 0,001 bis 1 Gew.-%, weiter bevorzugt im Bereich von 0,01 bis 0,5 Gew.-%, bezogen auf die Gesamtmenge der Riech- oder Aromastoffkomposition. Sofern innerhalb der bevorzugten Konzentrationsbereiche eine vergleichsweise niedrige Konzentration gewählt wird, kommt es in Abhängigkeit von den weiteren Komponenten der jeweiligen Komposition in manchen Fällen noch nicht zu der Vermittlung der oben angegebenen Eigengeruchs- oder - geschmacksnoten.

Riech- oder Aromastoffkompositionen, die eine oder mehrere erfindungsgemäße bzw. erfindungsgemäß zu verwendende Verbindungen der Formel (I) enthalten, können in flüssiger Form, unverdünnt oder mit einem Lösungsmittel verdünnt zur Parfümierung oder Aromatisierung eingesetzt werden.

Bevorzugte Lösungsmittel hierfür sind Ethanol, Isopropanol, Diethylenglycolmonoethyleter, Glycerin, Propylenglycol, 1,2-Butylenglycol, Dipropylenglycol, Diethylphtalat, Triethylcitrat, Isopropylmyristat, Triacetin und Pflanzenöle.

Des Weiteren können die erfindungsgemäßen Riech- oder Aromastoffkompositionen (wie oben beschrieben) an einem Trägerstoff adsorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate und Gasbeton oder organische Materialien wie Hölzer und Cellulose-basierende Stoffe sein.

Erfindungsgemäße Riech- oder Aromastoffkompositionen (wie oben beschrieben) können auch mikroverkapselt, sprühgetrocknet, als Einschluss-Komplexe oder als Extrusions-Produkte vorliegen und in dieser Form vorzugsweise einem zu parfümierenden Produkt hinzugefügt werden.

Gegebenenfalls können die Eigenschaften der derart modifizierten Riech- und/oder Aromastoffkompositionen durch sogenanntes "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Eine Mikroverkapselung erfindungsgemäßer Riech- und/oder Aromastoffkompositionen kann beispielsweise durch das sogenannte Koazervationsverfahren mit Hilfe von Kapselmaterialien z.B. aus polyurethan-artigen Stoffen oder Weichgelatine, erfolgen. Die sprühgetrockneten Riech- und/oder Aromastoffkompositionen können beispielsweise durch Sprühtrocknung einer die Riech- und/oder Aromastoffkompositionen enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z.B. durch Mischen von erfindungsgemäßen Riech- und/oder Aromastoffkompositionen und Cyclodextrinen oder Harnstoffderivaten in einem geeigneten Lösungsmittel, z.B. Wasser, hergestellt werden. Extrusions-Produkte können durch Verschmelzen der (erfindungsgemäßen) Riech- und/oder Aromastoffkompositionen mit einem geeigneten extrudierbaren Stoff und durch Extrusion mit nachfolgender Erstarrung, gegebenenfalls in einem geeigneten Lösungsmittel (z.B. Isopropanol) erfolgen.

Die erfindungsgemäßen Artikel enthaltend eine oder mehrere erfindungsgemäße bzw. erfindungsgemäß zu verwendende Verbindungen der Formel (I), werden gemäß einer bevorzugten Ausgestaltung hergestellt, indem die Verbindungen der Formel (I) als Substanz, als Lösung (z.B. in Ethanol, Wasser oder 1,2-Propylenglycol) oder in Form eines Gemisches mit einem festen oder flüssigen Trägerstoff (z.B. Maltodextrin, Stärke, Silicagel), sonstigen Aromen oder Aromastoffen und gegebenfalls weiteren Hilfsmitteln und/oder Stabilisatoren (z.B. natürliche oder künstliche Polysaccharide und/oder Pflanzengummen wie modifizierte Stärken oder Gummi Arabicum) in eine der Ernährung, der Mundpflege oder dem Genuss dienende Basis-Zubereitung eingearbeitet werden. Vorteilhafterweise können als Lösung und/oder Suspension oder Emulsion vorliegende erfindungsgemäße Zubereitungen auch durch Sprühtrocknung in eine feste erfindungsgemäße Zubereitung (Halbfertigware) überführt werden.

Die erfindungsgemäßen sprühgetrockneten festen Zubereitungen (als Beispiel erfindungsgemäßer Artikel) sind als Halbfertigwaren besonders gut zur Herstellung von weiteren erfindungsgemäßen Zubereitungen geeignet. In den erfindungsgemäßen sprühgetrockneten festen Zubereitungen sind vorzugsweise 50 bis 95 % Gew.-% Trägerstoffe, insbesondere Maltodextrin und/oder Stärke, 5 bis 40 % Hilfsstoffe, bevorzugt natürliche oder künstliche Polysaccharide und/oder Pflanzengummen wie modifizierte Stärken oder Gummi Arabicum enthalten.

Gemäß einer weiteren bevorzugten Ausführungsform werden zur Herstellung erfindungsgemäßer Zubereitungen ein oder mehrere erfindungsgemäße bzw. erfindungsgemäß zu verwendende Verbindungen der Formel (I) und gegebenenfalls andere Bestandteile der erfindungsgemäßen Zubereitung zunächst in Emulsionen, in Liposomen, z.B. ausgehend von Phosphatidylcholin, in Microsphären, in Nanosphären oder auch in Kapseln, Granulaten oder Extrudaten aus einer für Lebens- und Genussmittel geeigneten Matrix, z.B. aus Stärke, Stärkederivaten (z.B. modifizierte Stärke), Cellulose oder Cellulosederivaten (z.B. Hydroxypropylcellulose), anderen Polysacchariden (z.B. Dextrin, Alginat, Curdlan, Carageenan, Chitin, Chitosan, Pullulan), natürlichen Fetten, natürlichen Wachsen (z.B. Bienenwachs, Carnaubawachs), aus Proteinen, z.B. Gelatine oder sonstigen Naturprodukten (z.B. Schellack) eingearbeitet. Dabei können je nach Matrix die Produkte durch Sprühtrocknung, Sprühgranulation, Schmelzgranulation, Koazervation, Koagulation, Extrusion, Schmelzextrusion, Emulsionsverfahren, Beschichtung (Coating) oder andere geeignete Verkapselungsverfahren und gegebenenfalls eine geeignete Kombination der vorgenannten Verfahren erhalten werden. In einem weiteren bevorzugten Herstellungsverfahren für eine erfindungsgemäße Zubereitung wird bzw. werden erfindungsgemäße(n) bzw. erfindungsgemäß zu verwendende(n) Verbindung(en) der Formel (I) zunächst mit einem oder mehreren geeigneten Komplexbildnern, beispielsweise mit Cyclodextrinen oder Cyclodextrinderivaten, bevorzugt α- oder β-Cyclodextrin, komplexiert und in dieser komplexierten Form eingesetzt.

Erfindungsgemäße Riech- oder Aromastoffkompositionen werden vorzugsweise zur Herstellung parfümierter oder aromatisierter Produkte (Artikel) eingesetzt.

Die erfindungsgemäßen Riechstoffkompositionen (wie oben beschrieben) können vorteilhafterweise in konzentrierter Form, in Lösungen oder in wie oben beschriebener modifizierter Form für die Herstellung von parfümierten oder aromatisierten Artikeln verwendet werden.

Die erfindungsgemäßen bzw. erfindungsgemäß zu verwendenden Verbindungen der Formel (I) können in aromatisierte oder zu aromatisierende Produkte eingearbeitet werden, insbesondere der Ernährung, dem Genuss oder der Mundpflege dienende Zubereitungen.

In einem weiteren Aspekt betrifft die vorliegende Erfindung einen parfümierten und/oder aromatisierten Artikel, umfassend eine erfindungsgemäße Riechstoff- und/oder Aromastoffkomposition, vorzugsweise in einer der als bevorzugt oder besonders bevorzugt gekennzeichneten Ausgestaltungen, oder eine erfindungsgemäße bzw. erfindungsgemäß einzusetzende Verbindung der Formel (I), (I-A) oder (II), vorzugsweise in einer der als bevorzugt oder besonders bevorzugt gekennzeichneten Ausgestaltungen

Bevorzugte erfindungsgemäße Artikel sind dabei parfümierte Artikel, ausgewählt aus der Gruppe bestehend aus Waschmitteln, Hygiene- oder Pflegeprodukten, insbesondere im Bereich der Körper- und Haarpflege, der Kosmetik und des Haushalts, und aromatisierte Artikel, ausgewählt aus der Gruppe bestehend aus Lebensmitteln, Genussmitteln, Getränken, Mundpflegeprodukten (d.h. Mundhygieneprodukten) oder pharmazeutischen Produkten.

Bevorzugte erfindungsgemäße parfümierte und/oder aromatisierte Artikel (wie oben beschrieben) enthalten die erfindungsgemäßen bzw. erfindungsgemäß zu verwendenden Verbindungen der Formel (I) in einer Gesamtmenge von 0,0000005 Gew.-% bis 5 Gew.-%, bevorzugt von 0,00005 Gew.-% bis 2 Gew.-% besonders bevorzugt von 0,0001 Gew.-% bis 0,5 Gew.-% bezogen auf das Gesamtgewicht des Artiekls.

Weitere übliche Grund-, Hilfs- und Zusatzstoffe für die jeweiligen erfindungsgemäßen Artikel sind vorzugsweise in Mengen von bis zu 99,99 Gew.-%, bevorzugt von 10 bis 95 Gew.-%, bezogen auf das Gesamtgewicht des Artikels, enthalten. Ferner können die erfindungsgemäßen Artikel Wasser in einer Menge von bis zu 99 Gew.-%, vorzugsweise von 5 bis 80 Gew.-% umfassen, bezogen auf das Gesamtgewicht des Artikels.

Dementsprechend betrifft die vorliegende Erfindung auch einen parfümierten Artikel, umfassend eine erfindungsgemäße Riech- oder Aromastoffkomposition, vorzugsweise eine erfindungsgemäße Parfümkomposition (Parfümöl). Bevorzugte parfümierte bzw. zu parfümierende Artikel sind Parfüm-Extraits, Eau de Parfums, Eau de Toilettes, Rasierwässer, Eau de Colognes, Preshave-Produkte, Splash-Colognes und parfümierte Erfrischungstücher saure, alkalische und neutrale Reinigungsmittel, wie z.B. Fußbodenreiniger, Fensterglasreiniger, Geschirrspülmittel, Bad- und Sanitärreiniger, Scheuermilch, feste und flüssige WC-Reiniger, pulver- und schaumförmige Teppichreiniger, flüssige Waschmittel, pulverförmige Waschmittel, Wäschevorbehandlungsmittel wie Bleichmittel, Einweichmittel und Fleckenentferner, Wäscheweichspüler, Waschseifen, Waschtabletten, Desinfektionsmittel, Oberflächendesinfektionsmittel sowie Luftverbesserer in flüssiger, gelartiger oder auf einem festen Träger aufgebrachter Form, Aerosolspray, Wachse und Polituren wie Möbelpolituren, Fußbodenwachse, Schuhcremes, sowie Körperpflegemittel wie z.B. feste und flüssige Seifen, Duschgele, Shampoos, Rasierseifen, Rasierschäume, Badeöle, kosmetische Emulsionen vom Öl-in-Wasser-, Wasser-in-Öl- und Wasser-in-Öl-in-Wasser-Typ wie z.B. Hautcremes- und -lotionen, Gesichtscremes und - lotionen, Sonnenschutzcremes- und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukte wie z.B. Haarsprays, Haargele, festigende Haarlotionen, Haarspülungen, permanente und semipermanente Haarfärbemittel, Haarverformungsmittel wie Kaltwellen- und Haarglättungsmitteln, Haarwässer, Haarcremes und -lotionen, Deodorantien und Antiperspirantien wie z.B. Achselsprays, Roll-ons, Deosticks, Deocremes, sowie Produkte der dekorativen Kosmetik wie z.B. Lidschatten, Nagellacke, Make-ups, Lippenstifte, Mascara sowie Kerzen, Lampenöle, Räucherstäbchen, Insektizide, Repellentien und Treibstoffe.

Die der Ernährung oder dem Genuss dienenden erfindungsgemäßen Zubereitungen sind vorzugsweise Backwaren (z.B. Brot, Trockenkekse, Kuchen, sonstiges Gebäck), Süßwaren (z.B. Schokoladen, Fruchtgummi, Hart- und Weichkaramellen, Kaugummi, Lakritze), alkoholische oder nicht-alkoholische Getränke (z.B. Kaffee, Tee, Kakao, kakaohaltige Getränke, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke, Fleischprodukte (z.B. Schinken, Frischwurst- oder Rohwurstzubereitungen), Eier oder Eiprodukte (Trockenei, Eiweiß, Eigelb), Getreideprodukte (z.B. Frühstückscerealien, Müsliriegel), Milchprodukte (z.B. Milchgetränke, Milcheis, Joghurt, Kefir, Frischkäse, Weichkäse, Hartkäse, Trockenmilchpulver, Molke, Butter, Buttermilch), Fruchtzubereitungen (z.B. Konfitüren, Fruchteis, Fruchtsoßen), Gemüsezubereitungen (z.B. Ketchup, Soßen, Trockengemüse), Knabberartikel (z.B. gebackene oder frittierte Kartoffelchips oder Kartoffelteigprodukte, Extrudate auf Mais- oder Erdnussbasis), Produkte auf Fett- und Ölbasis oder Emulsionen derselben (z.B. Mayonnaise, Remoulade, Dressings), Fertiggerichte und Suppen, Gewürze, Würzmischungen sowie insbesondere Aufstreuwürzungen (Seasonings), die im Snackbereich Anwendung finden. Erfindungsgemäße Zubereitungen können z. B. als Halbfertigware oder als Würzmischung vorliegen. Erfindungsgemäße Zubereitungen können insbesondere als Halbfertigware zur Herstellung weiterer der Ernährung oder dem Genuss dienenden Zubereitungen dienen, insbesondere in sprühgetrockneter Form.

Der Mundpflege dienende erfindungsgemäße Zubereitungen sind insbesondere Mund-und/oder Zahnpflegemittel wie Zahnpasten, Zahngele, Zahnpulver, Mundwässer, Kaugummis und andere Mundpflegemittel.

Substanzen und Hilfsstoffe, welche ein erfindungsgemäßer parfümierter und/oder aromatisierter Artikel, insbesondere eine erfindungsgemäße kosmetische Zubereitung, dabei vorzugsweise topische kosmetische Zubereitung, enthaltend eine oder mehrere Verbindungen der Formel (I), zusätzlich enthalten kann, sind beispielsweise:

Konservierungsmittel, vorzugsweise die in US 2006/0089413 genannten, Abrasiva, Antiakne-Mittel und Mittel zu Sebumreduktion, vorzugsweise die in WO 2008/046791 genannten, Mittel gegen Hautalterung, vorzugsweise die in WO 2005/123101 genannten, antibakterielle Mittel, Anticellulitis-Mittel, Antischuppen-Mittel, vorzugsweise die in WO 2008/046795 genannten, entzündungshemmende Mittel, irritationsverhindernde Mittel, Antiirritantien (antiinflammatorische, irritationshemmende und irritationsverhindernde Mittel), vorzugsweise die in WO 2007/042472 und US 2006/0089413 genannten, antimikrobielle Mittel, vorzugsweise die in WO 2005/123101 genannten, Antioxidantien, vorzugsweise die in WO 2005/123101 genannten, Adstringentien, antiseptische Mittel, Antistatika, Binder, Puffer, Trägermaterialien, vorzugsweise die in WO 2005/123101 genannten, Chelatbildnder, vorzugsweise die in WO 2005/123101 genannten, Zellstimulantien, reinigende Mittel, pflegende Mittel, Enthaarungsmittel, oberflächenaktive Substanzen, deodorierende Mittel und Antiperspirantien, vorzugsweise die in WO 2005/123101 genannten, Weichmacher, Emulgatoren, vorzugsweise die in WO 2005/123101 genannten, Enzyme, ätherische Öle, vorzugsweise die in US 2008/0070825 genannten, Insektenrepellentien, vorzugsweise die in WO 2005/123101 genannten, Fasern, Filmbildner, Fixateure, Schaumbildner, Schaumstabilisatoren, Substanzen zum Verhindern des Schäumens, Schaumbooster, Fungizide, gelierende Mittel und gelbildende Mittel, vorzugsweise die in WO 2005/123101 genannten, Haarpflegemittel, Haarverformungsmittel, Haarglättungsmittel, Feuchtigkeitsregulatoren (feuchtigkeitsspendende, anfeuchtende und/oder feuchthaltende Substanzen), vorzugsweise die in WO 2005/123101 genannten, Osmolyte, vorzugsweise die in WO 2005/123101 genannten, kompatible Solute, vorzugsweise die in WO 01/76572 und WO 02/15686 genannten, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, vorzugsweise die in WO 2008/046676 genannten, Pulver, Proteine und Proteinhydrolysate, vorzugsweise die in WO 2005/123101 und WO 2008/046676 genannten, rückfettende Mittel, abschleifende Mittel, hautberuhigende Mittel, hautreinigende Mittel, hautpflegende Mittel, hautheilende Mittel (skin repair agents), vorzugsweise enthaltend Cholesterin und/oder Fettsäuren und/oder Ceramide und/oder Pseudoceramide, dabei bevorzugt die in WO 2006/053912 genannten, Hautaufhellungsmittel, vorzugsweise die in WO 2007/110415 genannten, hautschützende Mittel, hauterweichende Mittel, hautkühlende Mittel, vorzugsweise die in WO 2005/123101 genannten, hautwärmende Mittel, vorzugsweise die in WO 2005/123101 genannten, Stabilisatoren, UV-absorbierende Mittel und UV-Filter, vorzugsweise die in WO 2005/123101 genannten, Benzyliden-beta-dicarbonylverbindungen, vorzugsweise die in WO 2005/107692 genannten, alpha-Benzoylzimtsäurenitrile, vorzugsweise die in WO 2006/015954 genannten, AhR-Rezeptor-Antagonisten, vorzugsweise die in WO 2007/128723 und WO 2007/060256 genannten, Waschmittel, Weichspüler, suspendierende Mittel, Hautbräunungsmittel, vorzugsweise die in WO 2006/045760 genannten, Verdickungsmittel, Vitamine, vorzugsweise die in WO 2005/123101 genannten, Öle, Wachse und Fette, vorzugsweise die in WO 2005/123101 genannten, Phospholipide, vorzugsweise die in WO 2005/123101 genannten, Fettsäuren (gesättigte Fettsäuren, ein- oder mehrfach ungesättigte Fettsäuren, α-Hydroxysäuren, Polyhydroxyfettsäuren), vorzugsweise die in WO 2005/123101 genannten, Verflüssiger, Farbstoffe und farbschützende Mittel sowie Pigmente, vorzugsweise die in WO 2005/123101 genannten, Antikorrosiva, Alkohole und Polyole, vorzugsweise die in WO 2005/123101 genannten, Tenside, vorzugsweise die in WO 2005/123101 genannten, tierische Extrakte, Hefeextrakte, Extrakte von Algen oder Mikroalgen, Elektrolyte, Verflüssiger, organische Lösungsmittel, vorzugsweise die in WO 2005/123101 genannten, haarwachstumsmodulierende Mittel (haarwachstumsfördend oder haarwachstumshemmend), vorzugsweise die in EP 2168570 und EP 2193785 genannten oder Silikone und Silikonderivate, vorzugsweise die in WO 2008/046676 genannten.

Bevorzugte erfindungsgemäße Produkte sind (a) Parfümöle (als Beispiel für erfindungsgemäße Riechstoffkompositionen) für tensidhaltige Formulierungen, wie z.B. Reinigungsmittel, Waschmittel, Weichspüler sowie Körperpflegemittel sowie (b) die entsprechenden tensidhaltigen Formulierungen selbst.

Besonders bevorzugte erfindungsgemäße parfümierte Artikel sind daher Waschmittel, Hygiene-oder Pflegeprodukte, insbesondere im Bereich der Körper- und Haarpflege, der Kosmetik und des Haushalts.

Die bevorzugten tensidhaltigen Formulierungen, die im Rahmen der vorliegenden Erfindung eingesetzt werden können, umfassen neben den erfindungsgemäßen Riechstoffkompositionen allgemein Substanzen aus der Klasse der anionischen Tenside, wie zum Beispiel Carboxylate, Sulfate, Sulfonate und Phosphate, der kationischen Tenside, wie zum Beispiel quartäre Ammoniumsalze, der amphoteren Tenside, wie zum Beispiel Betaine, und der nichtionischen Tenside, wie zum Beispiel Ethoxylate und Propoxylate.

Unter den anionischen Tensiden sind die Sulfate und Sulfonate bevorzugt. Bei den Sulfaten sind solche mit 12 bis 18 Kohlenstoffatomen und einem Ethoxylierungsgrad von 1 bis maximal 5 bevorzugt. Insbesondere bevorzugt ist Natriumlaurylethersulfat, vorzugsweise mit einem mittleren Ethoxylierungsgrad von 2 bis 4.

Unter den Sulfonaten sind insbesondere die linearen Natriumalkylbenzolsulfonate mit durchschnittlich ca. 12 Kohlenstoffatomen in der Alkylkette, wobei diese aus homologen Resten mit 10 bis 14 Kohlenstoffatomen bestehen ("Dodecylbenzolsulfonat"), bevorzugt.

Aus der Gruppe der nichtionischen Tenside sind die ethoxylierten Fettalkohole, die durch Ethoxylierung von Alkoholen mit 12 bis 18 Kohlenstoffatomen erhalten werden (Fettalkoholethoxylate mit 12 bis 18 C-Atomen), bevorzugt. Der Ethoxylierungsgrad kann dabei in weiten Grenzen variieren, besonders bevorzugt sind jedoch Produkte mit einem durchschnittlichen Ethoxylierungsgrad von 5 bis 10, insbesondere von 7 Mol addiertem Ethylenoxid pro Mol Fettalkohol.

Unter den Betainen sind insbesondere solche vom Säureamidtyp mit der aufgeführten Struktur bevorzugt.

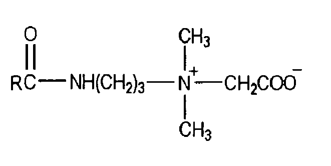

Ein bevorzugter Rest RC=0 ist dabei der Schnitt aus den Fettsäuren des Kokosöls, in dem die Laurylsäure mit 45-50% der Hauptbestandteil ist.

In Kombination mit ausgewählten Tensiden sind die günstigen Eigenschaften des erfindungsgemäßen bzw. erfindungsgemäß einzusetzenden Verbindungen der Formel (I) überraschend stark ausgeprägt. Eine entsprechende erfindungsgemäße Tensidformulierung (als Beispiel eines erfindungsgemäßen Artikels) umfasst eine oder mehrere Verbindungen der Formel (I) und ein oder mehrere Tenside, die ausgewählt sind aus der Gruppe bestehend aus:
- Lineare Alkylbenzolsulfonate (insbesondere die oben genannten, wie z.B. die linearen Natriumalkylbenzolsulfonate),
- Fettalkoholethoxylate mit 12-18 C-Atomen (insbesondere die oben genannten, also z.B. die mit dem oben als bevorzugt gekennzeichneten Ethoxylierungsgrad),
- Laurylethersulfate (insbesondere die oben genannten, also z.B. das oben genannte Natriumlaurylethersulfat) und
- Betaine (insbesondere die oben genannten, also z.B. Betaine vom Säureamidtyp mit der oben aufgeführten Struktur).

Lineare Alkylbenzolsulfonate und Fettalkoholethoxylate mit 12-18 C-Atomen werden dabei vorzugsweise nebeneinander eingesetzt, insbesondere in Vollwaschmittelpulvern.

Ebenso werden Laurylethersulfate (insbesondere das oben genannte Natriumlaurylethersulfat) und Betaine (insbesondere Betaine vom Säureamidtyp mit der oben aufgeführten Struktur) vorzugsweise nebeneinander eingesetzt, insbesondere in Feinwaschmitteln, Shampoos und Duschgelen.

Die Konzentration der oberflächenaktiven Stoffe in den erfindungsgemäßen Tensidformulierungen ist in der Regel unkritisch. Bevorzugte Konzentrationen sind abhängig vom Typ des Tensids und der jeweiligen Anwendung. Sie können zum Beispiel in speziellen Bleichprodukten kleiner 1 Gew.-%, in Seifen oder Waschpulver aber größer 99 Gew.-% sein.

Für bestimmte Anwendungsgebiete sind in erfindungsgemäßen Tensidformulierungen bestimmte Kombinationen und Konzentrationen bevorzugt. So sind erfindungsgemäße Mischungen (Waschmittelformulierungen) bevorzugt, in denen der Anteil an linearen Alkylbenzolsulfonaten im Bereich von 7 - 10 Gew.-% und/oder der Anteil an Fettalkoholethoxylaten mit 12-18 C-Atomen im Bereich von 3 - 6 Gew.-% liegt, jeweils bezogen auf die Gesamtmasse der Mischung. Zudem sind erfindungsgemäße Mischungen (Formulierungen für Feinwaschmittel, Shampoos und Duschgele) bevorzugt, in denen der Anteil an Natriumlaurylethersulfat im Bereich von 7 - 13 Gew.-% und/oder der Anteil an Betain (insbesondere Betain vom Säureamidtyp mit der oben aufgeführten Struktur) im Bereich von 1 - 3 Gew.-% liegt, jeweils bezogen auf die Gesamtmasse der Mischung.

Die erfindungsgemäßen Artikel und Zubereitungen im Sinne der vorliegenden Erfindung können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen. Erfindungsgemäße der Ernährung oder dem Genuss dienende Zubereitungen sind bevorzugt alkoholische oder nicht-alkoholische Getränke, die zudem vorzugsweise einen oder mehrere übliche Zusätze wie Fruchtbestandteile, im Lebensmittelbereich zugelassene anorganische oder organische Säuren, Antioxidantien, Trübungsmittel, Beschwerungsmittel, Süßungsmittel, Aromen, Farbstoffe, Verdickungsmittel, sogenannte "funktionelle" Zutaten und Konservierungsstoffe umfassen.

Als Fruchtbestandteile werden hier vor allem Fruchtaromen, Fruchtsäfte, Fruchtpürees und Fruchtsaftkonzentrate verstanden. Fruchtsäfte bzw. Fruchtsaftkonzentrate, die verwendet werden können, sind solche auf Basis von Zitrusfrüchten, beispielsweise Orange, Zitrone, Grapefruit und Mandarine, und anderen Früchten, beispielsweise Apfel oder Traube. Weiterhin können Fruchtsäfte und Fruchtsaftkonzentrate aus Beerenobst, wie Brombeere, Stachelbeere, Johannisbeere, Heidelbeere, Erdbeere und Himbeere verwendet werden. Weiterhin können Fruchtsäfte und Fruchtsaftkonzentrate aus exotischen Früchten, wie beispielsweise Guave, Papaya, Maracuja, Mango und Banane verwendet werden.

Als Süßungsmittel werden üblicherweise Zucker (insbesondere Saccharose), Süßstoffe, Zuckeraustauschstoffe und Süßungsmittelzusammensetzungen, sowie Mischungen davon eingesetzt.

Geeignete Süßstoffe sind insbesondere Acesulfam-K, Aspartam, Cyclamat (sowie dessen Na-und Ca-Salze), Neohesperidindihydrochalcon, Sucralose und Saccharin (sowie dessen Na-, K-und Ca-Salze). Pflanzliche Süßstoffe können ebenfalls verwendet werden, wie beispielsweise Glycyrrhicin und Thaumatin. Besonders bevorzugt sind Acesulfam-K, Aspartam, Cyclamat, Na-Cyclamat, Saccharin, Na-Saccharin und Sucralose.

Geeignete Zuckeraustauschstoffe sind Zuckeralkohole wie Isomatitol (E 953), Lactitol (E 966), Maltitol, Manitol (E 421), Sorbitol (E 420), Xylitol (E 967) sowie Gemische daraus. Weiterhin können als Süßungsmittel Mischungen aus synthetischem Süßstoff und entaromatisierten oder nicht entaromatisierten konzentrierten Fruchtzubereitungen verwendet werden.

Geeignete Emulgatoren in erfindungsgemäßen Getränken sind beispielsweise Gummi arabicum oder modifizierte Stärken, dabei bevorzugt Stärkenatriumoctenylsuccinat (E 1450).

Erfindungsgemäße alkoholische oder nicht-alkoholische Getränke können zudem Terpen-Öle enthalten. Bevorzugte Terpen-Öle im Rahmen der vorliegenden Erfindung umfassen oder bestehen aus Orangen-, Zitronen- und/oder Grapefruitöl oder daraus erhältlichen Fraktionen, bevorzugt Limonen (insbesondere d-Limonen) und/oder Orangenöl-Terpenen.

Bevorzugt einzusetzende Beschwerungsmittel sind SAIB (Saccharoseacetat-Isobutyrat, E 444), Estergum (E 445) sowie in eingeschränktem Maße Dammar Gum und BVO's (brominated vegetable oils). Diese ermöglichen stabile Getränketrübungen bei Emulsionsgetränken wie beispielsweise in Limonaden oder Fruchtsaftgetränken.

Ein erfindungsgemäßes Getränk enthält regelmäßig eine oder mehrere Genusssäuren. Bevorzugte Genusssäuren sind Citronensäure, Weinsäure, Milchsäure, Phosphorsäure und Äpfelsäure. Der pH-Wert erfindungsgemäßer Emulsionsgetränke liegt regelmäßig im Bereich von 3 bis 5.

Weiterhin können erfindungsgemäße alkoholische oder nicht-alkoholische Getränke Hydrokolloide als Verdickungsmittel enthalten. Geeignete Verdickungsmittel sind Carboxymethylcellulose, Xanthan, Johannisbrotkernmehl, Gellan, Guarkemmehl, Gummi arabicum, Carragen, Alginsäure, Alginate, Pektin sowie Mischungen daraus.

Erfindungsgemäße alkoholische oder nicht-alkoholische Getränke können weiterhin sogenannte "funktionelle" Zutaten enthalten, wie beispielsweise Vitamine (A, B, C, D, E, K), Mineralstoffe, Kräuterextrakte, Ballaststoffe, prebiotische Zutaten, Aminosäuren, Taurin und/oder Coffein.

Erfindungsgemäße alkoholische oder nicht-alkoholische Getränke können mit Lebensmittelfarbstoffen und/oder färbenden Lebensmittel eingefärbt werden (z.B. beta-Carotin).

Geeignete Konservierungsstoffe sind beispielsweise, Sorbinsäure, Benzoesäure und deren Alkalisalze.

Ein erfindungsgemäßes Getränk kann zudem natürliche und synthetische Antioxidantien beinhalten. Geeignete natürliche Antioxidantien sind beispielsweise Tocopherole, L-Ascorbinsäure, ihre Fettsäureester, wie L-Ascorbylpalmitat, Gallussäureester und Flavonoide. Geeignete synthetische Antioxidantien sind beispielsweise tert.-Butylhydroxyanisol und tert.-Butylhydrochinon.

Erfindungsgemäße Zubereitungen können insbesondere Aufstreuwürzungen sein oder darin enthalten sein. Geeignete Aufstreuwürzungen enthalten z.B. synthetische, natürliche oder naturidentische Aromastoffe sowie Trägerstoffe wie z.B. Maltodextrin, Salze wie z.B. Kochsalz, Gewürze wie z.B. Paprika und Pfeffer, Zuckerstoffe oder Zuckeraustauschstoffe bzw. Süßstoffe wie z.B. Saccharin und Geschmacksverstärker wie z.B. Mononatriumglutamat und/oder Inosinmonophosphat.

Die erfindungsgemäßen Zubereitungen (wie oben beschrieben) können hergestellt werden, indem die erfindungsgemäßen bzw. erfindungsgemäß zu verwendenden Verbindungen der Formel (I) als Lösung oder in Form eines Gemisches mit einem festen oder flüssigen Trägerstoff in die der Ernährung, der Mundpflege oder dem Genuss dienende Zubereitung eingearbeitet werden. Vorteilhafterweise können die somit gegebenenfalls als Lösung vorliegenden erfindungsgemäßen Zubereitungen durch Sprühtrocknung in eine feste Zubereitung überführt werden.

Zur Herstellung einer erfindungsgemäßen Zubereitung (wie oben beschrieben) können gemäß einer bevorzugten Ausgestaltung die erfindungsgemäßen bzw. erfindungsgemäß zu verwendenden Verbindungen der Formel (I) bzw. vorzugsweise eine erfindungsgemäße Aromastoffkompositionen (wie oben beschrieben) sowie gegebenenfalls andere Bestandteile der erfindungsgemäßen Zubereitung auch zuvor (d.h. vor der Einarbeitung in die Zubereitung) in Emulsionen, in Liposomen, z.B. ausgehend von Phosphatidylcholin, in Microsphären, in Nanosphären oder auch in Kapseln aus einer für Lebens- und Genussmittel geeigneten Matrix, z.B. aus Stärke, Stärkederivaten, anderen Polysacchariden, natürlichen Fetten, natürlichen Wachsen oder aus Proteinen, z.B. Gelatine, eingearbeitet werden. Eine weitere bevorzugte Ausgestaltung kennzeichnet sich dadurch, dass die erfindungsgemäßen bzw. erfindungsgemäß zu verwendenden Verbindungen der Formel (I), insbesondere eine erfindungsgemäße Aromakomposition (wie oben beschrieben), zuvor mit geeigneten Komplexbildnern, beispielsweise mit Cyclodextrinen oder Cyclodextrinderivaten, bevorzugt beta-Cyclodextrin, komplexiert werden und in dieser Form eingesetzt werden.

Als andere Bestandteile für die erfindungsgemäßen, der Ernährung oder dem Genuss dienenden Zubereitungen können weitere übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Ei, Knochen, Knorpel, Fisch, Krusten- und Schalentiere, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose), Zuckeralkohole (z.B. Sorbit, Mannitol, Xylitol), natürliche oder gehärtete Fette (z.B. Talg, Schmalz, Palmfett, Kokosfett, gehärtetes Pflanzenfett), fette Öle (z.B. Sonnenblumenöl, Erdnussöl, Maiskeimöl, Distelöl, Olivenöl, Walnussöl, Fischöl, Sojaöl, Sesamöl), Fettsäuren oder deren Salze (z.B. Kaliumstearat, Kaliumpalmitat), proteinogene oder nicht-proteinogene Aminosäuren und verwandte Verbindungen (z.B. Taurin, Kreatin, Kreatinin), Peptide, native oder prozessierte Proteine (z.B. Gelatine), Enzyme (z.B. Peptidasen, Glucosidasen, Lipasen), Nukleinsäuren, Nucleotide (Inositolphosphat), geschmacksmodulierende Stoffe (z.B. Natriumglutamat, 2-Phenoxypropionsäure), Emulgatoren (z.B. Lecithine, Diacylglycerole), Stabilisatoren (z.B. Carageenan, Alginat, Johannisbrotkernmehl, Guarkernmehl), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin), Süßstoffe (z.B. Saccharin, Cyclamat, Aspartam, Neotam, Neohesperidindihydrochalkon), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe sowie Geruchskorrigentien.

Gemäß einem weiteren Aspekt der vorliegenden Erfindung werden die erfindungsgemäßen Zubereitungen (wie oben beschrieben) vorzugsweise als Halbfertigwaren zur Aromatisierung von (weiteren) Zubereitungen (zum Beispiel zur Herstellung von Fertigwaren) eingesetzt.

Zahnpflegemittel (als Basis für der Mundpflege dienende erfindungsgemäße Zubereitungen wie oben beschrieben) umfassen im Allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, Süßstoffe, wie z.B. Saccharin, Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin und Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Kaugummis, als bevorzugte Ausgestaltung einer der Mundpflege dienenden erfindungsgemäßen Zubereitung (wie oben beschrieben), umfassen im Allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkoholen, Geschmackskorrigenzien für unangenehme Geschmackseindrücke, andere Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Aromen und Stabilisatoren oder Geruchskorrigentien.

Für die bevorzugt einzusetzenden Verbindungen der Formel (I) bzw. Mischungen bzw. Riech-und Aromastoffkompositionen gilt jeweils das vorangehend Gesagte entsprechend.

Die nachfolgenden Beispiele erläutern die Erfindung. Sofern nicht anders angegeben beziehen sich alle Angaben, insbesondere Anteile, Gehalte und Prozente auf das Gewicht.

Verwendete Abkürzungen: DPG = Dipropylenglycol, TEC = Triethylcitrat, IPM = Isopropylmyristat; BB = Benzoylbenzoat; Krist. = kristallin; Abs. = Absolue.

### Beispiel 1: Synthese von 2-Isobutyl-4-vinyl-[1,3]dioxolan

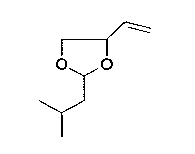

Eine Mischung aus 200 g 2-Buten-1,4-diol (2.27 mol, 1.00 Äquiv.), 196 g Isovaleraldehyd (2.27 mol, 1.00 Äquiv.) und 9.00 g Kupfer(I)chlorid (90.8 mmol, 0.04 Äquiv.) wurde in einer Mischung aus 100 g 55%iger Schwefelsäure und 50 g Cyclohexan bei 70 °C für 2 Stunden erhitzt. Nach Abkühlung wurden die Phasen getrennt, die organische Phase mit Wasser gewaschen und die wässrige Phase erneut abgetrennt. Nach Entfernung des Lösungsmittels wurde das erhaltene Rohprodukt durch eine Destillation über Natriumcarbonat gereinigt. Das Produkt wurde als klare Flüssigkeit (ca. 1:1 - Gemisch der cis- und trans-Isomere) mit einer Ausbeute von 40% der Theorie erhalten. Der Siedepunkt dieses Produktes lag bei 75 °C bei 25 mbar.

Geschmacksprofil dieses Gemisches aus cis- und trans-2-Isobutyl-4-vinyl-[1,3]dioxolan: Olive, fettig, Olivenöl, grün, krautig, haftfest, Mundfülle.

Die beiden Isomere cis- bzw. trans-2-Isobutyl-4-vinyl-[1,3]dioxolan wurden anschließend getrennt und separat geruchlich bewertet.

***Cis*-Isomer**: MS: m/z (%) = 155 (3), 126 (3), 11 (1), 99 (100), 85 (15), 71 (40), 69 (6), 57 (10), 43 (46), 41 (22), 27 (12). - ¹H NMR (400 MHz, CDCl₃): δ (ppm) = 0.96 (d, J = 6.7 Hz, 6 H), 1.58 (dd, J = 7.2, 5.3 Hz, 1 H), 1.59 (dd, J = 6.7, 5.1 Hz, 1 H), 1.84 (sept, J = 6.8 Hz, 1 H), 3.61 (dd, J = 7.9, 6.3 Hz, 1 H), 3.97 (dd, J = 7.9, 7.1 Hz, 1 H), 4.41-4.45 (m, 1 H), 4.98 (t, J = 5.2 Hz, 1 H), 5.20 (ddd, J = 10.3, 1.5, 1.0 Hz, 1 H), 5.34 (ddd, J = 17.1, 1.4, 1.0 Hz, 1 H), 5.84 (ddd, J = 17.1, 10.3, 7.2 Hz, 1 H). - ¹³C NMR (100 MHz, CDCl₃): δ (ppm) = 22.9 (2 x CH₃), 24.6 (CH), 42.9 (CH₂), 69.5 (CH₂), 77.6 (CH), 104.4 (CH), 118.0 (CH₂), 136.1 (CH).

Geruchsbeschreibung: Olive, insbesondere grüne Olive, natürlich, rosige Aspekte.

***Trans*-Isomer**: MS: m/z (%) = 155 (2), 126 (1), 99 (100), 85 (13), 71 (33), 69 (4), 57 (7), 43 (37), 41 (18), 27 (9). - ¹H NMR (400 MHz, CDCl₃): δ (ppm) = 0.95 (d, J = 6.7 Hz, 3 H), 0.96 (d, J = 6.7 Hz, 3 H), 1.55 (dd, J = 7.1, 5.2 Hz, 1 H), 1.56 (dd, J = 6.8, 5.2 Hz, 1 H), 1.84 (sept, J = 6.8 Hz, 1 H), 3.51 (dd, J = 8.3, 7.4 Hz, 1 H), 4.17 (ddd, J = 8.3, 6.4, 0.4 Hz, 1 H), 4.44-4.50 (m, 1 H), 5.05 (t, J = 5.2 Hz, 1 H), 5.21 (ddd, J = 10.3, 1.5, 1.0 Hz, 1 H), 5.33 (ddd, J = 17.1, 1.5, 1.1 Hz, 1 H), 5.83 (ddd, J = 17.1, 10.3, 6.8 Hz, 1 H). - ¹³C NMR (100 MHz, CDCl₃): δ (ppm) = 22.9 (2 x CH₃), 24.5 (CH), 42.9 (CH₂), 70.1 (CH₂), 76.7 (CH), 103.8 (CH), 117.6 (CH₂), 135.8 (CH).

Geruchsbeschreibung: Olive, insbesondere grüne Olive, Knoblauch, fettig.

### Beispiel 2: Synthese von 2-secButyl-4-vinyl-[1,3]dioxolan

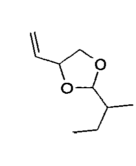

Eine Mischung aus 20.0 g 1-Buten-3,4-diol (227 mmol, 1.00 Äquiv.), 19.5 g 2-Methylbutyraldehyd (227 mmol, 1.00 Äquiv.) und 0.5 g p-Toluolsulfonsäure (0.27 mmol, 0.01 Äquiv.) wurden in 100 mL Cyclohexan 1 Stunde am Wasserabscheider erhitzt. Nach Abkühlung wurde der Ansatz mit 50 mL Wasser versetzt und für 15 Minuten gerührt. Anschließend wurden die Phasen getrennt und die organische Phase über Natriumcarbonat destilliert.

Es wurden 30.6 g 2-secButyl-4-vinyl-[1,3]dioxolan in 99.9% Reinheit isoliert (4 Isomere im Verhältnis 1:1:1:1). Dies entspricht einer Ausbeute von 86% der Theorie.

Siedepunkt: 67 °C / 17.5 mbar. - MS: m/z (%) = 156 (M⁺, 1), 155 (1), 126 (2), 111 (1), 99 (100), 85 (4), 71 (72), 55 (27), 43 (77), 41 (54), 29 (35). - ¹H NMR (400 MHz, CDCl₃): δ (ppm) = 0.90-0.96 (m, 24 H), 1.14-1.26 (m, 8 H), 1.54-1.68 (m, 4 H), 3.52 (dd, J = 8.3, 7.9 Hz, 2 H), 3.57 (dd, J = 7.7, 6.7 Hz, 1 H), 3.57 (dd, J = 7.7, 6.6 Hz, 1 H), 3.99 (dd, J = 7.7, 6.9 Hz, 2 H), 4.15 (dd, J = 8.2, 6.1 Hz, 1 H), 4.16 (dd, J = 8.2, 6.1 Hz, 1 H), 4.40-4.48 (m, 4 H), 4.78 (d, J = 4.3 Hz, 1 H), 4.79 (d, J = 4.3 Hz, 1 H), 4.85 (d, J = 4.3 Hz, 1 H), 4.86 (d, J = 4.3 Hz, 1 H), 5.21 (ddd, J = 10.3, 1.5, 0.9 Hz, 4 H), 5.31-5.38 (m, 4 H), 5.78-5.91 (m, 4 H). - ¹³C NMR (100 MHz, CDCl₃): δ (ppm) = 11.5 (3 x CH₃), 11.6 (CH₃), 13.1 (CH₃), 13.2 (CH₃), 13.3 (2 x CH₃), 24.2 (CH₂), 24.3 (CH₂), 24.4 (CH₂), 26.8 (CH₂), 38.5 (CH), 38.6 (CH), 38.8 (2 x CH), 69.7 (2 x CH₂), 70.3 (2 x CH₂), 77.1 (CH), 77.2 (CH), 77.6 (CH), 77.7 (CH), 107.8 (CH), 108.2 (3 x CH), 117.8 (2 x CH₂), 118.0 (2 x CH₂), 135.8 (4 x CH).

### Beispiel 3: Synthese von 2-Isopropyl-4-vinyl-[1,3]dioxolan

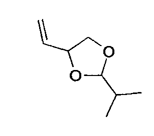

2-Isopropyl-4-vinyl-[1,3]dioxolan wurde in Analogie zu Beispiel 2 synthetisiert, ausgehend von 20.0 g 1-Buten-3,4-diol und 16.4 g Isobutyraldehyd. Es wurden 24.8 g 2-Isopropyl-4-vinyl-[1,3]dioxolan in 99.9% Reinheit isoliert (2 Isomere im Verhältnis 1:1). Dies entspricht einer Ausbeute von 77% der Theorie.

Siedepunkt: 56 °C / 27 mbar. - MS: m/z (%) = 142 (M⁺, 1), 112 (2), 99 (100), 71 (74), 56 (36), 54 (11), 43 (98), 41 (48), 39 (26), 29 (14), 27 (28). ¹H NMR (400 MHz, CDCl₃): δ (ppm) = 0.95 (d, J = 6.9 Hz, 3 H), 0.96 (d, J = 6.9 Hz, 3 H), 0.97 (d, J = 6.9 Hz, 3 H), 0.98 (d, J = 6.9 Hz, 3 H), 1.83 (dd, J = 6.9, 4.6 Hz, 1 H), 1.86 (dd, J = 6.9, 4.6 Hz, 1 H), 3.53 (dd, J = 8.1, 7.6 Hz, 1 H), 3.58 (dd, J = 7.8, 6.7 Hz, 1 H), 3.99 (dd, J = 7.7, 6.6 Hz, 1 H), 4.16 (dd, J = 8.2, 6.2 Hz, 1 H), 4.42-4.49 (m, 2 H), 4.72 (d, J = 4.6 Hz, 1 H), 4.79 (d, J = 4.6 Hz, 1 H), 5.20-5.25 (m, 2 H), 5.31-5.39 (m, 2 H), 5.79-5.91 (m, 2 H). - ¹³C NMR (100 MHz, CDCl₃): δ (ppm) = 16.7 (CH₃), 16.8 (2 x CH₃), 16.9 (CH₃), 32.0 (CH), 32.2 (CH), 69.8 (CH₂), 70.3 (CH₂), 77.2 (CH₂), 77.8 (CH₂), 108.5 (CH), 108.9 (CH), 117.8 (CH₂), 118.0 (CH₂), 135.7 (CH), 135.8 (CH).

### Beispiel 4: Synthese von 2-(2,4-Dimethylcyclohex-3-enyl)-4-vinyl-[1,3]dioxolan

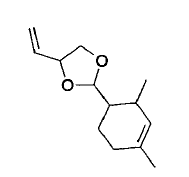

2-(2,4-Dimethylcyclohex-3-enyl)-4-vinyl-[1,3]dioxolan wurde in Analogie zu Beispiel 2 synthetisiert, ausgehend von 20.0 g 1-Buten-3,4-diol und 31.3 g 2,4-Dimethylcyclohex-3-encarbaldehyd. Es wurden 41.2 g 2-(2,4-Dimethylcyclohex-3-enyl)-4-vinyl-[1,3]dioxolan in 98.5% Reinheit isoliert (8 Isomere). Dies entspricht einer Ausbeute von 86% der Theorie.

Siedepunkt: 77 °C / 0.64 mbar. - MS: m/z (%) = 208 (M⁺, 1), 193 (2), 154 (3), 137 (8), 120 (21), 109 (35), 99 (64), 93 (21), 79 (19), 71 (79), 67 (41), 55 (24), 43 (100).

### Beispiel 5: Synthese von 2-(2,6-Dimethylhept-5-enyl)-4-vinyl-[1,3]dioxolan

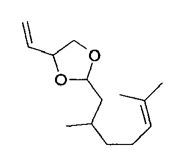

2-(2,6-Dimethylhept-5-enyl)-4-vinyl-[1,3]dioxolan wurde in Analogie zu Beispiel 2 synthetisiert, ausgehend von 20.0 g 1-Buten-3,4-diol und 35.0 g Citronellal. Es wurden 7.3 g 2-(2,6-Dimethylhept-5-enyl)-4-vinyl-[1,3]dioxolan in 94% Reinheit isoliert (4 Isomere im Verhältnis 1:1:1:1). Dies entspricht einer Ausbeute von 13% der Theorie.

Siedepunkt: 68 °C / 0.24 mbar. - MS : m/z (%) = 224 (M⁺, 1), 209 (1), 193 (3), 181 (1), 167 (4), 153 (6), 139 (38), 121 (31), 109 (31), 99 (58), 81 (55), 69 (98), 55 (37), 41 (100). - ¹H NMR (400 MHz, CDCl₃): δ (ppm) = 0.96 (d, J = 6.7 Hz, 12 H), 0.90-1.55 (m, 20 H), 1.60 (s, 12 H), 1.68 (s, 12 H), 1.92-2.05 (m, 8 H), 3.51 (dd, J = 8.3, 7.5 Hz, 1 H), 3.52 (dd, J = 8.3, 7.5 Hz, 1 H), 3.61 (dd, J = 7.8, 7.1 Hz, 1 H), 3.62 (dd, J = 7.8, 7.1 Hz, 1 H), 3.97 (dd, J = 7.9, 7.2 Hz, 1 H), 3.98 (dd, J = 7.9, 7.2 Hz, 1 H), 4.17 (dd, J = 8.2, 6.4 Hz, 1 H), 4.18 (dd, J = 8.2, 6.4 Hz, 1 H), 4.41-4.50 (m, 4 H), 4.98-5.13 (m, 8 H), 5.19-5.24 (m, 4 H), 5.31-5.37 (m, 4 H), 5.79-5.90 (m, 4 H). - ¹³C NMR (100 MHz, CDCl₃): δ (ppm) = 17.6 (2 x CH₃), 18.6 (CH₃), 18.7 (CH₃), 19.6 (CH₃), 19.8 (2 x CH₃), 19.9 (CH₃), 25.4 (4 x CH₂), 25.7 (4 x CH₃), 29.0 (3 x CH), 29.2 (CH), 36.9 (CH₂), 37.4 (CH₂), 37.5 (2 x CH₂), 41.2 (4 x CH₂), 69.5 (CH₂), 69.6 (CH₂), 70.1 (CH₂), 70.2 (CH₂), 77.6 (4 x CH), 103.8 (CH), 103.9 (CH), 104.4 (2 x CH), 117.6 (2 x CH₂), 117.9 (2 x CH₂), 124.2 (CH), 124.7 (3 x CH), 131.2 (4xC_{quart}.), 135.8 (2 x CH), 136.1 (2 x CH).

### Beispiel 6: Synthese von 2-Ethyl-2-(2-methylbutyl)-4-vinyl-[1,3]dioxolan

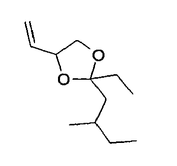

2-Ethyl-2-(2-methylbutyl)-4-vinyl-[1,3]dioxolan wurde in Analogie zu Beispiel 2 synthetisiert, ausgehend von 20.0 g 1-Buten-3,4-diol und 29.1 g 5-Methyl-3-heptanon. Es wurden 28.9 g 2-Ethyl-2-(2-methylbutyl)-4-vinyl-[1,3]dioxolan in 97% Reinheit isoliert (4 Isomere im Verhältnis 1:1:1:1). Dies entspricht einer Ausbeute von 62% der Theorie.

Siedepunkt: 109 °C / 14 mbar. - MS : m/z (%) = 169 (13), 127 (52), 113 (10), 99 (31), 86 (4), 71 (29), 57 (100), 43 (24), 29 (31). - ¹H NMR (400 MHz, CDCl₃): δ (ppm) = 0.86 (2t, J = 7.5 Hz, 6 H), 0.87 (t, J = 7.5 Hz, 3 H), 0.87 (t, J = 7.5 Hz, 3 H), 0.92 (3t, J = 7.5 Hz, 9 H), 0.93 (t, J = 7.5 Hz, 3 H), 0.94 (d, J = 6.4 Hz, 6 H), 0.95 (t, J = 6.4 Hz, 6 H), 1.13-1.24 (m, 4 H), 1.35-1.48 (m, 8 H), 1.50-1.58 (m, 4 H), 1.63-1.72 (m, 12 H), 3.53 (t, J = 8.0 Hz, 1 H), 3.55 (2t, J = 8.0 Hz, 2 H), 3.56 (t, J = 8.1 Hz, 1 H), 4.08 (dd, J = 8.0, 6.3 Hz, 1 H), 4.09 (dd, J = 8.0, 6.3 Hz, 1 H), 4.10 (2dd, J = 8.0, 6.3 Hz, 2 H), 4.43-4.52 (m, 4 H), 5.20 (3ddd, J = 10.3, 1.5, 1.0 Hz, 3 H), 5.21 (ddd, J = 10.3, 1.5, 1.0 Hz, 1 H), 5.34 (3ddd, J = 17.1, 1.4, 1.0 Hz, 3 H), 5.35 (ddd, J = 17.1, 1.4, 1.0 Hz, 1 H), 5.82 (ddd, J = 17.2, 10.3, 7.1 Hz, 2 H), 5.83 (ddd, J = 17.2, 10.3, 7.1 Hz, 2 H). - ¹³C NMR (100 MHz, CDCl₃): δ (ppm) = 8.1 (2 x CH₃), 8.2 (2 x CH₃), 11.3 (2 x CH₃), 11.4 (2 x CH₃), 20.5 (3 x CH₃), 20.7 (CH₃), 30.2 (CH), 30.3 (2 x CH₂), 30.3 (CH), 30.4 (CH), 30.4 (2 x CH₂), 30.6 (CH), 30.7 (2 x CH₂), 30.8 (2 x CH₂), 43.1 (2 x CH₂), 43.4 (CH₂), 43.5 (CH₂), 69.4 (CH₂), 69.5 (2 x CH₂), 69.6 (CH₂), 77.4 (CH), 77.5 (CH), 77.6 (CH), 77.7 (CH), 113.4 (2 x CH), 113.5 (2 x CH), 117.9 (2 x CH₂), 118.0 (CH₂), 118.1 (CH₂), 135.9 (3 x CH), 136.0 (CH).

### Beispiel 7: Synthese von 2-(2,4,4-Trimethylpentyl)-4-vinyl-[1,3]dioxolan

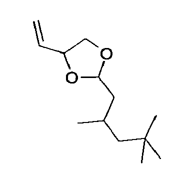

2-(2,4,4-Trimethylpentyl)-4-vinyl-[1,3]dioxolan wurde in Analogie zu Beispiel 2 synthetisiert, ausgehend von 20.0 g 1-Buten-3,4-diol und 32.3 g 3,5,5-Trimethylhexanal. Es wurden 41.2 g 2-(2,4,4-Trimethylpentyl)-4-vinyl-[1,3]dioxolan in 97% Reinheit isoliert (4 Isomere im Verhältnis 1:1:1:1). Dies entspricht einer Ausbeute von 83% der Theorie.

Siedepunkt: 62 °C / 0.88 mbar. - MS : m/z (%) = 138 (1), 109 (12), 99 (100), 83 (19), 71 (40), 57 (33), 43 (38), 29 (12). - ¹H NMR (400 MHz, CDCl₃): δ (ppm) = 0.90 (3s, 36 H), 0.99 (4d, J = 6.6 Hz, 12 H), 1.08 (dd, J = 14.0, 6.1 Hz, 2 H), 1.09 (dd, J = 14.0, 6.1 Hz, 2 H), 1.27 (dd, J = 14.0, 3.9 Hz, 2 H), 1.28 (dd, J = 14.0, 3.9 Hz, 2 H), 1.45-1.58 (m, 4 H), 1.62-1.71 (m, 4 H), 1.71-1.81 (m, 4 H), 3.51 (dd, J = 8.3, 7.4 Hz, 2 H), 3.61 (dd, J = 7.9, 6.4 Hz, 1 H), 3.61 (dd, J = 7.9, 6.3 Hz, 1 H), 3.96 (dd, J = 7.8, 7.0 Hz, 1 H), 3.97 (dd, J = 7.9, 7.0 Hz, 1 H), 4.17 (dd, J = 8.3, 6.4 Hz, 1 H), 4.17 (ddd, J = 8.2, 6.5, 1.4 Hz, 1 H), 4.40-4.45 (m, 2 H), 4.44-4.51 (m, 2 H), 4.96 (dd, J = 5.7, 4.5 Hz, 1 H), 4.97 (t, J = 5.0 Hz, 1 H), 5.03 (dd, J = 5.6, 4.7 Hz, 1 H), 5.04 (t, J = 5.1 Hz, 1 H), 5.20 (2ddd, J = 10.3, 1.5, 1.1 Hz, 2 H), 5.21 (ddd, J = 10.3, 1.5, 1.1 Hz, 1 H), 5.22 (ddd, J = 10.3, 1.5, 1.1 Hz, 1 H), 5.33 (2ddd, J = 17.2, 1.5, 1.1 Hz, 2 H), 5.34 (2ddd, J = 17.2, 1.5, 1.1 Hz, 2 H), 5.82 (2ddd, J = 17.2, 10.3, 7.2 Hz, 2 H), 5.84 (ddd, J = 17.2, 10.3, 7.2 Hz, 1 H), 5.85 (ddd, J = 17.2, 10.3, 7.2 Hz, 1 H). - ¹³C NMR (100 MHz, CDCl₃): δ (ppm) = 22.9 (CH₃), 23.0 (3 x CH₃), 25.8 (4 x CH), 30.0 (12 x CH₃), 31.2 (4 x C_{quart.}), 43.5 (3 x CH₂), 43.6 (CH₂), 51.4 (CH₂), 51.5 (3 x CH₂), 69.5 (CH₂), 69.6 (CH₂), 70.1 (2 x CH₂), 76.6 (CH), 76.7 (CH), 77.5 (CH), 77.6 (CH), 103.8 (CH), 103.9 (CH), 104.4 (2 x CH), 117.4 (CH₂), 117.6 (CH₂), 117.8 (CH₂), 117.9 (CH₂), 135.8 (2 x CH), 136.1 (2 x CH).

### Beispiel 8: Synthese von 2-(3,5-Dimethylhex-4-enyl)-2-methyl-4-vinyl-[1,3]dioxolan

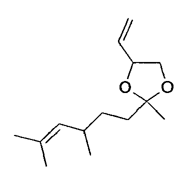

2-(3,5-Dimethylhex-4-enyl)-2-methyl-4-vinyl-[1,3]dioxolan wurde in Analogie zu Beispiel 2 synthetisiert, ausgehend von 20.0 g 1-Buten-3,4-diol und 35.0 g 5,7-Dimethyloct-6-en-2-on. Es wurden 35.5 g 2-(3,5-Dimethylhex-4-enyl)-2-methyl-4-vinyl-[1,3]dioxolan in 99.4% Reinheit isoliert (4 Isomere im Verhältnis 1:1:1:1). Dies entspricht einer Ausbeute von 69% der Theorie.

Siedepunkt: 104 °C / 7.7 mbar. - MS: m/z (%) = 209 (2), 153 (6), 123 (9), 113 (38), 96 (40), 83 (13), 71 (15), 55 (25), 43 (100), 29 (6). - ¹H NMR (400 MHz, CDCl₃): δ (ppm) = 0.91 (d, J = 6.7 Hz, 6 H), 0.92 (d, J = 6.7 Hz, 6 H), 1.20-1.65 (m, 16 H), 1.32 (s, 6 H), 1.35 (s, 6 H), 1.59 (2s, 12 H), 1.67 (2s, 12 H), 2.23-2.35 (m, 4 H), 3.53 (t, J = 7.9 Hz, 1 H), 3.54 (t, J = 8.0 Hz, 1 H), 3.59 (t, J = 8.2 Hz, 2 H), 4.08 (dd, J = 8.0, 6.2 Hz, 1 H), 4.09 (dd, J = 8.0, 6.2 Hz, 1 H), 4.09 (dd, J = 8.2, 6.2 Hz, 1 H), 4.10 (dd, J = 8.2, 6.2 Hz, 1 H), 4.41-4.48 (m, 2 H), 4.48-4.54 (m, 2 H), 4.85-4.89 (m, 4 H), 5.19-5.23 (m, 4 H), 5.31-5.38 (m, 4 H), 5.82 (ddd, J = 17.1, 10.3, 7.2 Hz, 2 H), 5.82 (ddd, J = 17.3, 10.1, 7.2 Hz, 1 H), 5.83 (ddd, J = 17.3, 10.1, 7.2 Hz, 1 H). - ¹³C NMR (100 MHz, CDCl₃): δ (ppm) = 18.0 (4 x CH₃), 21.4 (4 x CH₃), 25.8 (4 x CH₃), 31.9 (2 x CH₂), 32.0 (2 x CH₂), 32.6 (4 x CH), 37.4 (2 x CH₂), 37.8 (2 x CH₂), 69.3 (2 x CH₂), 69.5 (CH₂), 69.6 (CH₂), 77.1 (CH), 77.2 (CH), 77.8 (2 x CH), 111.2 (2 x C_{quart.}), 111.3 (2 x C_{quart.}), 118.0 (2 x CH₂), 118.1 (2 x CH₂), 130.1 (4 x C_{quart.}), 131.1 (4xC_{quart.}), 135.7 (2 x CH), 136.1 (2 x CH).

### Beispiel 9: Synthese von 2-Methyl-2-(4-methylpent-3-enyl)-4-vinyl-[1,3]dioxolan

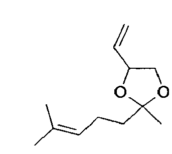

2-Methyl-2-(4-methylpent-3-enyl)-4-vinyl-[1,3]dioxolan wurde in Analogie zu Beispiel 2 synthetisiert, ausgehend von 20.0 g 1-Buten-3,4-diol und 28.6 g 6-Methylhept-5-en-2-on. Es wurden 36.5 g 2-Methyl-2-(4-methylpent-3-enyl)-4-vinyl-[1,3]dioxolan in 96% Reinheit isoliert (2 Isomere im Verhältnis 1:1). Dies entspricht einer Ausbeute von 78% der Theorie.

Siedepunkt: 80 °C / 1.4 mbar. - MS: m/z (%) = 196 (M⁺, 2), 125 (4), 113 (30), 108 (4), 93 (3), 83 (5), 69 (14), 55 (8), 43 (100), 27 (5). - ¹H NMR (400 MHz, CDCl₃): δ (ppm) = 1.35 (s, 3 H), 1.38 (s, 3 H), 1.60-1.63 (m, 6 H), 1.67-1.69 (m, 6 H), 2.14-2.3 (m, 8 H), 3.55 (t, J = 8.0 Hz, 1 H), 3.61 (t, J = 8.1 Hz, 1 H), 4.11 (dd, J = 8.0, 6.2 Hz, 1 H), 4.12 (dd, J = 8.1, 6.2 Hz, 1 H), 4.46 (ddd, J = 8.1, 6.2, 1.0 Hz, 1 H), 4.53 (ddd, J = 8.1, 6.2, 1.0 Hz, 1 H), 5.07-5.15 (m, 2 H), 5.21 (ddd, J = 10.3, 1.5, 0.8 Hz, 1 H), 5.22 (ddd, J = 10.3, 1.5, 0.9 Hz, 1 H), 5.35 (ddd, J = 17.1, 1.4, 1.0 Hz, 1 H), 5.36 (ddd, J = 17.1, 1.4, 1.0 Hz, 1 H), 5.83 (ddd, J = 17.1, 10.2, 7.1 Hz, 1 H), 5.83 (ddd, J = 17.1, 10.3, 7.1 Hz, 1 H). - ¹³C NMR (100 MHz, CDCl₃): δ (ppm) = 17.6 (2 x CH₃), 22.7 (CH₂), 22.8 (CH₂), 24.2 (CH₃), 24.8 (CH₃), 25.7 (2 x CH₃), 39.3 (CH₂), 39.8 (CH₂), 69.3 (CH₂), 69.6 (CH₂), 77.2 (CH), 77.9 (CH), 110.8 (C_{quart.}), 110.9 (C_{quart.}), 118.1 (2 x CH₂), 124.0 (CH), 124.1 (CH), 131.6 (C_{quart.}), 131.7 (C_{quart.}), 135.6 (CH), 136.1 (CH).

### Beispiel 10: Synthese von 8-tertButyl-2-vinyl-1,4-dioxaspiro-[4.5]-decan

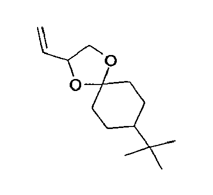

8-*tert*Butyl-2-vinyl-1,4-dioxaspiro-[4.5]-decan wurde in Analogie zu Beispiel 2 synthetisiert, ausgehend von 20.0 g 1-Buten-3,4-diol und 35.0 g 4-tertButylcyclohexanon. Es wurden 43.7 g 8-tertButyl-2-vinyl-1,4-dioxaspiro-[4.5]-decan in 99% Reinheit isoliert (2 Isomere im Verhältnis 1:1). Dies entspricht einer Ausbeute von 79% der Theorie.

Siedepunkt: 82 °C / 0.95 mbar. - MS: m/z (%) = 194 (1), 167 (2), 153 (18), 139 (5), 125 (100), 112 (3), 97 (5), 84 (6), 69 (12), 55 (94), 41 (28), 29 (6). -¹H NMR (400 MHz, CDCl₃): δ (ppm) = 0.86 (s, 18 H), 0.98-1.10 (m, 2 H), 1.21-1.63 (m, 16 H), 3.59 (dd, J = 8.2, 7.4 Hz, 1 H), 3.60 (t, J = 7.7 Hz, 1 H), 4.09 (dd, J = 8.1, 6.3 Hz, 2 H), 5.46-5.54 (m, 2 H), 5.18-5.22 (m, 2 H), 5.34 (ddd, J = 17.2, 1.5, 1.0 Hz, 1 H), 5.35 (ddd, J = 17.2, 1.5, 1.0 Hz, 1 H), 5.82 (ddd, J = 17.2, 10.1, 7.0 Hz, 1 H), 5.84 (ddd, J = 17.2, 10.3, 7.0 Hz, 1 H). - ¹³C NMR (100 MHz, CDCl₃): δ (ppm) = 24.7 (2 x CH₂), 24.8 (CH₂), 24.9 (CH₂), 27.7 (6 x CH₃), 32.3 (2 x C_{quart.}), 35.3 (CH₂), 35.7 (CH₂), 36.1 (CH₂), 36.6 (CH₂), 47.1 (CH), 47.4 (CH), 68.9 (CH₂), 69.0 (CH₂), 76.7 (CH), 77.2 (CH), 110.0 (2 x C_{quart.}), 117.7 (CH₂), 117.9 (CH₂), 136.3 (CH), 136.4 (CH).

### Beispiel 11: Synthese von 2-(2,6-Dimethylhepta-1,5-dienyl-4-vinyl-[1,3]dioxolan

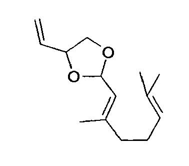

2-(2,6-Dimethylhepta-1,5-dienyl-4-vinyl-[1,3]dioxolan wurde in Analogie zu Beispiel 2 synthetisiert, ausgehend von 20.0 g 1-Buten-3,4-diol und 34.5 g Citral. Es wurden 17.9 g 2-(2,6-Dimethylhepta-1,5-dienyl-4-vinyl-[1,3]dioxolan in 93% Reinheit isoliert (4 Isomere im Verhältnis 1.5:1.5:1:1). Dies entspricht einer Ausbeute von 33% der Theorie.

Siedepunkt: 107 °C / 2.1 mbar. - MS: m/z (%) = 207(1), 179 (1), 165 (6), 152 (11), 135 (7), 121 (11), 109 (18), 99 (21), 81 (15), 69 (87), 55 (26), 41 (100). - ¹H NMR (400 MHz, CDCl₃): δ (ppm) = 1.60-1.61 (m, 15 H), 1.68-1.69 (m, 15 H), 1.77-1.80 (m, 15 H), 2.02-2.20 (m, 20 H), 3.51 (dd, J = 7.9, 7.2 Hz, 1 H), 3.55 (dd, J = 8.3, 7.4 Hz, 1.5 H), 3.65 (dd, J = 7.8, 6.3 Hz, 1 H), 3.66 (dd, J = 7.8, 6.3 Hz, 1.5 H), 4.00 (dd, J = 7.8, 7.0 Hz, 1 H), 4.01 (dd, J = 7.8, 7.0 Hz, 1.5 H), 4.20 (dd, 8.2, 6.0 Hz, 1 H), 4.22 (dd, J = 8.3, 6.3 Hz, 1.5 H), 4.43-4.56 (m, 5 H), 5.07-5.15 (m, 5 H), 5.20-5.40 (m, 15 H), 5.57 (d, J = 7.5 Hz, 1 H), 6.60 (d, J = 7.1 Hz, 1.5 H), 5.64 (d, J = 7.5 Hz, 1 H), 5.67 (d, J = 7.2 Hz, 1.5 H), 5.80-5.92 (m, 5 H). - ¹³C NMR (100 MHz, CDCl₃): δ (ppm) = 17.0 (CH₃), 17.7 (CH₃), 23.6 (CH₃), 25.7 (CH₃), 26.1 (CH₂), 26.9 (CH₂), 32.6 (CH₂), 39.5 (CH₂), 76.8 (CH), 76.9 (CH), 77.7 (CH), 101.1 (CH), 100.4 (CH), 100.6 (CH), 101.0 (CH), 117.5 (CH₂), 117.7 (CH₂), 118.1 (CH₂), 118.2 (CH₂), 120.9 (CH), 122.0 (CH), 123.6 (CH), 123.7 (CH), 131.9 (C_{quart.}), 132.2 (C_{quart.}), 135.6 (CH), 135.7 (CH), 144.7 (C_{quart.}), 144.8 (C_{quart.}), 144.9 (C_{quart.}), 145.1 (C_{quart.}).

### Beispiel 12: Synthese von 2-[2-(4-Methylcyclohex-3-enyl)-propyl]-4-vinyl-[1,3]dioxolan

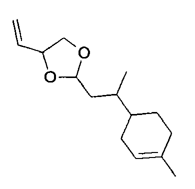

2-[2-(4-Methylcyclohex-3-enyl)-propyl]-4-vinyl-[1,3]dioxolan wurde in Analogie zu Beispiel 2 synthetisiert, ausgehend von 20.0 g 1-Buten-3,4-diol und 37.7 g Limonenal. Es wurden 40.0 g 2-[2-(4-Methylcyclohex-3-enyl)-propyl]-4-vinyl-[1,3]dioxolan in 98% Reinheit isoliert (8 Isomere). Dies entspricht einer Ausbeute von 73% der Theorie.

Siedepunkt: 90 °C / 0.22 mbar. - MS : m/z (%) = 235 (1), 205 (6), 179 (2), 165 (4), 161 (2), 148 (65), 139 (24), 133 (56), 121 (64), 105 (42), 93 (79), 81 (44), 71 (99), 67 (47), 55 (47), 43 (100).

### Beispiel 13: Synthese von 2-Phenyl-4-vinyl-[1,3]dioxolan

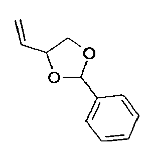

2-Phenyl-4-vinyl-[1,3]dioxolan wurde in Analogie zu Beispiel 2 synthetisiert, ausgehend von 25.0 g 1-Buten-3,4-diol und 30.1 g Benzaldehyd. Es wurden 31.1 g 2-Phenyl-4-vinyl-[1,3]dioxolan in 96% Reinheit isoliert (2 Isomere im Verhältnis 1:1). Dies entspricht einer Ausbeute von 60% der Theorie.

Siedepunkt: 73 °C / 0.70 mbar. - MS: m/z (%) = 176 (M⁺, 3), 175 (16), 145 (7), 131 (3), 117 (24), 105 (78), 99 (5), 91 (28), 77 (46), 71 (10), 63 (7), 54 (100), 51 (24), 39 (19), 27 (13). - ¹H NMR (400 MHz, CDCl₃): δ (ppm) = 3.72 (dd, J = 8.2, 7.3 Hz, 1 H), 3.79 (dd, J = 7.9, 6.7 Hz, 1 H), 4.17 (dd, J = 7.9, 6.9 Hz, 1 H), 4.31 (ddd, J = 8.2 , 6.3, 0.3 Hz, 1 H), 4.61-4.69 (m, 2 H), 5.26 (ddd, J = 10.3, 1.3, 0.9 Hz, 1 H), 5.27 (ddd, J = 10.4, 1.3, 1.1 Hz, 1 H), 5.40 (ddd, J = 17.1, 1.3, 1.0 Hz, 1 H), 5.41 (ddd, J = 17.1, 1.3, 1.1 Hz, 1 H), 5.87 (s, 1 H), 5.92 (ddd, J = 17.1, 10.4, 2.5 Hz, 1 H), 5.94 (ddd, J = 17.2, 10.3, 2.8 Hz, 1 H), 5.98 (s, 1 H), 7.35-7.40 (m, 6 H), 7.47-7.52 (m, 4 H). - ¹³C NMR (100 MHz, CDCl₃): δ (ppm) = 70.0 (CH₂), 70.4 (CH₂), 77.3 (CH), 78.3 (CH), 103.7 (CH), 104.4 (CH), 118.1 (CH₂), 118.4 (CH₂), 126.4 (2 x CH), 126.6 (2 x CH), 128.3 (4 x CH), 129.1 (CH), 129.3 (CH), 135.3 (CH), 135.4 (CH), 137.6 (C_{quart.}), 138.0 (C_{quart.}).

### Beispiel 14: Synthese von 2-Cyclohexyl-4-vinyl-[1,3]dioxolan

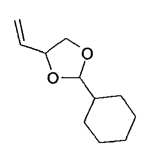

2-Cyclohexyl-4-vinyl-[1,3]dioxolan wurde in Analogie zu Beispiel 2 synthetisiert, ausgehend von 25.0 g 1-Buten-3,4-diol und 31.8 g Cyclohexancarbaldehyd. Es wurden 38.3 g 2-Cyclohexyl-4-vinyl-[1,3]dioxolan in 97% Reinheit isoliert (2 Isomere im Verhältnis 2:1). Dies entspricht einer Ausbeute von 72% der Theorie.

Siedepunkt: 67 °C / 1.6 mbar. - MS : m/z (%)= 152 (1), 134 (1), 126 (1), 108 (1), 99 (100), 81 (10), 71 (39), 67 (9), 55 (23), 43 (32), 27 (10). - ¹H NMR (400 MHz, CDCl₃): δ (ppm) = 1.06-1.28 (m, 15 H), 1.50-1.70 (m, 6 H), 1.72-1.85 (m, 12 H), 3.51 (dd, J = 8.2, 7.5 Hz, 1 H), 3.56 (dd, 7.7, 6.6 Hz, 2 H), 3.98 (dd, J = 7.8, 6.9 Hz, 2 H), 4.14 (dd, J = 8.2, 5.0 Hz, 1 H), 4.39-4.46 (m, 3 H), 4.69 (d, J = 5.0 Hz, 2 H), 4.77 (d, J = 4.9 Hz, 1 H), 5.21 (ddd, J = 10.3, 1.5, 1.0 Hz, 3 H), 5.33 (ddd, J = 17.1, 1.4, 1.2 Hz, 1 H), 5.34 (ddd, J = 17.2, 1.4, 1.0 Hz, 2 H), 5.82 (ddd, J = 17.1, 10.4, 7.0 Hz, 1 H), 5.83 (ddd, J = 17.2, 10.2, 7.2 Hz, 2 H). - ¹³C NMR (100 MHz, CDCl₃): δ (ppm) = 25.7 (CH₂), 25.8 (CH₂), 26.4 (CH₂), 27.2 (CH₂), 27.3 (CH₂), 27.4 (CH₂), 41.8 (CH), 42.0 (CH), 69.7 (CH₂), 70.2 (CH₂), 77.1 (CH), 77.6 (CH), 107.8 (CH), 108.2 (CH), 117.8 (CH₂), 118.0 (CH₂), 135.7 (CH), 135.8 (CH).

### Beispiel 15: Synthese von 2-(2,2,3-Trimethylcyclopent-3-enylmethyl)-4-vinyl-[1,3]dioxolan

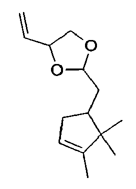

2-(2,2,3-Trimethylcyclopent-3-enylmethyl)-4-vinyl-[1,3]dioxolan wurde in Analogie zu Beispiel 2 synthetisiert, ausgehend von 25.0 g 1-Buten-3,4-diol und 43.2 g Campholenaldehyd. Es wurden 43.8 g 2-(2,2,3-Trimethylcyclopent-3-enylmethyl)-4-vinyl-[1,3]dioxolan in 97% Reinheit isoliert (4 Isomere im Verhältnis 2:2:1:1). Dies entspricht einer Ausbeute von 71% der Theorie.

Siedepunkt: 87 °C / 1.0 mbar. - MS: m/z (%) = 168 (4), 153 (24), 136 (7), 119 (4), 108 (100), 93 (64), 79 (14), 67 (18), 54 (17), 43 (53). - ¹H NMR (400 MHz, CDCl₃): δ (ppm) = 0.76 (s, 6 H), 0.77 (s, 12 H), 0.98 (s, 6 H), 0.99 (s, 12 H), 1.60-1.61 (s, 18 H), 1.65-1.85 (m, 14 H), 1.88-2.02 (m, 10 H), 2.31-2.42 (m, 6 H), 3.52 (dd, J = 8.3, 7.4 Hz, 1 H), 3.53 (dd, J = 8.3, 7.4 Hz, 1 H), 3.62 (dd, J = 7.8, 6.4 Hz, 2 H), 3.63 (dd, J = 7.8, 6.4 Hz, 2 H), 3.98 (dd, J = 7.8, 7.0 Hz, 2 H), 3.99(dd, J = 7.8, 7.0 Hz, 2 H), 4.18 (dd, J = 8.3, 6.4 Hz, 1 H), 4.19 (dd, J = 8.3, 6.4 Hz, 1 H), 4.42-4.53 (m, 6 H), 4.98 (dd, J = 4.0, 0.9 Hz, 2 H), 4.99 (dd, J = 4.6, 0.9 Hz, 2 H), 5.05 (dd, J = 4.1, 1.7 Hz, 1 H), 5.06 (dd, J = 4.1, 1.7 Hz, 1 H), 5.19-5.25 (m, 12 H), 5.33 (ddd, J = 17.1, 1.3, 0.7 Hz, 1 H), 5.34 (ddd, J = 17.1, 1.3, 0.7 Hz, 2 H), 5.34 (dd, J = 17.1, 1.2 Hz, 1 H), 5.34 (dd, J = 17.1, 1.2 Hz, 2 H), 5.83 (ddd, J = 17.1, 10.3, 6.8 Hz, 1 H), 5.84 (ddd, J = 17.1, 10.3, 6.8 Hz, 1 H), 5.85 (ddd, J = 17.2, 10.3, 7.1 Hz, 2 H), 5.86 (ddd, J = 17.1, 10.4, 7.2 Hz, 2 H). - ¹³C NMR (100 MHz, CDCl₃): δ (ppm) = 12.6 (CH₃), 19.7 (CH₃), 25.5 (CH₃), 34.7 (CH₂), 35.7 (CH₂), 35.8 (CH₂), 45.8 (CH), 46.9 (C_{quart.}), 69.5 (CH₂), 69.7 (CH₂), 70.1 (CH₂), 70.2 (CH₂), 76.7 (CH), 76.9 (CH), 77.6 (CH), 77.7 (CH), 104.6 (CH), 104.7 (CH), 105.1 (CH), 105.3 (CH), 117.5 (CH₂), 117.9 (CH₂), 121.8 (CH), 135.8 (CH), 136.0 (CH), 148.2 (C_{quart.}).

### Beispiel 16: Synthese von 2-Vinyl-1,4-dioxaspiro[4.5]decan

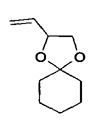

2-Vinyl-1,4-dioxa-spiro[4.5]decan wurde in Analogie zu Beispiel 2 synthetisiert, ausgehend von 25.0 g 1-Buten-3,4-diol und 27.9 g Cyclohexanon. Es wurden 32.9 g 2-Vinyl-1,4-dioxaspiro[4.5]decan in 98% Reinheit isoliert. Dies entspricht einer Ausbeute von 66% der Theorie.

Siedepunkt: 73 °C / 8.9 mbar. - MS: m/z (%) = 168 (M⁺, 11), 139 (13), 125 (56), 112 (14), 97 (7), 84 (8), 69 (10), 55 (100), 41 (18), 27 (9). - ¹H NMR (400 MHz, CDCl₃): δ (ppm) = 1.35-1.45 (m, 2 H), 1.55-1.67 (m, 8 H), 3.60 (dd, J = 8.1, 7.5 Hz, 1 H), 4.01 (dd, J = 8.1, 6.2 Hz, 1 H), 4.50 (dddd, J = 7.5, 7.2, 6.2, 0.9 Hz, 1 H), 5.21 (ddd, J = 10.3, 1.5, 0.9 Hz, 1 H), 5.35 (ddd, J = 17.1, 1.5, 1.0 Hz, 1 H), 5.83 (ddd, J = 17.1, 10.3, 7.2 Hz, 1 H). - ¹³CNMR (100 MHz, CDCl₃): δ (ppm) = 23.9 (CH₂), 24.0 (CH₂), 25.2 (CH₂), 35.5 (CH₂), 36.3 (CH₂), 69.0 (CH₂), 77.0 (CH), 110.0 (C_{quart.}), 117.9 (CH₂), 136.2 (CH).

### Beispiel 17: Synthese von 7-Methyl-2-vinyl-1,4-dioxaspiro[4.5]decan

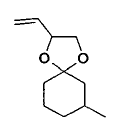

7-Methyl-2-vinyl-1,4-dioxaspiro[4.5]decan wurde in Analogie zu Beispiel 2 synthetisiert, ausgehend von 25.0 g 1-Buten-3,4-diol und 31.8 g 3-Methylcyclohexanon. Es wurden 38.1 g 7-Methyl-2-vinyl-1,4-dioxaspiro[4.5]decan in 96% Reinheit isoliert (4 Isomere im Verhältnis 1:1:1:1). Dies entspricht einer Ausbeute von 71 % der Theorie.

Siedpunkt: 56 °C / 2.1 mbar. - MS: m/z (%) = 182 (M⁺, 5), 167 (2), 152 (2), 139 (64), 125 (18), 11 (15), 95 (15), 84 (7), 69 (100), 55 (66), 41 (30), 27 (11). - ¹H NMR (400 MHz, CDCl₃): δ (ppm)=0.90(d,J=6.4Hz,6H),0.91 (d, J = 6.4 Hz, 3 H), 0.92 (d, J = 6.4 Hz, 3 H), 1.10-1.82 (m, 36 H), 3.56-3.62 (m, 4 H), 4.07-4.13 (m, 4 H), 4.45-4.54 (m, 4 H), 5.17-5.23 (m, 4 H), 5.31-5.38 (m, 4 H), 5.78-5.88 (m, 4 H). - ¹³C NMR (100 MHz, CDCl₃): δ (ppm) = 22.3 (3 x CH₃), 22.4 (CH₃), 23.1 (CH₂), 23.2 (CH₂), 23.3 (CH₂), 23.4 (CH₂), 30.3 (CH), 30.4 (CH), 30.5 (CH), 30.7 (CH), 33.9 (4 x CH₂), 34.6 (CH₂), 34.9 (CH₂), 35.3 (CH₂), 35.9 (CH₂), 43.6 (CH₂), 44.1 (CH₂), 44.4 (CH₂), 44.9 (CH₂), 68.8 (CH₂), 68.9 (CH₂), 69.1 (2 x CH₂), 76.9 (CH), 77.0 (CH), 77.2 (2 x CH), 110.4 (4 x C_{quart.}), 117.8 (CH₂), 117.9 (2 x CH₂), 118.0 (CH₂), 136.1 (CH), 136.2 (2 x CH), 136.3 (CH).

### Beispiel 18: Synthese von 2-(2-Methylpropenyl)-4-vinyl-[1,3]dioxolan

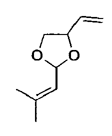

2-(2-Methylpropenyl)-4-vinyl-[1.3]dioxolan wurde in Analogie zu Beispiel 2 synthetisiert, ausgehend von 25.0 g 1-Buten-3,4-diol und 23.9 g 3-Methylbut-2-enal. Es wurden 5.7 g 2-(2-Methylpropenyl)-4-vinyl-[1.3]dioxolan in 96% Reinheit isoliert (2 Isomere im Verhältnis 1:1). Dies entspricht einer Ausbeute von 13% der Theorie.

Siedepunkt: 95 °C / 1.8 mbar. - MS: m/z (%) = 153 (2), 139 (2), 124 (1), 109 (5), 95 (16), 83 (50), 67 (25), 54 (100), 39 (28), 27 (17). - ¹H NMR (400 MHz, CDCl₃): δ (ppm) = 1.76-1.78 (m, 12 H), 3.55 (dd, J = 8.3, 7.5 Hz, 1 H), 3.66 (dd, J = 7.8, 6.4 Hz, 1 H), 4.01 (dd, J = 7.8, 7.0 Hz, 1 H), 4.21 (ddd, J = 8.3, 6.4, 0.4 Hz, 1 H), 4.45-4.51 (m, 1 H), 4.49-4.55 (m, 1 H), 5-21-5.25 (m, 2 H), 5.25-5.30 (m, 2 H), 5.33-5.39 (m, 2 H), 5.58 (d, J = 7.3 Hz, 1 H), 5.65 (d, J = 7.4 Hz, 1 H), 5.81-5.92 (m, 2 H). - ¹³C NMR (100 MHz, CDCl₃): δ (ppm) = 18.4 (2 x CH₃), 25.9 (2 x CH₃), 69.6 (CH₂), 70.2 (CH₂), 76.9 (CH), 77.8 (CH), 100.4 (CH), 100.9 (CH), 117.7 (CH₂), 118.2 (CH₂), 121.5 (CH), 121.6 (CH), 135.6 (CH), 135.8 (CH), 141.5 (2 x C_{quart.}).

### Beispiel 19: Synthese von 2-Methyl-2-(3-methylbutyl)-4-vinyl-[1,3]dioxolan

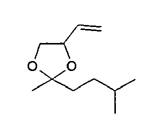

2-Methyl-2-(3-methylbutyl)-4-vinyl-[1,3]dioxolan wurde in Analogie zu Beispiel 2 synthetisiert, ausgehend von 25.0 g 1-Buten-3,4-diol und 23.9 g 5-Methyl-2-hexanon. Es wurden 33.1 g 2-Methyl-2-(3-methylbutyl)-4-vinyl-[1,3]dioxolan in 99% Reinheit isoliert (2 Isomere im Verhältnis 1:1). Dies entspricht einer Ausbeute von 63% der Theorie.

Siedepunkt: 35 °C / 1.0 mbar. - MS: m/z (%) = 169 (3), 154 (1), 128 (3), 113 (64), 99 (8), 85 (12), 71 (19), 55 (9), 43 (100), 29 (6). - ¹H NMR (400 MHz, CDCl₃): δ (ppm) = 0.89 (2d, J = 6.6 Hz, 12 H), 1.26-1.33 (m, 4 H), 1.34 (s, 3 H), 1.37 (s, 3 H), 1.49-1.57 (m, 2 H), 1.62-1.69 (m, 4 H), 3.55 (t, J = 7.9 Hz, 1 H), 3.60 (t, J = 8.1 Hz, 1 H), 4.08-4.12 (m, 2 H), 4.43-4.49 (m, 1 H), 4.49-4.55 (m, 1 H), 5.20-5.24 (m, 2 H), 5.32-5.38 (m, 2 H), 5.78-5.88 (m, 2 H). - ¹³C NMR (100 MHz, CDCl₃): δ (ppm) = 22.5 (CH₃), 22.6 (3 x CH₃), 24.1 (CH₃), 24.8 (CH₃), 28.2 (CH), 28.3 (CH), 32.9 (CH₂), 33.0 (CH₂), 37.3 (CH₂), 37.8 (CH₂), 69.3 (CH₂), 69.6 (CH₂), 77.2 (CH), 77.8 (CH), 111.2 (C_{quart}), 111.3 (C_{quart}), 118.1 (2 x CH₂), 135.6 (CH), 136.0 (CH).

Sofern in den nachfolgenden Beispielen die erfindungsgemäß bevorzugte Verbindung 2-Isobutyl-4-vinyl-[1,3]dioxolan eingesetzt wurde, handelte es sich dabei jeweils um ein 1:1 - Gemisch der cis- und trans-Isomere.

### Beispiel 20: Fine Fragrance

| **Riechstoffe** | **Parfümöl A1** (Vergleich) | **Parfümöl A2** (erfindungsgemäß) |
|---|---|---|
| AMBRAROME 10% in DPG | 20,0 | 20,0 |
| BENZOIN SIAM RESIN 50% in BB | 25,0 | 25,0 |
| BOULEAU ABS. 10% in DPG | 7,0 | 7,0 |
| CEDREN | 15,0 | 15,0 |
| CISTUS LABDANUM ABS. 10% in DPG | 25,0 | 25,0 |
| COUMARIN | 60,0 | 60,0 |
| DAMASCON BETA 10% in DPG | 10,0 | 10,0 |
| DIPROPYLENGLYCOL | 580,0 | 540,0 |
| EUGENOL | 2,0 | 2,0 |
| IONON BETA | 15,0 | 15,0 |
| ISO E SUPER | 100,0 | 100,0 |
| LINALOOL | 15,0 | 15,0 |
| LINALYLACETAT | 20,0 | 20,0 |
| METHYL CYCLOPENTENOLON-3,2,2, NATÜRLICH, 10% in DPG | 3,0 | 3,0 |
| OLIBANUM COEUR 50% in TEC | 30,0 | 30,0 |
| OPOPONAX ÖL 10% in DPG | 10,0 | 10,0 |
| SANDALORE | 10,0 | 10,0 |
| LEDER BASE (SUEDERAL LT) | 3,0 | 3,0 |
| TOLU RESIN 50% in DPG | 15,0 | 15,0 |
| VANILLIN | 20,0 | 20,0 |
| VETIVER ÖL JAVA 10% in DPG | 15,0 | 15,0 |
| **2-ISOBUTYL-4-VINYL-[1,3]DIOXOLAN 10% in DPG** | | **40,0** |
| | 1.000,0 | 1.000,0 |

Durch Zusatz von 2-Isobutyl-4-vinyl-[1,3]dioxolan zur Duftzusammensetzung (bei einer Dosierung von 6% des Parfümöls in Ethanol) wird die ledrige Note verstärkt. Weiterhin verleiht 2-Isobutyl-4-vinyl-[1,3]dioxolan der Komposition mehr Kraft und Fülle.

### Beispiel 21: Bodylotion

| **Komponente** | **Gew.-%** | **Gew.-%** |
|---|---|---|
| Paraffinöl | 5,00 | 5,00 |
| Isopropylpalmitat | 5,00 | 5,00 |
| Cetylalkohol | 2,00 | 2,00 |
| Bienenwachs | 2,00 | 2,00 |
| Ceteareth-20 | 2,00 | 2,00 |
| PEG-20-Glycerylstearat | 1,50 | 1,50 |
| Glycerin | 3,00 | 3,00 |
| Phenoxyethanol | 0,50 | - |
| Parabene (Mischung aus Methyl-, Ethyl-, Propyl-, Butyl-, Isobutylparaben) | - | 0,50 |
| Parfümöl B1 bzw. B2 | 0,50 | 0,50 |
| Wasser | Ad 100 | Ad 100 |

### Parfümöle für Bodylotion des Beispiels 21

| **Riechstoffe** | **Parfümöl B1** (Vergleich) | **Parfümöl B2** (erfindungsgemäß) |
|---|---|---|
| AGRUMEX LC | 5,0 | 5,0 |
| ALDEHYDE C14 SOGENANNT | 2,0 | 2,0 |
| AMAROCIT® | 2,0 | 2,0 |
| AMBERWOOD® F | 10,0 | 10,0 |
| BENZOIN SIAM RESIN 50% IN BB | 3,0 | 3,0 |
| BERGAMOT SYNTHESSENCE AFRIKANISCH | 40,0 | 40,0 |
| ZEDERNHOLZÖL VIRGINIA | 30,0 | 30,0 |
| CEDRAMBER | 15,0 | 15,0 |
| CITRONELLOL | 6,0 | 6,0 |
| COUMARIN | 3,0 | 3,0 |
| DIETHYLMALONAT | 8,0 | 8,0 |
| DIHYDROMYRCENOL | 8,0 | 8,0 |
| ETHYLDECADIENOAT TRANS CIS-2,4 10% in IPM | 10,0 | 10,0 |
| ETHYL VANILLIN | 4,0 | 4,0 |
| ETHYLENBRASSYLAT | 120,0 | 120,0 |
| FLOROSA | 30,0 | 30,0 |
| GERANIOL | 5,0 | 5,0 |
| GERANYLACETAT | 2,0 | 2,0 |
| INGWERÖL CHINESISCH | 2,0 | 2,0 |
| GIVESCONE 10% in DPG | 6,0 | 6,0 |
| HEDIONE | 65,0 | 65,0 |
| HELIONAL | 30,0 | 30,0 |
| HEXENOL CIS-3 10% in DPG | 10,0 | 10,0 |
| HEXENYLACETAT CIS-3 10% in DPG | 20,0 | 20,0 |
| HEXYLACETAT | 3,0 | 3,0 |
| HEXYL SALICYLAT | 65,0 | 65,0 |
| INDOLE 10% in DPG | 3,0 | 3,0 |
| ISO E SUPER | 225,0 | 225,0 |
| LILIAL | 30,0 | 30,0 |
| LINALOOL | 35,0 | 35,0 |
| LINALYLACETAT | 40,0 | 40,0 |
| METHYLANTHRANILAT | 2,0 | 2,0 |
| METHYL NAPHTYL KETON BETA KRIST. | 3,0 | 3,0 |
| NEROLI BASE | 6,0 | 6,0 |
| ORANGENÖL BRASILIANISCH | 60,0 | 60,0 |
| PRENYLACETAT 10% in DPG | 10,0 | 10,0 |
| SANDRANOL® | 10,0 | 10,0 |
| STYRALLYLACETAT 10% in DPG | 8,0 | 8,0 |
| SYMROXANE® | 3,0 | 3,0 |
| TETRAHYDROLINALOOL | 50,0 | 50,0 |
| TUBEROSE ABS. 1% in DPG | 1,0 | 1,0 |
| DIPROPYLENGYCOL (DPG) | 10,0 | |
| **2-ISOBUTYL-4-VINYL-[1,3]DIOXOLAN 0,1% in DPG** | | **10,0** |
| | 1.000,0 | 1.000,0 |

Durch Zusatz von 2-Isobutyl-4-vinyl-[1,3]dioxolan zur Duftzusammensetzung ergibt sich eine natürlichere, stärkere rosige Note. Außerdem wird die indolische Topnote geglättet und der Nachgeruch cremiger.

### Beispiel 22: Weichspüler

| **Material** | **Hersteller** | **Chemischer Name** | **Funktion** | **Gew.-%** |
|---|---|---|---|---|
| Entionisiertes Wasser | | Wasser | Lösungsmittel | 72,4 |
| Rewoquat WE 18 | Evonic Goldschmidt GmbH | Dialkylesterammoniumethosulfate | Kationisches Tensid | 16,6 |
| Mergal K9N | Honeywell Austria GmbH | 5-Chloro-2-methyl-3-(2H)-isothiazolone und 2-methyl-3-(2H)-isothiazolone | Konservierungsmittel | 0,10 |
| Dow Corning 1520 Antifoam | Dow Corning GmbH, Deutschland | Polydimethyl-siloxane | Entschäumer | 0,30 |
| Magnesiumchlorid 1 %ige Lösung | | Magnesium Chlorid Lösung | Konsistenzgeber | 10,00 |
| Parfumöl C1 bzw. C2 | Symrise | | Parfum (Fragrance) | 0,60 |

### Parfümöle für die Weichspüler des Beispiels 22

| **Riechstoffe** | **Parfümöl C1** (Vergleich) | **Parfümöl C2** (erfindungsgemäß) |
|---|---|---|
| ACETESSIGSAEUREETHYLESTER | 15,0 | 15,0 |
| ALDEHYD C14 SOGENANNT | 75,0 | 75,0 |
| ALLYLCAPRONAT | 15,0 | 15,0 |
| BENZALDEHYD | 5,0 | 5,0 |
| BENZYLACETAT | 50,0 | 50,0 |
| CASSIS 345B TYPE BASE | 15,0 | 15,0 |
| CITRONELLOL | 30,0 | 30,0 |
| DECALACTON GAMMA | 60,0 | 60,0 |
| DIPROPYLENGLYCOL (DPG) | 145,0 | 144,0 |
| ETHYLACETAT | 5,0 | 5,0 |
| ETHYLBUTYRAT | 5,0 | 5,0 |
| GLOBALIDE® | 65,0 | 65,0 |
| HEDIONE | 100,0 | 100,0 |
| HERBAFLORAT | 170,0 | 170,0 |
| HEXENOL CIS-3 | 15,0 | 15,0 |
| HEXYLACETAT | 75,0 | 75,0 |
| IONON BETA | 5,0 | 5,0 |
| ISOAMYLACETAT | 3,0 | 3,0 |
| ISORALDEIN 70 | 35,0 | 35,0 |
| LIGUSTRAL | 10,0 | 10,0 |
| LINALOOL | 45,0 | 45,0 |
| METHYLCINNAMAT | 25,0 | 25,0 |
| ROSENOXID | 2,0 | 2,0 |
| SANDRANOL® | 25,0 | 25,0 |
| VANILLIN 10%in DPG | 5,0 | 5,0 |
| **2-ISOBUTYL-4-VINYL-[1,3]DIOXOLAN 10% in DPG** | | **1,0** |
| | 1.000,0 | 1.000,0 |

Durch Zusatz von 2-Isobutyl-4-vinyl-[1,3]dioxolan zur Duftzusammensetzung ergibt sich eine natürlichere, fruchtigere Note.

### Beispiel 23: Seife

| **Material** | **Hersteller** | **Chemischer Name** | **Funktion** | **Gew.-%** |
|---|---|---|---|---|
| Entionisiertes Wasser | | Water | Lösungsmittel | 2,0 |
| Seifen Basen Mix | Verschiedene | Sodium tallowates / palm itates | Tenside | 96,95 |
| Titandioxid | Kronos Titan GmbH, Deutschland | Titanium Dioxide | Farbstoff / Aufheller | 1,0 |
| 2-Isobutyl-4-vinyl-[1,3]dioxolan | Symrise | | Parfum (Fragrance) | 0,05 |

Der Zusatz von 2-Isobutyl-4-vinyl-[1,3]dioxolan ergibt eine schöne natürliche Oliven-Note, die an "Savon de Marseille" erinnert. Darüber hinaus wird der Basengeruch der Seife durch den Anteil an 2-Isobutyl-4-vinyl-[1,3]dioxolan sehr gut überdeckt.

### Beispiel 24: Olivenöl Aromakonzentrat

| | | |
|---|---|---|
| **Aromastoffe** | **Aromakonzentrat D1** (Vergleich) | **Aromakonzentrat D2** (erfindungsgemäß) |
| HEXENOL CIS-3 | 2,0 | 2,0 |
| TERPINEOL ALPHA L KRIST. | 2,0 | 2,0 |
| DECENAL TRANS-2 | 2,0 | 2,0 |
| ETHYLCAPRINAT | 2,0 | 2,0 |
| BUTTERSAEURE | 5,0 | 5,0 |
| ETHYLBUTYRAT | 5,0 | 5,0 |
| CAPRYLSAEURE NATÜRLICH | 5,0 | 5,0 |
| ETHYLLAURAT | 5,0 | 5,0 |
| GUAIACOL | 5,0 | 5,0 |
| ETHYLMETHYLBUTYRAT-2 | 7,0 | 7,0 |
| HEXENAL TRANS-2 10% in TRIACETIN | 10,0 | 10,0 |
| ESSIGSAEURE | 10,0 | 10,0 |
| DECADIENAL TRANS, TRANS-2,4 10% in Pflanzenöl | 10,0 | 10,0 |
| PHENYLETHYLALKOHOL | 20,0 | 20,0 |
| LINALOOL | 10,0 | 10,0 |
| METHYLBUTTERSAEURE-2 | 20,0 | 20,0 |
| DIMETHYLPHENOL-2,5 10% in Ethanol | 200,0 | 200,0 |
| TRIACETIN | 680,0 | 630,0 |
| **2-ISOBUTYL-4-VINYL-[1,3]DIOXOLAN** | | **50,0** |
| | 1.000,0 | 1.000,0 |

Das Aromakonzentrat D2 enthaltend 5% 2-Isobutyl-4-vinyl-[1,3]dioxolan wies eine authentischere, ölige, fettige, leicht grüne Note auf. Diese erinnerte mehr an frisches, kalt gepresstes Olivenöl. Ferner zeichnete sich das erfindungsgemäße Aromakonzentrat D2 durch mehr Fülle und Mundgefühl aus.

### Beispiel 25: Waschpulver

| **Material** | **Hersteller** | **Chemischer Name** | **Funktion** | **Gew.-%** |
|---|---|---|---|---|
| Natrium Metasilicat Pentahydrat | Akzo Nobel Chemicals, Deutschland | Sodium Metasilicate Pentahydrate | | 48,0 |
| Natriumhydrogencarbonat | Verschiedene | Sodium hydrogen carbonate | Alkali | 15,0 |
| Natriumpercarbonat | Verschiedene | Sodium carbonate peroxyhydrate | Bleichmittel | 15,0 |
| Peractive AC Blue | Clariant GmbH, Deutschland | TAED / Na-Carboxymethylcellulose | Aktivator | 5,00 |
| Genapol OA-080 | Clariant GmbH, Deutschland | Oxoalkohol C14-15, 8EO | Nichtionisches Tensid | 3,00 |
| Texapon K12 Pulver | Cognis Deutschland GmbH | Sodium Lauryl Sulphate C12 | Anionisches Tensid | 7,00 |
| Tinopal CBS-X | Ciba, Deutschland | | Aufheller | 0,50 |
| Savinase 6.0 T, Type W | Novozymes | Protease | Enzym | 0,40 |
| Termamyl 120 T | Novozymes | Alpha-Amylase | Enzym | 0,30 |
| Natriumsulfat | Verschiende | Sodium Sulphate | Füllstoff | 5,50 |
| Parfumöl E1 bzw. E2 | Symrise | | Parfum (Fragrance) | 0,30 |

### Parfümöle für die Waschpulver des Beispiels 25:

| **Riechstoffe** | **Parfümöl E1** (Vergleich) | **Parfümöl E2** (erfindungsgemäß) |
|---|---|---|
| ROSMARINÖL | 5,0 | 5,0 |
| DIHYDROMYRCENOL | 140,0 | 140,0 |
| HERBAFLORAT (ESSIGSAEURETRICYCLO[5.2.1.0]-4-DECEN-8-YLESTER) | 50,0 | 50,0 |
| HEXYLZIMTALDEHYD ALPHA | 125,0 | 125,0 |
| CUMARIN | 10,0 | 10,0 |
| DIPHENYLOXID | 5,0 | 5,0 |
| LILIAL^{®} (PROPANAL, 2-METHYL-3-(4-TERT.-BUTYLPHENYL)-) | 40,0 | 40,0 |
| GALAXOLID^{®} 50% in DEP (1,1,2,3,3,8-Hexamethyl-1,2,3,5,7,8-hexahydro-6-oxa-cyclopenta[b]naphthalen) | 150,0 | 150,0 |
| ISORALDEIN^{®} 70 ((E)-3-Methyl-4-(2,6,6-trimethyl-cyclohex-2-enyl)-but-3-en-2-on) | 30,0 | 30,0 |
| ALLYLAMYLGLYCOLAT | 7,0 | 7,0 |
| ISO E SUPER^{®} (BICYCLO[4.4.0]DECEN, 3-ACETYL-3,4,10,10-TETRAMETHYL-1 (6)-) | 80,0 | 80,0 |
| ROSEN BASE | 100,0 | 100,0 |
| GALBANUM BASE | 15,0 | 15,0 |
| DIPROPYLENGLYCOL (DPG) | 5,0 | 4,9 |
| APFEL BASE | 25,0 | 25,0 |
| OZONIL 10% in DPG (DODECENYLCYANID, 1Z-) | 15,0 | 15,0 |
| MELONAL^{®} 10% in DPG (HEPTENAL, 2,6-DIMETHYL-5-) | 3,0 | 3,0 |
| METHYLOCTINCARBONAT 10% in DPG | 5,0 | 5,0 |
| DAMASCON DELTA 10% in DPG (1-(2,6,6-Trimethyl-cyclohex-3-enyl)-but-2-en-1-one) | 7,0 | 7,0 |
| SANDRANOL^{®} (BUTENOL, 2-ETHYL-4-(2,2,3-TRIMETHYL-3-CYCLOPENTENYL)-2E-) | 20,0 | 20,0 |
| VERTOCITRAL (CYCLOHEXEN, TRANS-2,4-DIMETHYL-1-FORMYL-3-) | 3,0 | 3,0 |
| AGRUMEX (ESSIGSAEURE-2-TERT.-BUTYLCYCLOHEXYLESTER) | 100,0 | 100,0 |
| BENZYLACETON | 10,0 | 10,0 |
| ALDEHYD C12 MNA (Methylnonylacetaldehyd) | 5,0 | 5,0 |
| PROJASMON P (CYCLOPENTANON, 2-HEPTYL-) | 10,0 | 10,0 |
| NEROLIN YARA YARA | 15,0 | 15,0 |
| INTRELEVENALDEHYD (10-Undecenal) 10% in DPG | 5,0 | 5,0 |
| PATCHOULIÖL | 15,0 | 15,0 |
| **2-ISOBUTYL-4-VINYL-[1,3]DIOXOLAN** | | **0,1** |
| | 1.000,0 | 1.000,0 |

Bei einer Dosierung von 0.3% der Parfümöl E1 bzw. E2 in einem Waschpulver (powder detergent) ergibt sich folgender Befund: der Anteil von 2-Isobutyl-4-vinyl-[1,3]dioxolan in Parfümöl E2 bewirkt eine Verstärkung der grünen Noten. Weiterhin strahlt die Komposition mit 2-Isobutyl-4-vinyl-[1,3]dioxolan insgesamt mehr Frische aus.

### Beispiel 26: Allzweckreiniger

| **Material** | **Hersteller** | **Chemischer Name** | **Funktion** | **Gew.-%** |
|---|---|---|---|---|
| Entionisiertes Wasser | | Water | Lösungsmittel | 59,6 |
| Mergal K9N | Troy Chemie, Seelze | 5-Chloro-2-methyl-3-(2H)-isothiazolone und 2-methyl-3-(2H)-isothiazolone | Konservierungsmittel | 0,1 |
| Trinatriumcitrat Dihydrat | Verschiedene | Tri Sodium Citrate Dihydrate | Komplexbildner | 3,0 |
| Zetesol NL-2 | Zschimmer & Schwarz, Deutschland | Fatty alcohol C12-14-sulfate, Sodium | Anionisches Tensid | 30,0 |
| Imbentin C/125/055 | Dr. W. Kolb AG Chem. | Fatty alcohol C12-C15, 8EO | Nichtionisches Tensid | 5,0 |
| Ethanol 96% | Verschiedene | Ethanol | Lösungsmittel | 2,0 |
| Parfumöl F1 bzw. F2 | Symrise | | Parfum (Fragrance) | 0,3 |

### Parfümöle für die Allzweckreiniger des Beispiels 26:

| **Riechstoffe** | **Parfümöl F1** (Vergleich) | **Parfümöl F2** (erfindungsgemäß) |
|---|---|---|
| CEDERNHOLZÖL | 8,0 | 8,0 |
| AMBROCENIDE^{®} 10% in DPG | 1,0 | 1,0 |
| ROSMARINÖL | 8,0 | 8,0 |
| DIHYDROMYRCENOL | 80,0 | 80,0 |
| ISOBUTYLCHINOLIN | 0,5 | 0,5 |
| AMBROXAN | 1,0 | 1,0 |
| LIGUSTRAL (CYCLOHEXEN, TRANS-2,4-DIMETHYL-1-FORMYL-3-) | 2,0 | 2,0 |
| AMYLSALICYLAT N/ISO | 24,0 | 24,0 |
| CITRONELLOL | 8,0 | 8,0 |
| VERTOFIX (1-(3,6,8,8-Tetramethyl-2,3,4,7,8,8a-hexahydro-1 H-3a,7-methano-azulen-5-yl)-ethanone) | 30,0 | 30,0 |
| HEXYLZIMTALDEHYD ALPHA | 50,0 | 50,0 |
| CUMARIN | 4,0 | 4,0 |
| COUMARONE (BENZOFURAN, 2-ACETYL-) | 0,6 | 0,6 |
| ISOBORNYLACETAT | 30,0 | 30,0 |
| KAMPFER | 8,0 | 8,0 |
| LILIAL^{®} (PROPANAL, 2-METHYL-3-(4-TERT.-BUTYLPHENYL)-) | 30,0 | 30,0 |
| LINALOOL | 40,0 | 40,0 |
| LINALYLACETAT | 40,0 | 40,0 |
| TERPINEOL | 100,0 | 100,0 |
| GALAXOLID^{®} 50% in DEP (1,1,2,3,3,8-Hexamethyl-1,2,3,5,7,8-hexahydro-6-oxa-cyclopenta[b]naphthalin) | 24,0 | 24,0 |
| GLOBALIDE^{®} (PENTADECEN-1,15-OLID, 11E/Z) | 5,0 | 5,0 |
| ETHYLENBRASSYLAT (BRASSYLSÄUREETHANDIOLESTER) | 24,0 | 24,0 |
| AGRUNITRIL (HEPTENYLCYANID, 2,6-DIMETHYL-5-) | 16,0 | 16,0 |
| ISORALDEIN^{®} 70 (E)-3-Methyl-4-(2,6,6-trimethyl-cyclohex-2-enyl)-but-3-en-2-on) | 20,0 | 20,0 |
| OZONIL (DODECENYLCYANID, 1Z-) | 1,5 | 1,5 |
| ALLYLAMYLGLYCOLAT (ESSIGSÄUREALLYLESTER, 2-METHYLBUTOXY-) | 15,0 | 15,0 |
| DAMASCONE DELTA ((1-(2,6,6-Trimethyl-cyclohex-3-enyl)-but-2-en-1-on) | 2,0 | 2,0 |
| TIMBEROL^{®} (CYCLOHEXAN, 2,2,6-TRIMETHYL-1-(3-HYDROXYHEXYL)-) | 2,0 | 2,0 |
| ESTRAGOL | 1,0 | 1,0 |
| MYSORANE^{®} BASE | 10,0 | 10,0 |
| DIPROPYLENGLYCOL | 346,2 | 346,15 |
| CITRONENÖL TERPENE | 20,0 | 20,0 |
| GERANIOL | 8,0 | 8,0 |
| GERANIUM BASE | 5,0 | 5,0 |
| PATCHOULI BASE | 8,0 | 8,0 |
| GALBASCONE 10% in DPG (CYCLOHEXEN, 4,4-DIMETHYL-2-(1-OXO-4-PENTENYL)-) | 2,0 | 2,0 |
| CALONE^{®} 1951 10% in DPG (BENZODIOXEPINON, 7-METHYL-3,4-DIHYDRO-3-) | 4,0 | 4,0 |
| ISOBUTYLCHINOLIN | 0,2 | 0,2 |
| EUGENOL | 1,5 | 1,5 |
| FARENAL^{®} (UNDECENAL, 2,6,10-TRIMETHYL-9-) | 0,5 | 0,5 |
| LIMETTENÖL | 12,0 | 12,0 |
| ANETHOL | 4,0 | 4,0 |
| METHYLNAPHTHYLKETON BETA | 3,0 | 3,0 |
| **2-ISOBUTYL-4-VINYL-[1,3]DIOXOLAN** | | **0,05** |
| | 1.000,0 | 1.000,0 |

Bei einer Dosierung von 0.3% des Parfümöls im Allzweckreiniger ergibt sich folgender Befund: der Anteil von 2-Isobutyl-4-vinyl-[1,3]dioxolan in Parfümöl F2 bewirkt eine Verstärkung der holzig-moosigen Note. Zudem wird die Diffusivität erhöht.

### Beispiel 27: Shampoo

| **Material** | **Hersteller** | **INCI-Name** | **Gew.-%** |
|---|---|---|---|
| Entionisiertes Wasser | | Water | 71,5 |
| Plantacare PS 10 | Cognis Deutschland GmbH | Sodium Laureth Sulfate, Lauryl Glucoside | 20,0 |
| Euperlan PK 771 | Cognis Deutschland GmbH | Glycol Distearate, Sodium Lauryl Sulfate, Cocamide MEA, Laureth-10 | 6,0 |
| Dragocid Liquid | Symrise | Phenoxyethanol, Methylparaben, Ethylparaben, Butylparaben, Propylparaben, Isobutylparaben | 0,5 |
| Natriumchlorid | | Sodium Chloride | 1,4 |
| Citronensäure Mono-hydrat kristallin | | Citric Acid | 0,1 |
| Parfumöl G1 bzw. G2 | Symrise | Parfum (Fragrance) | 0,5 |

### Parfümöle für die Shampoos des Beispiels 27:

| **Riechstoffe** | **Parfümöl G1** (Vergleich) | **Parfümöl G2** (erfindungsgemäß) |
|---|---|---|
| NELKENBLÜTENÖL | 10,0 | 10,0 |
| PATCHOULIÖL | 79,5 | 79,5 |
| DIHYDROMYRCENOL | 60,0 | 60,0 |
| ISORALDEIN^{®} ((E)-3-Methyl-4-(2,6,6-trimethyl-cyclohex-2-enyl)-but-3-en-2-on) | 20,0 | 20,0 |
| EBANOL (PENTEN-2-ON, 3-METHYL-5-(2,2,3-TRIMETHYL-3-CYCLOPENTENYL)-4E/Z-) | 15,0 | 15,0 |
| GLOBALIDE^{®} (PENTADECEN-1,15-OLID, 11E/Z) | 60,0 | 60,0 |
| HEXYLZIMTALDEHYD ALPHA | 40,0 | 40,0 |
| CUMARIN | 20,0 | 20,0 |
| LILIAL^{®} (PROPANAL, 2-METHYL-3-(4-TERT.-BUTYLPHENYL)-) | 30,0 | 30,0 |
| LINALOOL | 20,0 | 20,0 |
| LINALYLACETAT | 30,0 | 30,0 |
| VANILLIN | 20,0 | 20,0 |
| LYRAL^{®} (CYCLOHEXEN, 4-FORMYL-2-(4-HYDROXY-4-METHYL-PENTYL)-) | 40,0 | 40,0 |
| HEDION^{®} (METHYL-CIS/TRANS-DIHYDROJASMONAT) | 30,0 | 30,0 |
| EVERNYL^{®} (BENZOESAEUREMETHYLESTER, 2,4-DIHYDROXY-3,6-DIMETHYL-) | 5,0 | 5,0 |
| CEDRAMBER (CEDRYLMETHYLETHER) | 20,0 | 20,0 |
| ISO E SUPER^{®} (BICYCLO[4.4.0]DECEN, 3-ACETYL-3,4,10,10-TETRAMETHYL-1 (6)-) | 150,5 | 150,0 |
| GERANIUM BASE | 20,0 | 20,0 |
| BERGAMOTTE BASE | 80,0 | 80,0 |
| BEIFUSSÖL | 10,0 | 10,0 |
| GALBANUMÖL 10% in DPG | 15,0 | 15,0 |
| AMBROCENIDE^{®} 0,1% in DPG | 20,0 | 20,0 |
| CYCLOGALBANAT^{®} 10% in DPG (CYCLOHEXYLOXYESSIGSÄUREALLYLESTER) | 5,0 | 5,0 |
| CISTUSÖL 10% in DPG | 10,0 | 10,0 |
| KRAUSEMINZÖL (SPEARMINT) 10% in DPG | 10,0 | 10,0 |
| AURELIONE (CYCLOHEXADECENON, 7/8E/Z-) | 120,0 | 120,0 |
| AMBROXID | 5,0 | 5,0 |
| MANDARINENÖL | 5,0 | 5,0 |
| LAVANDINÖL GROSSO | 30,0 | 30,0 |
| CITRONENÖL | 20,0 | 20,0 |
| **2-ISOBUTYL-4-VINYL-[1,3]DIOXOLAN** | | **0,5** |
| | 1.000,0 | 1.000,0 |

Bei einer Dosierung von 0.5% des Parfümöls im Shampoo ergibt sich folgender Befund: der Zusatz von 2-Isobutyl-4-vinyl-[1,3]dioxolan in Parfümöl G2 verstärkt den fruchtigen Charakter. Ferner erscheint die Komposition viel cremiger und runder.

### Beispiel 28: Duschgel

| **Material** | **Hersteller** | **INCI-Name** | **Gew.-%** |
|---|---|---|---|
| Entionisiertes Wasser | | Wasser | 76,3 |
| Plantacare PS 10 | Cognis Deutschland GmbH | Sodium Laureth Sulfate, Lauryl Glucoside | 20,0 |
| Dragocid Liquid | Symrise | Phenoxyethanol, Methylparaben, Ethylparaben, Butylparaben, Propylparaben, Isobutylparaben | 0,5 |
| Natriumchlorid | | Sodium Chloride | 1,4 |
| Citronensäure Monohydrat kristallin | | Citric Acid | 1,3 |
| Parfumöl H1 bzw. H2 | Symrise | Parfum (Fragrance) | 0,5 |

### Parfümöl für die Duschgele des Beispiels 28:

| **Riechstoff(e)** | **Parfümöl H1** (Vergleich) | **Parfümöl H2** (erfindungsgemäß) |
|---|---|---|
| CASSIS BASE | 10,0 | 10,0 |
| NELKENBLÜTENÖL | 5,0 | 5,0 |
| PATCHOULIÖL | 5,0 | 5,0 |
| DIHYDROMYRCENOL | 60,0 | 60,0 |
| HEXYLSALICYLAT | 20,0 | 20,0 |
| HEDION^{®} (METHYL-CIS/TRANS-DIHYDROJASMONAT) | 210,0 | 210,0 |
| ORANGENÖL | 25,0 | 25,0 |
| GLOBALIDE^{®} (PENTADECEN-1,15-OLID, 11E/Z) | 20,0 | 20,0 |
| POLYSANTOL^{®} (PENTEN-2-OL, 3,3-DIMETHYL-5-(2,2,3-TRIMETHYL-3-CYCLOPENTENYL)-4-) | 5,0 | 5,0 |
| LAVANDINÖL GROSSO | 20,0 | 20,0 |
| YSAMBER^{®} K (ISOLONGIFOLANONETHANDIOLKETAL) | 20,0 | 20,0 |
| HEXYLZIMTALDEHYD ALPHA | 100,0 | 100,0 |
| LILIAL^{®} (PROPANAL, 2-METHYL-3-(4-TERT.-BUTYLPHENYL)-) | 50,0 | 50,0 |
| LINALOOL | 60,0 | 60,0 |
| LINALYLACETAT | 50,0 | 50,0 |
| TERPINEOL | 20,0 | 20,0 |
| ETHYLENBRASSYLAT (BRASSYLSÄUREETHANDIOLESTER) | 20,0 | 20,0 |
| ALLYLAMYLGLYCOLAT (ESSIGSÄUREALLYLESTER, 2-METHYLBUTOXY-) | 10,0 | 10,0 |
| ISO E SUPER^{®} (BICYCLO[4.4.0]DECEN, 3-ACETYL-3,4,10,10-TETRAMETHYL-1 (6)-) | 50,0 | 50,0 |
| KEPHALIS (CYCLOHEXANON, 3,3,5,5-TETRAMETHYL-4-(1-ETHOXYVINYL)-) | 5,0 | 5,0 |
| BERGAMOTTE BASE | 100,0 | 100,0 |
| FLORALOZONE (PROPANAL, 2-METHYL-2-(4-ETHYLBENZYL)- | 10,0 | 10,0 |
| MANDARINENALDEHYD 10% in TEC (DODECENAL, 2E-) | 5,0 | 5,0 |
| LlGUSTRAL^{®} 10% in DPG (CYCLOHEXEN, TRANS-2,4-DIMETHYL-1-FORMYL-3-) | 10,0 | 10,0 |
| DAMASCON ALPHA 1 % in DPG ((E/Z)-1-(2,6,6-Trimethyl-cyclohex-2-enyl)-but-2-en-1-on) | 20,0 | 20,0 |
| FARENAL^{®} 1% in DPG (UNDECENAL, 2,6,10-TRIMETHYL-9-) | 20,0 | 20,0 |
| LEAFOVERT^{®} 10% in DPG (KOHLENSAEURE-3Z-HEXENYLMETHYLESTER) | 20,0 | 20,0 |
| CALONE^{®} 1951 10% in DPG (BENZODIOXEPINON, 7-METHYL-3,4-DIHYDRO-3-) | 30,0 | 30,0 |
| DIPROPYLENGLYCOL | 20,0 | 19,8 |
| **2-ISOBUTYL-4-VINYL-[1,3]DIOXOLAN** | | **0,2** |
| | 1.000,0 | 1.000,0 |

Bei einer Dosierung von 0.5% des Parfümöls im Duschgel ergibt sich folgender Befund: der Zusatz von 2-Isobutyl-4-vinyl-[1,3]dioxolan in Parfümöl H2 verleiht der Komposition eine strahlendere aromatische Note.

### Beispiel 29: Transparente Deo-Stifte (Formulierungen A und B) bzw. Deo-Creme-Stifte (Formulierungen C und D)

| **Komponente** | **A** | **B** | **C** | **D** |
|---|---|---|---|---|
| | **Gew.-%** | **Gew.-%** | **Gew.-%** | **Gew.-%** |
| Aluminium Zirconium Tetrachlorohydrat - Glycin Komplex | 25,00 | 20,00 | 25,00 | 20,00 |
| Dimethicon (10 Cst) | - | - | 5,00 | 5,00 |
| Cyclopentasiloxan | - | 0,50 | 1,00 | 0,50 |
| Petrolatum | 5,00 | 4,70 | 5,00 | 5,00 |
| Ozokerit | 1,00 | 1,50 | - | - |
| Stearylalkohol | 12,00 | 12,00 | - | - |
| 2-Butyloctansäure | 0,50 | - | 0,50 | - |
| Wachs | - | - | 1,25 | 1,25 |
| PPG-14 Butylether | 9,00 | 9,00 | - | - |
| Gehärtetes Rapsöl | - | - | 5,00 | 5,00 |
| Siliciumdioxid | - | - | 1,00 | - |
| Farnesol | 0,25 | - | 0,25 | - |
| Paraffinöl | 0,50 | 0,50 | - | - |
| Hydriertes Rizinusöl (castor wax) | 3,50 | 3,50 | - | - |
| Talk | 4,00 | 4,00 | - | - |
| Behenylalkohol | 0,20 | 0,20 | - | - |
| d-Panthenyltriacetat | 1,00 | 1,00 | - | - |
| Konservierungsmittel | q.s. | q.s. | q.s. | q.s. |
| Parfümöl G2 aus Beispiel 27 | 1,50 | - | 1,15 | - |
| Parfümöl H2 aus Beispiel 28 | | 0,90 | - | 0,75 |
| Wasser | Ad 100 | Ad 100 | Ad 100 | Ad 100 |

### Beispiel 30: Antiperspirant-Roll-On

| **Komponente** | **Gew.-%** | **Gew.-%** |
|---|---|---|
| Caprylyl Trimethicon (SilCare TM Silicone 31 M 50) | 0,30 | 0,30 |
| Steareth-20 (GENAPOL TM HS 200) | 3,00 | 3,00 |
| Steareth-2 (GENAPOL TM HS 020) | 1,50 | 1,50 |
| Dicaprylyl Ether (Cetiol TM OE) | 2,00 | 2,00 |
| Coco Caprylat/Caprat (Cetiol TM LC) | 2,00 | 2,00 |
| Glycerin | 2,00 | 2,00 |
| Glyceryl Stearat (Cutina TM GMS) | 2,00 | 2,00 |
| Octyldodecanol (Eutanol TM G) | 1,00 | 1,00 |
| Stearyl alkohol | 2,50 | 2,50 |
| Aluminiumchlorohydrat gemäß Beispiel 1 der EP | 10,00 | 10,00 |
| 1321431 | | |
| Avocado Extract Persea Gratissima | 0,30 | 0,20 |
| Parfümöl G2 aus Beispiel 27 | 0,50 | - |
| Parfümöl H2 aus Beispiel 28 | - | 0,60 |
| Wasser | Ad 100 | Ad 100 |

### Beispiel 31: Antiperspirant-Stick

| **Komponente** | **Gew.-%** | **Gew.-%** |
|---|---|---|
| Phenyl Trimethicon (SilCare TM Silicone 15 M 50) | 13,50 | 13,50 |
| Cetearyl Alkohol | Ad 100 | Ad 100 |
| Cetiol CC (Dicaprylyl Carbonat) | 13,50 | 13,50 |
| Stearinsäure | 3,50 | 3,50 |
| PEG-40-Hydriertes Rizinusöl (Emulsogen TM HCO 040) | 4,10 | 4,10 |
| PEG-8 Distearate (Cithrol 4 DS) | 4,10 | 4,10 |
| Petrolatum | 6,90 | 6,90 |
| Aluminiumchlorohydrat | 13,80 | 13,80 |
| Aluminium Zirconium Trichlorohydrex Gly | 19,50 | 20,00 |
| Ethylhexylglycerin (Octoxyglycerin) | 0,30 | 0,20 |
| 4-Methyl-4-phenyl-2-pentanol (Vetikol) | 0,25 | 0,10 |
| Parfümöl A2 aus Beispiel 20 | 1,00 | - |
| Parfümöl G2 aus Beispiel 27 | - | 0,80 |

### Beispiel 32: Aerosolspray

| **Komponente** | **Gew.-%** | **Gew.-%** | **Gew.-%** |
|---|---|---|---|
| Octyldodecanol | 0,50 | - | 0,50 |
| Phenoxyethanol | - | - | 0,30 |
| 1,2-Pentandiol | 1,00 | 1,00 | 0,50 |
| 1,2-Hexandiol | 0,25 | 0,15 | 0,25 |
| 1,2-Octandiol | 0,25 | 0,25 | 0,25 |
| Farnesol | - | 0,25 | 0,15 |
| Ethylhexylglycerin (Octoxyglycerin) | 0,50 | 0,30 | 0,50 |
| Parfümöl A2 aus Beispiel 20 | 0,80 | - | 0,50 |
| Parfümöl H2 aus Beispiel 28 | - | 1,15 | 0,50 |
| Ethanol | Ad 100 | Ad 100 | Ad 100 |

Die nach Zusammenmischen der jeweils angegebenen Komponenten erhaltene Mischung wurde mit einem Propan-Butan-Gemisch (2:7) im Gewichtsverhältnis 2 : 3 in einen Aerosolbehälter abgefüllt.

### Beispiel 33: Haarconditioner mit UV-Schutz

| **Komponente** | **INCI Name** | **Gew.-%** | **Gew.-%** |
|---|---|---|---|
| Lanette O | Cetearyl Alcohol | 4,00 | 4,00 |
| Dragoxat 89 | Ethylhexyl Isononanoate | 4,00 | 4,00 |
| Emulsiphos | Potassium Cetyl Phosphate, Hydrogenated Palm Glycerides | 0,50 | 0,50 |
| Natrosol 250 HR | Hydroxyethylcellulose | 0,25 | 0,25 |
| Neo Heliopan Hydro | Phenylbenzimidazole Sulfonic Acid | 2,00 | 2,00 |
| L- Arginin | Arginine | 1,20 | 1,20 |
| Benzophenon-4 | Benzophenone-4 | 0,50 | 0,50 |
| Neo Heliopan AP | Disodium Phenyl Dibenzimidazole Tetrasulfonate | 0,50 | 1,00 |
| Edeta BD | Disodium EDTA | 0,05 | 0,05 |
| Dragocide Liquid | Phenoxyethanol (and) Methylparaben (and) Butyparaben (and) Ethyparaben (and) Propylparaben | 0,80 | 0,80 |
| Dow Corning 949 Cationic Emulsion | Amodimethicone, Cetrimonium Chloride, Trideceth-12 | 2,00 | 2,00 |
| Dow Corning 5200 | Laurylmethicone Copolyol | 0,50 | 0,50 |
| Parfümöl B2 aus Beispiel 21 | Parfum | 0,95 | - |
| Parfümöl H2 aus Beispiel 28 | Parfum | | 1,25 |
| Wasser | Water (Aqua) | Ad 100 | Ad 100 |

### Beispiel 34: Sonnenschutzspray

| **Teil** | **Rohstoffe** | **INCI Bezeichnung** | **Gew.-%** |
|---|---|---|---|
| **A** | Wasser, demineralisiert | Water (Aqua) | 69,50 |
| | Glycerin | Glycerin | 4,00 |
| | 1,3 Butylenglykol | Butylene Glycol | 5,00 |
| | D-Panthenol | Panthenol | 0,50 |
| | Lara Care A-200 | Galactoarabinan | 0,25 |
| **B** | Baysiloneöl M 10 | Dimethicone | 1,00 |
| | Edeta BD | Disodium EDTA | 0,10 |
| | Copherol 1250 | Tocopheryl Acetate | 0,50 |
| | Cetiol OE | Dicaprylyl Ether | 3,00 |
| | Neo Heliopan^{®} HMS | Homosalate | 5,00 |
| | Neo Heliopan^{®} AV | Ethylhexyl Methoxycinnamate | 6,00 |
| | Neo Heliopan^{®} 357 | Butyl Methoxydibenzoylmethane | 1,00 |
| | Corapan TQ | Diethylhexylnaphthalate | 2,00 |
| | Alpha Bisabolol | Bisabolol | 0,10 |
| | Pemulen TR-2 | Acrylates/C10-30 Alkyl Acrylate Crosspolymer | 0,25 |
| **C** | Phenoxyethanol | Phenoxyethanol | 0,70 |
| | Solbrol M | Methylparaben | 0,20 |
| | Solbrol P | Propylparaben | 0,10 |
| **D** | NaOH, 10 %ig | Sodium Hydroxide | 0,60 |
| **E** | Parfümöl B2 aus Beispiel 21 | Fragrance (Parfum) | 0,20 |

### Herstellungsverfahren

Teil A: Lara Care A-200 unter Rühren in den anderen Bestandteilen von Teil A lösen.
Teil B: Alle Rohstoffe (ohne Pemulen) einwiegen und die kristallinen Substanzen unter Erwärmen lösen. Pemulen eindispergieren. Teil B zu Teil A geben und 1 Minute homogenisieren.
Teile C-E: zugeben und nochmals 1-2 Minuten mit dem Ultra Turrax homogenisieren.

### Beispiel 35: Sonnenschutz Softcreme (W/O), Lichtschutzfaktor (SPF) 40

| **Teil** | **Rohstoffe** | **INCI Bezeichnung** | **Gew.-%** |
|---|---|---|---|
| **A** | Dehymuls PGPH | Polyglyceryl-2 Dipolyhydroxystearate | 5,00 |
| | Copherol 1250 | Tocopheryl acetate | 0,50 |
| | Permulgin 3220 | Ozokerite | 0,50 |
| | Zinkstearat | Zinc Stearate | 0,50 |
| | Tegosoft TN | C12-15 Alkyl Benzoate | 10,00 |
| | Neo Heliopan^{®} E1000 | Isoamyl-p-Methoxycinnamate | 2,00 |
| | Neo Heliopan^{®} 303 | Octocrylene | 5,00 |
| | Neo Heliopan^{®} MBC | 4-Methylbenzylidene Camphor | 3,00 |
| | Zinkoxid neutral | Zinc Oxide | 5,00 |
| **B** | Wasser, destilliert | Water (Aqua) | Ad 100 |
| | EDETA BD | Disodium EDTA | 0,10 |
| | Glycerin | Glycerin | 4,00 |
| | Phenoxyethanol | Phenoxyethanol | 0,70 |
| | Solbrol M | Methylparaben | 0,20 |
| | Solbrol P | Propylparaben | 0,10 |
| | Magnesiumsulfat | Magnesium Sulfate | 0,50 |
| **C** | Parfümöl B2 aus Beispiel 21 | Parfum (Fragrance) | 0,30 |

### Herstellungsverfahren

Teil A: Auf ca. 85 °C erhitzen.
Teil B: Auf ca. 85 °C erhitzen (ohne Zinkoxid; Zinkoxid mit dem Ultra Turrax eindispergieren). B zu A geben. Unter Rühren abkühlen lassen.
Teil C: Zugeben und anschließend homogenisieren.

### Beispiel 36: Sonnenschutzmilch (W/O)

| **Teil** | **Rohstoffe** | **INCI Bezeichnung** | **Gew.-%** |
|---|---|---|---|
| **A** | Dehymuls PGPH | Polyglyceryl-2 Dipolyhydroxystearate | 3,00 |
| | Bienenwachs 8100 | Beeswax | 1,00 |
| | Monomuls 90-0-18 | Glyceryl Oleate | 1,00 |
| | Zinkstearat | Zinc stearate | 1,00 |
| | Cetiol SN | Cetearyl Isononanoate | 5,00 |
| | Cetiol OE | Dicaprylyl Ether | 5,00 |
| | Tegosoft TN | C12-15 Alkyl Benzoate | 4,00 |
| | Vitamin E | Tocopherol | 0,50 |
| | Solbrol P | Propylparaben | 0,10 |
| | Neo Heliopan^{®} OS | Ethylhexyl Salicylate | 5,00 |
| | Neo Heliopan^{®} AV | Ethylhexyl Methoxycinnamate | 7,50 |
| | Uvinul^{®} T150 | Ethylhexyl Triazone | 1,50 |
| **B** | Wasser, destilliert | Water (Aqua) | Ad 100 |
| | Trilon BD | Disodium EDTA | 0,10 |
| | Glycerin | Glycerin | 5,00 |
| | Solbrol M | Methylparaben | 0,20 |
| | Phenoxyethanol | Phenoxyethanol | 0,70 |
| | Neo Heliopan^{®} AP 10%ige Lösung, neutralisiert mit NaOH | Disodium Phenyl Dibenzimidazole Tetrasulfonate | 15,00 |
| **C** | Parfümöl G2 aus Beispiel 27 | Parfum (Fragrance) | 0,25 |
| | Alpha Bisabolol | Bisabolol | 0,10 |

### Herstellungsverfahren

Teil A: Auf ca. 85 °C erhitzen.
Teil B: Auf ca. 85 °C erhitzen. B zu A geben. Unter Rühren abkühlen lassen.
Teil C: Zugeben und anschließend homogenisieren.

### Beispiel 37

Herstellung eines Aromas vom Eucalyptus-Menthol-Typ unter Verwendung von 2-Isobutyl-4-vinyl-[1,3]dioxolan

| **Komponente** | **Gew.-%** |
|---|---|
| Anethol | 10 |
| Pfefferminzöl Mentha piperita Typ Willamette | 20 |
| Pfefferminzöl Mentha arvensis, rektifiziert | 20 |
| 1,8-Cineol (Eucalyptol) | 5 |
| /-Menthol | 44 |
| **2-Isobutyl-4-vinyl-[1,3]dioxolan** | 1 |
| Total | 100 |

Das so erhaltene Aroma wurde in eine Standard-Zahnpasta-Masse auf Kieselsäure-Basis in einer Konzentration von 1,2 Gew.-% eingearbeitet. Die Zahnpasta wurde von einem sensorisch geschulten Experten-Panel unter Praxisbedingungen getestet. Der Geschmack der erfindungsgemäßen Zahnpasta wurde als voller und angenehmer beurteilt der einer Zahnpasta mit einem entsprechenden Aroma ohne 2-Isobutyl-4-vinyl-[1,3]dioxolan.

### Beispiel 38

Herstellung eines Aromas vom Krauseminz-Typ unter Verwendung von 2-Isobutyl-4-vinyl-[1,3]dioxolan.

| **Komponente** | **Gew.-%** |
|---|---|
| /-Menthol | 29 |
| /-Carvon | 20 |
| Krauseminzöl Typ Native | 20 |
| Anethol | 5 |
| Pfefferminzöl Mentha arvensis rektifiziert | 10 |
| Pfefferminzöl Mentha piperita Typ Willamette | 15 |
| **2-Isobutyl-4-vinyl-[1,3]dioxolan** | **1** |
| Total | 100 |

Das so erhaltene Aroma wurde in eine Zahnpasta-Masse in einer Konzentration von 1,2 Gew.-% eingearbeitet, wobei die Zahnpasta-Masse zu 65 Gew.-% aus Natriumbicarbonat bestand. Die resultierende Zahnpasta wurde von einem sensorisch geschulten Experten-Panel unter Praxisbedingungen getestet. Das Aroma dieser Zahnpasta wurde als schöner, kräftiger Krauseminzgeschmack in Verbindung mit einem voluminösen Frischegeschmack bewertet.

### Beispiel 39

Herstellung eines Aromas mit einem würzig-aromatischen Geschmackseindruck unter Verwendung von 2-Isobutyl-4-vinyl-[1,3]dioxolan.

| **Komponente** | **Gew.-%** |
|---|---|
| /-Menthol | 29 |
| Pfefferminzöl Mentha arvensis, rektifiziert | 25 |
| Pfefferminzöl Mentha piperita Typ Willamette | 15 |
| Anethol | 10 |
| Krauseminzöl Typ Native | 10 |
| Zimtaldehyd | 5 |
| Eugenol | 5 |
| **2-Isobutyl-4-vinyl-[1,3]dioxolan** | **1** |
| Total | 100 |

Dieses Aroma wurde in eine Standard-Zahnpasta-Masse auf Kieselsäure-Basis in einer Konzentration von 1,2 Gew.-% eingearbeitet. Die Zahnpasta wurdenvon einem sensorisch geschulten Experten-Panel unter Praxisbedingungen getestet. Die sensorische Bewertung der Zahnpasta ergab einen sehr schönen, vollen, minzigen, fruchtig-frischen Geschmack.

### Beispiel 40

Herstellung eines Aromas mit Wintergrün-Geschmack unter Verwendung von 2-Isobutyl-4-vinyl-[1,3]dioxolan.

| **Komponente** | **Gew.-%** |
|---|---|
| Anethol | 10 |
| Pfefferminzöl Mentha arvensis | 12 |
| Pfefferminzöl Mentha piperita Typ Willamette | 12 |
| Methylsalicylat | 25 |
| /-Menthol | 40 |
| **2-Isobutyl-4-vinyl-[1,3]dioxolan** | **1** |
| Total | 100 |

Das so erhaltene Aroma wurde in eine Standard-Zahnpasta-Masse auf Kieselsäure-Basis in einer Konzentration von 1,2 Gew.-% eingearbeitet. Die Zahnpasta wurde von einem sensorisch geschulten Experten-Panel unter Praxisbedingungen getestet. Der Geschmack dieser erfindungsgemäßen Zahnpasta wurde als voller und etwas süßer beurteilt als der einer Zahnpasta mit einem entsprechenden Aroma ohne 2-Isobutyl-4-vinyl-[1,3]dioxolan.

### Beispiel 41

Herstellung eines Aromas mit Pfefferminzgeschmack unter Verwendung von 2-Isobutyl-4-vinyl-[1,3]dioxolan.

| **Komponente** | **Gew.-%** |
|---|---|
| Anethol | 9 |
| Pfefferminzöl Mentha arvensis | 50 |
| /-Menthon | 20 |
| /-Menthol | 20 |
| **2-Isobutyl-4-vinyl-[1,3]dioxolan** | **1** |
| Total | 100 |

Das so erhaltene Aroma wurde in eine zuckerfreie Standard-Kaugummi-Masse in einer Konzentration von 1,5 Gew.-% eingearbeitet. Die Kaugummis wurden von einem geschulten Experten-Panel auf ihre sensorische Qualität getestet. Das Aroma zeigte eine ausgeprägte fruchtigfrische Note, wobei zudem der Pfefferminzgeschmack verstärkt wurde. Das Aroma sorgte ferner für einen vollen Geschmackseindruck des Kaugummis.

### Beispiel 42

Herstellung eines Mundwasser-Aromas unter Verwendung von 2-Isobutyl-4-vinyl-[1,3]dioxolan.

| **Komponente** | **Gew.-%** |
|---|---|
| Anethol | 30 |
| Eucalyptol | 25 |
| /-Menthol | 44 |
| **2-Isobutyl-4-vinyl-[1,3]dioxolan** | **1** |
| Total | 100 |

Das Aroma wurde in einer Konzentration von 0,15 Gew.-% in ein gebrauchsfertiges Mundwasser bzw. in einer Konzentration von 3 Gew.-% in ein Mundwasserkonzentrat eingearbeitet. Die jeweilige sensorische Bewertung durch ein geschultes Experten-Panel zeigte, dass das Aroma durch die Anwesenheit von 2-Isobutyl-4-vinyl-[1,3]dioxolan eine volle, sehr angenehme fruchtigfrische Note enthält.

Die in den nachfolgenden Beispielen 43 bis 58 eingesetzten Aromakompositionen eignen sich für einen Einsatz in einer ganzen Reihe von unterschiedlichen Artikeln, wobei der Einsatz nicht nur auf Artikel wie Zahnpasten, Kaugummis oder Bonbons eschränkt ist. Bei allen nachfolgenden Beispielen konnte insbesondere ein voller und angenehmer Geschmackseindruck wahrgenommen werden.

### Beispiel 43: Zahnpasta ('Silica opaque')

Alle Angaben, soweit nicht anders vermerkt, in Gew-%.

| **Bestandteil** | **A** | **B** |
|---|---|---|
| | | |
| Entionisiertes Wasser | 26,53 | 26,53 |
| Sorbitol 70% | 45 | Ad 100 |
| Solbrol M Na-Salz | 0,15 | 0,15 |
| Trinatriumphosphat | 0,1 | 0,1 |
| Saccharin | 0,2 | 0,2 |
| Natriummonofluorphosphat | 1,12 | 1,12 |
| PEG 1500 | 5 | 5 |
| Sident 9 (Abrasive Silica) | 10 | 10 |
| Sident 22 S (Dickende Silica) | 8 | 8 |
| Natriumcarboxymethylcellulose | 0,9 | 0,9 |
| Titan (IV) oxid | 0,5 | 0,5 |
| Natriumlaurylsulfat (SLS) | 1,5 | 1,5 |
| Pellitorin-Lösung (enthaltend 10% Pellitorin) | - | 0,025 |
| Aroma vom Eucalyptus-Menthol-Typ aus Beispiel 37 | 1 | 1 |

### Beispiel 44: Zahnpasta (Calciumcarbonat-Base)

| **Bestandteil** | **A** | **B** |
|---|---|---|
| | | |
| Entionisiertes Wasser | 27,5 | Ad 100 |
| Saccharin | 0,2 | 0,2 |
| Solbrol M Natriumsalz | 0,2 | 0,2 |
| Natriummonofluorphosphat | 0,8 | 0,8 |
| Sorbitol 70% | 29 | 29 |
| Calciumcarbonat | 35 | 35 |
| Sident 22 S (Verdickende Silica) | 2,5 | 2,5 |
| Natriumcarboxymethylcellulose | 1,3 | 1,3 |
| Titandioxid | 0,5 | 0,5 |
| Natriumlaurylsulfat | 2 | 2 |
| Pellitorin-Lösung (enthaltend 10% Pellitorin) | - | 0,02 |
| Aroma vom Eucalyptus-Menthol-Typ aus Beispiel 37 | 1 | 1 |

### Beispiel 45: Zahnpasta mit Bleichwirkung

| **Bestandteil** | **A** | **B** |
|---|---|---|
| | | |
| Polyphosphat (Glass H, (n ≈ 21), Astaris) | 7 | 7 |
| Calciumperoxid | 1 | - |
| Na-percarbonat | - | 11 |
| Poloxamer 407 | 5 | 2 |
| Polyethylenglycol | 3 | - |
| Sorbitol, 70 %ig in Wasser | - | 22 |
| Glycerin | 43,8 | 12,5 |
| 1,2-Propylenglycol | 4 | - |
| Na-Saccharin | 0,4 | 0,2 |
| Natriumbicarbonat | - | 5 |
| Natriumcarbonat | 2 | 2 |
| Silica | 20 | 22 |
| Na-Carboxymethylcellu lose | 0,6 | 0,55 |
| Natriumlaurylsulfat | 1 | 4 |
| Xanthan Gum | 0,2 | 0,2 |
| Titandioxid (Anatas) | 0,5 | 0,5 |
| Aroma vom Typ Eucalyptus-Menthol aus Beispiel 37 | 1 | |
| Aroma vom Typ würzig-aromatisch aus Beispiel 39 | | 1,25 |
| Wasser dest. | Ad 100 | Ad 100 |

### Beispiel 46: Zahnpasten mit Zinn- und Zinksalzen

| **Bestandteil** | **A** | **B** | **C** |
|---|---|---|---|
| | | | |
| Natriumfluorid NaF | 0,42 | 0,5 | - |
| Zinnfluorid SnF₂ | - | 0,9 | 0,95 |
| Zinnchlorid SeCl2 | 1,5 | - | 2 |
| Zinklactat | 2 | 2 | - |
| Zinkcarbonat ZnCO₃ | - | 1 | 1,5 |
| Na-gluconat | - | 0,67 | 1,5 |
| Poloxamer 407 | 14,5 | - | - |
| Polyethylenglycol | 1 | 3 | - |
| Sorbitol, 70 %ig in Wasser | - | 38 | 37,5 |
| Glycerin | 37,5 | 5 | 14,4 |
| 1,2-Propylenglycol | 7 | 5 | - |
| Na-Saccharin | 0,3 | 0,5 | 0,5 |
| Abrasiv-Silica | 20 | 22,5 | 25 |
| Natriumhydroxid | - | 0,1 | 0,2 |
| Natriumlaurylsulfat | - | 2 | 1,5 |
| Na-polyphosphat | - | - | 4 |
| Tetranatriumpyrophosphat | 1 | 2,5 | - |
| Farbstoff(1%ig in Wasser) | 0,4 | 0,5 | 0,5 |
| Aroma vom Eucalyptus-Menthol-Typ aus Beispiel 37 | 0,95 | - | - |
| Aroma vom Typ würzig-aromatisch aus Beispiel 39 | - | 1,2 | - |
| Aroma vom Typ Wintergrün aus Beispiel 40 | - | - | 1,15 |
| Wasser dest. | Ad 100 | Ad 100 | Ad 100 |

### Beispiel 47: Zahnpasta auf Phosphatbasis

| **Bestandteil** | **Gew.-%** |
|---|---|
| | |
| Entionisiertes Wasser | 36,39 |
| Glycerin | 20 |
| Solbrol M (Natriumsalz) | 0,15 |
| Natriummonofluorphosphat | 0,76 |
| Saccharin | 0,2 |
| Dicalciumphosphat-Dihydrat | 36 |
| Aerosil® 200 (Silica) | 3 |
| Natriumcarboxymethylcellulose | 1,2 |
| Natriumlaurylsulfat (Texapon) | 1,3 |
| Aroma vom Typ Krauseminze aus Beispiel 38 | 1 |

### Beispiel 48: Zahnpasta (transparente Gelformulierung)

| **Bestandteil** | **A** | **B** |
|---|---|---|
| | | |
| Sorbitol 70% | 63 | Ad 100 |
| Entionisiertes Wasser | 11,31 | 11,31 |
| Saccharin | 0,2 | 0,2 |
| Natriummonofluorphosphat | 1,14 | 1,14 |
| Solbrol | 0,15 | 0,15 |
| Trinatriumphosphat | 0,1 | 0,1 |
| PEG 1500 ( PEG 32) | 5 | 5 |
| Sident 9 (Abrasive Silica) | 8 | 8 |
| Sident 22 S (Verdickende Silica) | 8 | 8 |
| Natriumcarboxymethylcellulose | 0,6 | 0,6 |
| Natriumlaurylsulfat | 1,5 | 1,5 |
| Pellitorin-Lösung (enthaltend 10% Pellitorin) | - | 0,025 |
| Aroma vom Typ würzig-aromatisch ausBeispiel 39 | 1 | - |
| Aroma vom Typ Wintergrün aus Beispiel 40 | - | 1 |

### Beispiel 49: Mundwasserkonzentrat mit Aroma vom Typ Wintergrün

| **Bestandteil** | **Gew.-%** |
|---|---|
| | |
| Ethylalkohol 96% | 42 |
| Cremophor RH 455 | 5 |
| Entionisiertes Wasser | 48,67 |
| Allantoin | 0,2 |
| Natriumsaccharin 450 | 0,1 |
| Colour L-Blue 5000 (1 % in Wasser) | 0,03 |
| Aroma vom Typ Wintergrün aus Beispiel 40 | 4 |

### Beispiel 50: Mundwasser (,ready to use' ohne Alkohol)

| **Bestandteil** | **Gew.-%** |
|---|---|
| | |
| Cremophor RH 455 | 1,8 |
| Entionisiertes Wasser | 87,57 |
| Sorbitol 70% | 10 |
| Natriumfluorid | 0,18 |
| Natriumsaccharin 450 | 0,1 |
| Solbrol M Natriumsalz | 0,15 |
| Mundwasser-Aroma aus Beispiel 42 | 0,2 |

### Beispiel 51: Mundwasser (,ready to use' mit Alkohol)

| **Bestandteil** | **A** | **B** | **C** |
|---|---|---|---|
| | | | |
| Ethylalkohol 96% | 10 | 5 | 7 |
| Cremophor CO 40 | 1 | 1 | 1 |
| Benzoesäure | 0,1 | 0,12 | 0,1 |
| Entionisiertes Wasser | Ad 100 | Ad 100 | Ad 100 |
| Sorbitol 70% | 5 | 1 | 5 |
| Natriumsaccharin 450 | 0,07 | 0,05 | 0,05 |
| L-Blue 5000 (1 % in Wasser) | 0,1 | 0,1 | 0,1 |
| Glycerin | - | 8 | - |
| 1,2-Propylenglycol | - | 2 | 3 |
| Cetylpyridiniumchlorid | - | - | 0,07 |
| Wasserstoffperoxid (35% H₂O₂ in Wasser) | - | 3 | 4 |
| Aroma vom Typ Wintergrün aus Beispiel 40 | 0,25 | - | - |
| Mundwasser-Aroma aus Beispiel 42 | - | 0,25 | 0,25 |

### Beispiel 52: Zahncreme und Mundwasser als 2-in-1 Produkt

| **Bestandteil** | **Gew.-%** |
|---|---|
| Ethanol, 96%ig | 5 |
| Sorbitol, 70 %ig in Wasser | 40 |
| Glycerin | 20 |
| Saccharin | 0,2 |
| Na-Monofluorphosphat | 0,76 |
| Solbrol M, Na-Salz | 0,15 |
| Abrasivkieselsäure (Sident 9) | 20 |
| Verdickungskieselsäure (Sident 22S) | 2 |
| Na-Carboxymethylcellu lose | 0,3 |
| Natriumlaurylsulfat | 1,2 |
| Grüner Farbstoff (1%ig in Wasser) | 0,5 |
| Aroma vom Typ Eucalyptus-Menthol aus Beispiel 37 | 1 |
| Wasser dest. | Ad 100 |

### Beispiel 53: Standard Kaugummi

| **Bestandteil** | **A** | **B** |
|---|---|---|
| Gum Base (Kaugummibase) | 21 | 21 |
| Glucosesirup | 17 | 17 |
| Glycerin | 0,5 | 0,5 |
| Zucker gepulvert | Ad 100 | Ad 100 |
| Aroma vom Typ Pfefferminz aus Beispiel 41 | 2 | - |
| Aroma vom Typ Krauseminz aus Beispiel 38 | - | 2 |

### Beispiel 54: Zuckerfreier Kaugummi

| **Bestandteil** | **A** | **B** |
|---|---|---|
| Gum Base (Kaugummibase) | 30 | 30 |
| Sorbit gepulvert | 40 | Ad 100 |
| Isomalt gepulvert | 9,5 | 9,5 |
| Xylitol | 2 | 2 |
| Mannit D | 3 | 3 |
| Aspartam | 0,1 | 0,1 |
| Acesulfam K | 0,1 | 0,1 |
| EmulgumTM (Soja-Lecithine mit hohem Gehalt an Phospholipiden) | 0,3 | 0,3 |
| Sorbitol (70% in Wasser) | 13 | 13 |
| 1,2-Propylenglycol | - | 1 |
| Glycerin | 1 | - |
| Pellitorin-Lösung (enthaltend 10% Pellitorin) | - | 0,035 |
| Aroma vom Typ Pfefferminz aus Beispiel 41 | 1 | 1 |

### Beispiel 55: Kaugummis (mit Zucker und zuckerfrei)

| **Bestandteil** | **A** | **B** |
|---|---|---|
| Gum Base (Kaugummibase) | 21 | 30 |
| Glycerin | 0,5 | 1 |
| Glucosesirup | 16,5 | - |
| Puderzucker | Ad 100 | - |
| Sorbitol (in Pulverform) | - | Ad 100 |
| Palatinit | - | 9,5 |
| Xylitol | - | 2 |
| Mannitol | - | 3 |
| Aspartam | - | 0,1 |
| Acesulfam K | - | 0,1 |
| EmulgumTM (Emulgator) | - | 0,3 |
| Sorbitol 70%, in Wasser | - | 14 |
| Aroma vom Typ Krauseminz aus Beispiel 38 | 1 | 1,4 |

### Beispiel 56: Zuckerfreie Kaugummis

Die Kaugummibase K1 bestand aus 2,0% Butyl Rubber (Isobuten-Isopren-Copolymer, MW = 400000, 6,0% Polyisobuten (MW = 43.800), 43,5% Polyvinylacetat (MW = 12.000), 31,5% Polyvinylacetat (MW = 47.000), 6,75% Triacetin und 10,25% Calciumcarbonat. Die Herstellung der Kaugummibase K1 und der Kaugummis kann analog zu US 5,601,858 erfolgen.

| **Bestandteil** | **A** | **B** | **C** |
|---|---|---|---|
| Kaugummibase K1 | 26 | 27 | 26 |
| Triacetin | 0,25 | 0,25 | 0,25 |
| Lecithin | 0,5 | 0,5 | 0,5 |
| Sorbitol, kristallin | Ad 100 | Ad 100 | Ad 100 |
| Mannitol | 15,3 | 15,2 | 15,1 |
| Glycerin | 12,1 | 12 | 11,8 |
| Saccharin-Na | 0,17 | - | 0,1 |
| Verkapseltes Aspartam | 1,08 | 1,18 | 1,08 |
| Amorphes Silica | 1 | 1 | 1 |
| Baumwollsamenöl | 0,5 | 0,5 | 0,5 |
| Polyoxyethylen-sorbitan-monolaurat (E-432) | 1 | 1 | 1 |
| Verkapseltes /-Carvon (Beladung: 30%) | - | 0,2 | - |
| /-Menthyl-/-lactat | - | - | 0,2 |
| Aroma vom Typ Krauseminz aus Beispiel 38 | 1 | - | 1,7 |
| Aroma vom Typ Pfefferminz aus Beispiel 41 | 0,5 | 1,4 | - |

### Beispiel 57: Bonbon ('Hardboiled candy'), zuckerfrei

| **Bestandteil** | **A** | **B** |
|---|---|---|
| Wasser | 2,24 | 2,24 |
| Isomalt | 94,98 | Ad 100 |
| Xylitol | 2,4 | 2,4 |
| Sucralose | 0,03 | 0,03 |
| Acesulfam K | 0,05 | 0,05 |
| Citronensäure | 0,05 | 0,05 |
| Pellitorin-Lösung (enthaltend 10% Pellitorin) | - | 0,0075 |
| Aroma vom Typ Eucalyptus-Menthol aus Beispiel 37 | 0,25 | 0,2 |

### Beispiel 58: Bonbons ('Hardboiled candy')

| **Bestandteil** | **A** | **B** |
|---|---|---|
| Wasser | 2,75 | 2,5 |
| Zucker | 60,1 | Ad 100 |
| Glucosesirup | 36,9 | 36 |
| Maltose | - | 2 |
| Palmkernöl | - | 0,8 |
| Citronensäure | - | 0,25 |
| Ginseng Extrakt | - | 0,4 |
| Blauer Farbstoff | - | 0,01 |
| Aroma vom Typ Krauseminz aus Beispiel 38 | 0,25 | 0,35 |

## Patentansprüche

1. Riechstoff- und/oder Aromastoffkomposition umfassend eine, zwei, drei oder mehrere Verbindungen der Formel (I), 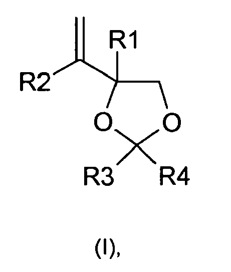 einschließlich der Stereoisomeren, wobei
R1 und R2 jeweils unabhängig voneinander entweder Wasserstoff oder Methyl, vorzugsweise Wasserstoff ist, und
a) R3 und R4 einen Ring mit insgesamt 5, 6, 7 oder 8 Ring-Kohlenstoff-Atomen bilden, wobei der Ring optional eine oder zwei Doppelbindungen und/oder optional 1 bis maximal 3 Sauerstoffatome enthält, und wobei der Ring optional substituiert ist mit einer oder mehreren verzweigten oder unverzweigten, verbrückten oder unverbrückten, Alkylgruppen, Alkenylgruppen, Cycloalkylgruppen, Cycloalkenylgruppen, Arylgruppen, Arylalkylgruppen, Alkoxyalkylgruppen oder Alkoxyarylgruppen, und wobei R3 und R4 zusammen insgesamt 3 bis 30 Kohlenstoff-Atome umfassen,
oder
b) R3 entweder Wasserstoff oder ein organischer Rest mit 1 bis 15 Kohlenstoff-Atomen ist, wobei der organische Rest optional 1 bis maximal 3 Sauerstoff-Atome enthält, vorzugsweise R3 entweder Wasserstoff oder ein gesättigtes oder ungesättigtes, aromatisches oder aliphatisches, verzweigtes oder unverzweigtes, cyclisches oder lineares, verbrücktes oder unverbrücktes Strukturelement mit 1 bis 15 Kohlenstoff-Atomen ist, wobei das Strukturelement optional 1 bis maximal 3 Sauerstoff-Atome enthält und das Strukturelement optional substituiert ist mit 1 bis maximal 3 Alkyl- und/oder Alkenylgruppen und/oder mit einer oder zwei Hydroxygruppen,
und
c) R4 ein organischer Rest mit 2 bis 15 Kohlenstoff-Atomen ist, wobei der organische Rest optional 1 bis maximal 3 Sauerstoff-Atome enthält, vorzugsweise R4 ein gesättigtes oder ungesättigtes, aromatisches oder aliphatisches, verzweigtes oder unverzweigtes, cyclisches oder lineares, verbrücktes oder unverbrücktes Strukturelement mit 2 bis 15 Kohlenstoff-Atomen ist, wobei das Strukturelement optional 1 bis maximal 3 Sauerstoff-Atome enthält und das Strukturelement optional substituiert ist mit 1 bis maximal 3 Alkyl- und/oder Alkenylgruppen und/oder mit einer oder zwei Hydroxygruppen,
und wobei wenigstens eine der folgenden Bedingungen gilt:
i. sofern R4 ein verzweigter oder unverzweigter Alkyl-Rest mit 4 bis 15 Kohlenstoff-Atomen mit optional ein, zwei oder mehreren Doppelbindungen ist, an Position 3 zu Position 4 von R4 eine Kohlenstoff-Kohlenstoff-Einfachbindung vorliegt
oder
ii. sofern R4 ein verzweigter oder unverzweigter Alkyl-Rest mit 2 bis 15 Kohlenstoff-Atomen mit optional ein, zwei oder mehreren Doppelbindungen ist, R3 nicht Wasserstoff ist,
und wobei die Riechstoff- und/oder Aromastoffkomposition zumindest 3, 4, 5, 6, 7, 8 oder mehr weitere Riech- und/oder Aromastoffe enthält, bei denen es sich jeweils nicht um eine Verbindung der Formel (I) handelt,
und wobei die Riechstoff- und/oder Aromastoffkomposition nicht 2-Hexyl-4-vinyl-1,3-dioxolan enthält.

2. Riechstoff- und/oder Aromastoffkomposition nach Anspruch 1, wobei
R1 und R2 jeweils unabhängig voneinander entweder Wasserstoff oder Methyl, jeweils vorzugsweise Wasserstoff ist,
und
a) R3 und R4 zusammen ein cyclisches Alkylsystem mit 5, 6, 7 oder 8 Kohlenstoff-Atomen ergeben, das optional substituiert ist mit einem oder mehreren verzweigten oder unverzweigten Alkyl-Resten ausgewählt aus der Gruppe bestehend aus Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, und wobei das Alkylsystem optional eine oder zwei Doppelbindungen enthält,
oder
b) R3 entweder Wasserstoff oder ein verzweigter oder unverzweigter Alkyl-Rest mit 1 bis 15 Kohlenstoff-Atomen und optional ein, zwei oder mehreren Doppelbindungen oder ein cyclisches Alkylsystem mit 5, 6 oder 7 Kohlenstoff-Atomen mit optional ein, zwei oder mehreren Doppelbindungen ist, wobei der Alkyl-Rest vorzugsweise ausgewählt ist aus der Gruppe bestehend aus Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl,
und
1. R4 ein Aromat ist, wobei der Aromat optional mit einem, zwei oder mehreren Alkyl-Resten mit 1 bis 3 Kohlenstoff-Atomen und/oder einem, zwei oder mehreren Hydroxy-Resten und/oder einem, zwei oder mehreren Alkoxy-Resten substituiert ist,
oder
2. R4 ein mono-, bi- oder tricyclisches Alkylsystem ist, das optional mit einem, zwei oder mehreren Alkyl-Resten, Hydroxy-Resten oder Alkoxy-Resten substituiert ist, wobei die Alkyl-Reste vorzugsweise ausgewählt sind aus der Gruppe bestehend aus Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl,
oder
3. R4 ein verzweigter oder unverzweigter Alkyl-Rest mit 2 bis 15 Kohlenstoff-Atomen mit optional ein, zwei oder mehreren Doppelbindungen oder ein Ringsystem mit 5, 6 oder 7 Kohlenstoff-Atomen mit optional ein, zwei oder mehreren Doppelbindungen, ist.

3. Riechstoff- und/oder Aromastoffkomposition gemäß Anspruch 1 oder 2, **dadurch gekennzeichnet, dass** die Riechstoff- und/oder Aromastoffkomposition eine, zwei, drei oder mehrere Verbindungen der Formel (II): 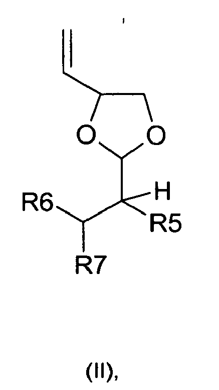 einschließlich der Stereoisomeren, umfasst, wobei
a) R5 und R6 jeweils unabhängig voneinander entweder Wasserstoff oder Methyl ist,
und
b) R7 ein organischer Rest mit 1 bis 10 Kohlenstoff-Atomen ist, wobei der organische Rest optional 1 bis maximal 3 Sauerstoff-Atome enthält, vorzugsweise R7 ein gesättigtes oder ungesättigtes, aromatisches oder aliphatisches, verzweigtes oder unverzweigtes, cyclisches oder lineares, verbrücktes oder unverbrücktes Strukturelement mit 1 bis 10 Kohlenstoff-Atomen ist, wobei das Strukturelement optional 1 bis maximal 3 Sauerstoff-Atome enthält und das Strukturelement optional substituiert ist mit 1 bis maximal 3 Alkyl- und/oder Alkenylgruppen und/oder mit einer Hydroxygruppe,
und
wobei sofern R7 ein verzweigter oder unverzweigter Alkyl-Rest mit 1 bis 10 Kohlenstoff-Atomen ist, an Position 1 zu Position 2 von R7 eine Kohlenstoff-Kohlenstoff-Einfachbindung vorliegt.

4. Riechstoff- und/oder Aromastoffkomposition gemäß Anspruch 3, **dadurch gekennzeichnet, dass** die Riechstoff- und/oder Aromastoffkomposition eine, zwei, drei oder mehrere Verbindungen der Formel (II) umfasst, wobei
a) R5 und R6 jeweils unabhängig voneinander entweder Wasserstoff oder Methyl ist, und
b) R7 ein verzweigter oder unverzweigter Alkyl-Rest mit 1 bis 10 Kohlenstoff-Atomen, vorzugsweise ausgewählt aus der Gruppe bestehend aus Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, ist,
oder
R7 ein Cyloalkyl-Rest mit 5, 6 oder 7 Kohlenstoff-Atomen ist, der optional substituiert ist mit einem oder mehreren Alkyl-Resten, ausgewählt aus der Gruppe bestehend aus Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, einem Hydroxy-Rest oder einem Alkoxy-Rest.

5. Riechstoff- und/oder Aromastoffkomposition gemäß einem vorangehenden Ansprüche, **dadurch gekennzeichnet, dass** die Riechstoff- und/oder Aromastoffkomposition eine, zwei, drei oder mehrere der Verbindungen, ausgewählt aus der Gruppe bestehend aus:
- 2-Isobutyl-4-vinyl-[1,3]dioxolan,
- 2-secButyl-4-vinyl-[1,3]dioxolan,
- 2-Isopropyl-4-vinyl-[1,3]dioxolan,
- 2-(3,5-Dimethylcyclohex-3-enyl)-4-vinyl-[1,3]dioxolan,
- 2-(2,4-Dimethylcyclohex-3-enyl)-4-vinyl-[1,3]dioxolan,
- 2-(2,6-Dimethylhept-5-enyl)-4-vinyl-[1,3]dioxolan,
- 2-Ethyl-2-(2-methylbutyl)-4-vinyl-[1,3]dioxolan,
- 2-(2,4,4-Trimethylpentyl)-4-vinyl-[1,3]dioxolan,
- 2-(3,5-Dimethylhex-4-enyl)-2-methyl-4-vinyl-[1,3]dioxolan,
- 2-Methyl-2-(4-methylpent-3-enyl)-4-vinyl-[1,3]dioxolan,
- 8-tertButyl-2-vinyl-1,4-dioxaspiro-[4.5]-decan,
- 2-(2,6-Dimethylhepta-1,5-dienyl-4-vinyl-[1,3]dioxolan,
- 2-[2-(4-Methylcyclohex-3-enyl)-propyl]-4-vinyl-[1,3]dioxolan,
- 2-Phenyl-4-vinyl-[1,3]dioxolan,
- 2-Cyclohexyl-4-vinyl-[1,3]dioxolan,
- 2-(2,2,3-Trimethylcyclopent-3-enylmethyl)-4-vinyl-[1,3]dioxolan,
- 2-Vinyl-1,4-dioxaspiro[4.5]decan,
- 7-Methyl-2-vinyl-1,4-dioxaspiro[4.5]decan,
- 2-(2-Methylpropenyl)-4-vinyl-[1,3]dioxolan, und
- 2-Methyl-2-(3-methylbutyl)-4-vinyl-[1,3]dioxolan.
umfasst.

6. Riechstoff- und/oder Aromastoffkomposition gemäß einem der vorherigen Ansprüche, **dadurch gekennzeichnet, dass** die Riechstoff- und/oder Aromastoffkomposition eine Gesamtmenge an Verbindungen der Formel (I) und/oder (II) von 0,0001 bis 70 Gew.-%, vorzugsweise 0,001 bis 50 Gew.-% und besonders bevorzugt 0,01 bis 20 Gew.-% umfasst, jeweils bezogen auf die Gesamtmenge an Riech- und/oder Aromastoffen, wobei die Riechstoff- oder Aromastoffkomposition vorzugsweise einen, zwei, drei, vier, fünf oder mehrere Riech- und/oder Aromastoffe umfasst, die eine blumige und/oder fruchtige Geruchs- oder Geschmacksnote vermitteln.

7. 2-(2,6-Dimethylhept-5-enyl)-4-vinyl-[1,3]dioxolan

8. Verwendung einer Verbindung der Formel (I) 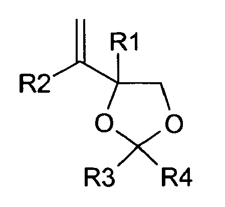 (I), einschließlich der Stereoisomeren,
wobei
R1 und R2 jeweils unabhängig voneinander entweder Wasserstoff oder Methyl, vorzugsweise Wasserstoff ist, und
a) R3 und R4 einen Ring mit insgesamt 5, 6, 7 oder 8 Ring-Kohlenstoff-Atomen bilden, wobei der Ring optional eine oder zwei Doppelbindungen und/oder optional 1 bis maximal 3 Sauerstoffatome enthält, und wobei der Ring optional substituiert ist mit einer oder mehreren verzweigten oder unverzweigten, verbrückten oder unverbrückten, Alkylgruppen, Alkenylgruppen, Cycloalkylgruppen, Cycloalkenylgruppen, Arylgruppen, Arylalkylgruppen, Alkoxyalkylgruppen oder Alkoxyarylgruppen, und wobei R3 und R4 zusammen insgesamt 3 bis 30 Kohlenstoff-Atome umfassen,
oder
b) R3 entweder Wasserstoff oder ein organischer Rest mit 1 bis 15 Kohlenstoff-Atomen ist, wobei der organische Rest optional 1 bis maximal 3 Sauerstoff-Atome enthält, vorzugsweise R3 entweder Wasserstoff oder ein gesättigtes oder ungesättigtes, aromatisches oder aliphatisches, verzweigtes oder unverzweigtes, cyclisches oder lineares, verbrücktes oder unverbrücktes Strukturelement mit 1 bis 15 Kohlenstoff-Atomen ist, wobei das Strukturelement optional 1 bis maximal 3 Sauerstoff-Atome enthält und das Strukturelement optional substituiert ist mit 1 bis maximal 3 Alkyl- und/oder Alkenylgruppen und/oder mit einer oder zwei Hydroxygruppen,
und
R4 ein organischer Rest mit 2 bis 15 Kohlenstoff-Atomen ist, wobei der organische Rest optional 1 bis maximal 3 Sauerstoff-Atome enthält, vorzugsweise R4 ein gesättigtes oder ungesättigtes, aromatisches oder aliphatisches, verzweigtes oder unverzweigtes, cyclisches oder lineares, verbrücktes oder unverbrücktes Strukturelement mit 2 bis 15 Kohlenstoff-Atomen ist, wobei das Strukturelement optional 1 bis maximal 3 Sauerstoff-Atome enthält und das Strukturelement optional substituiert ist mit 1 bis maximal 3 Alkyl- und/oder Alkenylgruppen und/oder mit einer oder zwei Hydroxygruppen und wobei wenigstens eine der folgenden Bedingungen gilt:
i. sofern R4 ein verzweigter oder unverzweigter Alkyl-Rest mit 4 bis 15 Kohlenstoff-Atomen mit optional ein, zwei oder mehreren Doppelbindungen ist, an Position 3 zu Position 4 von R4 eine Kohlenstoff-Kohlenstoff-Einfachbindung vorliegt
oder
ii. sofern R4 ein verzweigter oder unverzweigter Alkyl-Rest mit 2 bis 15 Kohlenstoff-Atomen mit optional ein, zwei oder mehreren Doppelbindungen ist, R3 nicht Wasserstoff ist,
als Riech- und/oder Aromastoff zum Vermitteln, Verstärken und/oder Modifizieren einer, zweier oder mehrerer Geruchs- und/oder Geschmacksnoten, wobei keine Geruchs- und/oder Geschmacksnote Ananas vermittelt wird.

9. Verwendung nach Anspruch 8, wobei
R1 und R2 jeweils unabhängig voneinander entweder Wasserstoff oder Methyl, vorzugsweise Wasserstoff ist
und
a) R3 und R4 zusammen ein cyclisches Alkylsystem mit 5, 6, 7 oder 8 Kohlenstoff-Atomen ergeben, das optional substituiert ist mit einem oder mehreren verzweigten oder unverzweigten Alkyl-Resten ausgewählt aus der Gruppe bestehend aus Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, und wobei das Alkylsystem optional eine oder zwei Doppelbindungen enthält,
oder
b) R3 entweder Wasserstoff oder ein verzweigter oder unverzweigter Alkyl-Rest mit 1 bis 15 Kohlenstoff-Atomen und optional ein, zwei oder mehreren Doppelbindungen oder ein cyclisches Alkylsystem mit 5, 6 oder 7 Kohlenstoff-Atomen mit optional ein, zwei oder mehreren Doppelbindungen ist, wobei der Alkyl-Rest vorzugsweise ausgewählt ist aus der Gruppe bestehend aus Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl,
und
1. R4 ein Aromat ist, wobei der Aromat optional mit einem, zwei oder mehreren Alkyl-Resten mit 1 bis 3 Kohlenstoff-Atomen und/oder einem, zwei oder mehreren Hydroxy-Resten und/oder einem, zwei oder mehreren Alkoxy-Resten substituiert ist,
oder
2. R4 ein mono-, bi- oder tricyclisches Alkylsystem ist, das optional mit einem, zwei oder mehreren Alkyl-Resten, Hydroxy-Resten oder Alkoxy-Resten substituiert ist, wobei die Alkyl-Reste vorzugsweise ausgewählt sind aus der Gruppe bestehend aus Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl,
oder
3. R4 ein verzweigter oder unverzweigter Alkyl-Rest mit 2 bis 15 Kohlenstoff-Atomen mit ein, zwei oder mehreren Doppelbindungen oder ein Ringsystem mit 5, 6 oder 7 Kohlenstoff-Atomen mit ein, zwei oder mehreren Doppelbindungen, ist,
und wobei wenigstens eine der folgenden Bedingungen gilt:
i. sofern R4 ein verzweigter oder unverzweigter Alkyl-Rest mit 4 bis 15 Kohlenstoff-Atomen mit optional ein, zwei oder mehreren Doppelbindungen ist, an Position 3 zu Position 4 von R4 eine Kohlenstoff-Kohlenstoff-Einfachbindung vorliegt
oder
ii. sofern R4 ein verzweigter oder unverzweigter Alkyl-Rest mit 2 bis 15 Kohlenstoff-Atomen mit optional ein, zwei oder mehreren Doppelbindungen ist, R3 ein verzweigter oder unverzweigter Alkyl-Rest mit 1 bis 15 Kohlenstoff-Atomen und optional ein, zwei oder mehreren Doppelbindungen ist,
vorzugweise einer Verbindung gemäß Formel (II) 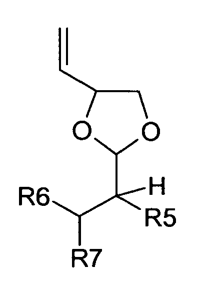 (II), einschließlich der Stereoisomeren,
wobei
a) R5 und R6 jeweils unabhängig voneinander entweder Wasserstoff oder Methyl ist,
und
b) R7 ein verzweigter oder unverzweigter Alkyl-Rest mit 1 bis 10 Kohlenstoff-Atomen, vorzugsweise ausgewählt aus der Gruppe bestehend aus Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, ist,
oder
R7 ein Cyloalkyl-Rest mit 5, 6 oder 7 Kohlenstoff-Atomen ist, der optional substituiert ist mit einem oder mehreren Alkyl-Resten, ausgewählt aus der Gruppe bestehend aus Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, einem Hydroxy-Rest oder einem Alkoxy-Rest,
wobei sofern R7 ein verzweigter oder unverzweigter Alkyl-Rest mit 1 bis 10 Kohlenstoff-Atomen ist, an Position 1 zu Position 2 von R7 eine Kohlenstoff-Kohlenstoff-Einfachbindung vorliegt.

10. Verwendung gemäß Anspruch 8 oder 9, wobei die Verbindung ausgewählt ist aus der Gruppe bestehend aus:
- 2-Isobutyl-4-vinyl-[1,3]dioxolan,
- 2-secButyl-4-vinyl-[1,3]dioxolan,
- 2-Isopropyl-4-vinyl-[1,3]dioxolan,
- 2-(3,5-Dimethylcyclohex-3-enyl)-4-vinyl-[1,3]dioxolan,
- 2-(2,4-Dimethylcyclohex-3-enyl)-4-vinyl-[1,3]dioxolan,
- 2-(2,6-Dimethylhept-5-enyl)-4-vinyl-[1,3]dioxolan,
- 2-Ethyl-2-(2-methylbutyl)-4-vinyl-[1,3]dioxolan,
- 2-(2,4,4-Trimethylpentyl)-4-vinyl-[1,3]dioxolan,
- 2-(3,5-Dimethylhex-4-enyl)-2-methyl-4-vinyl-[1,3]dioxolan,
- 2-Methyl-2-(4-methylpent-3-enyl)-4-vinyl-[1,3]dioxolan,
- 8-*tert*Butyl-2-vinyl-1,4-dioxaspiro-[4.5]-decan,
- 2-(2,6-Dimethylhepta-1,5-dienyl-4-vinyl-[1,3]dioxolan,
- 2-[2-(4-Methylcyclohex-3-enyl)-propyl]-4-vinyl-[1,3]dioxolan,
- 2-Phenyl-4-vinyl-[1,3]dioxolan,
- 2-Cyclohexyl-4-vinyl-[1,3]dioxolan,
- 2-(2,2,3-Trimethylcyclopent-3-enylmethyl)-4-vinyl-[1,3]dioxolan,
- 2-Vinyl-1,4-dioxaspiro[4.5]decan,
- 7-Methyl-2-vinyl-1,4-dioxaspiro[4.5]decan,
- 2-(2-Methylpropenyl)-4-vinyl-[1,3]dioxolan, und
- 2-Methyl-2-(3-methylbutyl)-4-vinyl-[1,3]dioxolan.

11. Verwendung gemäß einem der Ansprüche 8 bis 10 zum Vermitteln, Verstärken und/oder Modifizieren einer, zweier, dreier oder mehrerer der Geruchs- und/oder Geschmacksnoten blumig, fettig, grüne Oliven, ledrig, Frucht, Grün, Blüte, Rose, Myrrhe, Birne, Mango, Grapefruit, Melone, Zitrone, Linalool, Mirabelle, Krauseminz, Phenolisch, Pilz, Metallisch, Kamille, Apfel, Carbinol, Aromatisch, Wässrig, Frisch, Kraut.

12. Parfümierter und/oder aromatisierter Artikel, umfassend eine Riechstoff- und/oder Aromastoffkomposition nach einem der Ansprüche 1 bis 5 und/oder eine Verbindung nach Anspruch 6 oder 7,
wobei der parfümierte Artikel vorzugsweise ausgewählt ist aus der Gruppe bestehend aus Waschmitteln, Hygiene- oder Pflegeprodukten, insbesondere im Bereich der Körper- und Haarpflege, der Kosmetik und des Haushalts, und der aromatisierte Artikel vorzugsweise ausgewählt ist aus der Gruppe bestehend aus Lebensmitteln, Genussmitteln, Getränken, Mundpflegeprodukten oder pharmazeutischen Produkten.

13. Verfahren zum Vermitteln, Modifizieren und/oder Verstärken eines Geruchs und/oder Geschmacks ohne Vermittlung des Geruchs und/oder Geschmacks der Note Ananas, wobei eine sensorisch wirksame Menge
einer Riechstoff- und/oder Aromastoffkomposition gemäß einem der Ansprüche 1 bis 6 oder
- einer Verbindung gemäß Anspruch 7 mit einem Erzeugnis in Kontakt gebracht oder gemischt wird,
vorzugsweise zum Vermitteln, Modifizieren und/oder Verstärken einer, zweier oder mehrerer der Geruchs- und/oder Geschmacksnoten blumig, fettig, grüne Oliven, ledrig, Frucht, Grün, Blüte, Rose, Myrrhe, Birne, Mango, Grapefruit, Melone, Zitrone, Linalool, Mirabelle, Krauseminz, Phenolisch, Pilz, Metallisch, Kamille, Apfel, Carbinol, Aromatisch, Wässrig, Frisch, Kraut.

## Claims

1. Fragrance and/or flavoring composition comprising one, two, three or more compounds of formula (I), 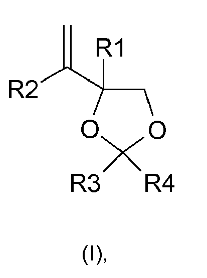 including the stereoisomers, wherein
R1 and R2 in each case independent of each other are either hydrogen or methyl, preferably hydrogen, and
a) R3 and R4 form a ring with 5, 6, 7 or 8 ring carbon atoms in total, wherein the ring optionally comprises one or two double bonds and/or optionally 1 to maximum 3 oxygen atoms, and wherein the ring is optionally substituted with one or more branched or unbranched, bridged or unbridged, alkyl groups, alkenyl groups, cycloalkyl groups, cycloalkenyl groups, aryl groups, arylalkyl groups, alkoxyalkyl groups or alkoxyaryl groups, and wherein R3 and R4 together comprise 3 to 30 carbon atoms in total,
or
b) R3 is either hydrogen or an organic residue with 1 to 15 carbon atoms, wherein the organic residue optionally comprises 1 to maximum 3 oxygen atoms, preferably R3 is either hydrogen or a saturated or unsaturated, aromatic or aliphatic, branched or unbranched, cyclic or linear, bridged or unbridged structural element with 1 to 15 carbon atoms, wherein the structural element optionally comprises 1 to maximum 3 oxygen atoms and the structural element is optionally substituted with 1 to maximum 3 alkyl and/or alkenyl groups and/or with one or two hydroxy groups,
and
c) R4 is an organic residue with 2 to 15 carbon atoms, wherein the organic residue optionally comprises 1 to maximum 3 oxygen atoms, preferably R4 is a saturated or unsaturated, aromatic or aliphatic, branched or unbranched, cyclic or linear, bridged or unbridged structural element with 2 to 15 carbon atoms, wherein the structural element optionally comprises 1 to maximum 3 oxygen atoms and the structural element is optionally substituted with 1 to maximum 3 alkyl and/or alkenyl groups and/or with one or two hydroxy groups,
and wherein at least one of the following conditions applies:
i. if R4 is a branched or unbranched alkyl residue with 4 to 15 carbon atoms with optionally one, two or more double bonds, there is a carbon-carbon single bond at position 3 to position 4 of R4
or
ii. if R4 is a branched or unbranched alkyl residue with 2 to 15 carbon atoms
with optionally one, two or more double bonds, R3 is not hydrogen, und wherein the fragrance and/or flavoring composition comprises at least 3, 4, 5, 6, 7, 8 or more further fragrances and/or flavorings, each of these not being a compound of formula (I),
and wherein the fragrance and/or flavoring composition does not contain 2-hexyl-4-vinyl-1,3-dioxolane.

2. Fragrance and/or flavoring composition according to claim 1, wherein
R1 and R2 in each case independent of each other are either hydrogen or methyl, preferably in each case hydrogen,
and
a) R3 and R4 together form a cyclic alkyl system with 5, 6, 7 or 8 carbon atoms, which is optionally substituted with one or more branched or unbranched alkyl residues selected from the group consisting of methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl, and wherein the alkyl system optionally comprises one or two double bonds,
or
b) R3 is either hydrogen or a branched or unbranched alkyl residue with 1 to 15 carbon atoms and optionally one, two or more double bonds or a cyclic alkyl system with 5, 6 or 7 carbon atoms with optionally one, two or more double bonds, wherein the alkyl residue is preferably selected from the group consisting of methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl,
and
1. R4 is an aromatic compound, wherein the aromatic compound is optionally substituted with one, two or more alkyl residues with 1 to 3 carbon atoms and/or one, two or more hydroxy residues and/or one, two or more alkoxy residues,
or
2. R4 is a mono-, bi- or tri-cyclic alkyl system, which is optionally substituted with one, two or more alkyl residues, hydroxy residues or alkoxy residues, wherein the alkyl residues are preferably selected from the group consisting of methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl,
or
3. R4 is a branched or unbranched alkyl residue with 2 to 15 carbon atoms with optionally one, two or more double bonds or a ring system with 5, 6 or 7 carbon atoms with optionally one, two or more double bonds.

3. Fragrance and/or flavoring composition according to claim 1 or 2, **characterized in that** the fragrance and/or flavoring composition comprises one, two, three or more compounds of formula (II): 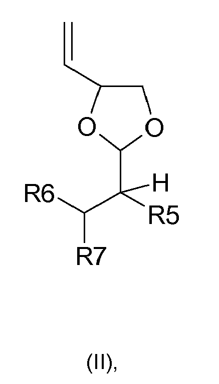 including the stereoisomers, wherein
a) R5 and R6 in each case independent of each other are either hydrogen or methyl,
and
b) R7 is an organic residue with 1 to 10 carbon atoms, wherein the organic residue optionally comprises 1 to maximum 3 oxygen atoms, preferably R7 is a saturated or unsaturated, aromatic or aliphatic, branched or unbranched, cyclic or linear, bridged or unbridged structural element with 1 to 10 carbon atoms, wherein the structural element optionally comprises 1 to maximum 3 oxygen atoms and the structural element is optionally substituted with 1 to maximum 3 alkyl and/or alkenyl groups and/or with a hydroxy group,
and
wherein if R7 is a branched or unbranched alkyl residue with 1 to 10 carbon atoms, there is a carbon-carbon single bond at position 1 to position 2 of R7.

4. Fragrance and/or flavoring composition according to claim 3, **characterized in that** the fragrance and/or flavoring composition comprises one, two, three or more compounds of formula (II), wherein
a) R5 and R6 in each case independent of each other are either hydrogen or methyl,
and
b) R7 is a branched or unbranched alkyl residue with 1 to 10 carbon atoms, preferably selected from the group consisting of methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl,
or
R7 is a cycloalkyl residue with 5, 6 or 7 carbon atoms, which is optionally substituted with one or more alkyl residues selected from the group consisting of methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl, a hydroxy residue or an alkoxy residue.

5. Fragrance and/or flavoring composition according one of the preceding claims, **characterized in that** the fragrance and/or flavoring composition comprises one, two, three or more of the compounds selected from the group consisting of:
- 2-isobutyl-4-vinyl-[1,3]dioxolane,
- 2-secbutyl-4-vinyl-[1,3]dioxolane,
- 2-isopropyl-4-vinyl-[1,3]dioxolane,
- 2-(3,5-dimethylcyclohex-3-enyl)-4-vinyl-[1,3]dioxolane,
- 2-(2,4-dimethylcyclohex-3-enyl)-4-vinyl-[1,3]dioxolane,
- 2-(2,6-dimethylhept-5-enyl)-4-vinyl-[1,3]dioxolane,
- 2-ethyl-2-(2-methylbutyl)-4-vinyl-[1,3]dioxolane,
- 2-(2,4,4-trimethylpentyl)-4-vinyl-[1,3]dioxolane,
- 2-(3,5-dimethylhex-4-enyl)-2-methyl-4-vinyl-[1,3]dioxolane,
- 2-methyl-2-(4-methylpent-3-enyl)-4-vinyl-[1,3]dioxolane,
- 8-tertbutyl-2-vinyl-1,4-dioxaspiro-[4.5]-decane,
- 2-(2,6-dimethylhepta-1,5-dienyl-4-vinyl-[1,3]dioxolane,
- 2-[2-(4-methylcyclohex-3-enyl)-propyl]-4-vinyl-[1,3]dioxolane,
- 2-phenyl-4-vinyl-[1,3]dioxolane,
- 2-cyclohexyl-4-vinyl-[1,3]dioxolane,
- 2-(2,2,3-trimethylcyclopent-3-enylmethyl)-4-vinyl-[1,3]dioxolane,
- 2-vinyl-1,4-dioxaspiro[4.5]decane,
- 7-methyl-2-vinyl-1,4-dioxaspiro[4.5]decane,
- 2-(2-methylpropenyl)-4-vinyl-[1,3]dioxolane, and
- 2-methyl-2-(3-methylbutyl)-4-vinyl-[1,3]dioxolane.

6. Fragrance and/or flavoring composition according one of the preceding claims, **characterized in that** the fragrance and/or flavoring composition comprises a total amount of compounds of formula (I) and/or (II) from 0,0001 to 70 wt.-%, preferably 0,001 to 50 wt.-% and particularly preferred 0,01 to 20 wt.-%, in each case with respect to the total amount of fragrances and/or flavorings, wherein the fragrance and/or flavoring composition preferably comprises one, two, three, four, fife or more fragrances and/or flavorings, which convey a flowery and/or fruity fragrance or flavoring note.

7. 2-(2,6-Dimethylhept-5-enyl)-4-vinyl-[1,3]dioxolane

8. Use of a compound of formula (I) 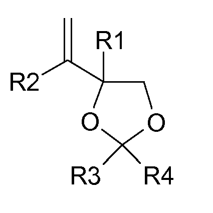 (I), including the stereoisomers,
wherein
R1 and R2 in each case independent of each other are either hydrogen or methyl, preferably hydrogen, and
a) R3 and R4 form a ring with 5, 6, 7 or 8 ring carbon atoms in total, wherein the ring optionally comprises one or two double bonds and/or optionally 1 to maximum 3 oxygen atoms, and wherein the ring is optionally substituted with one or more branched or unbranched, bridged or unbridged, alkyl groups, alkenyl groups, cycloalkyl groups, cycloalkenyl groups, aryl groups, arylalkyl groups, alkoxyalkyl groups or alkoxyaryl groups, and wherein R3 and R4 together comprise 3 to 30 carbon atoms in total,
or
b) R3 is either hydrogen or an organic residue with 1 to 15 carbon atoms, wherein the organic residue optionally comprises 1 to maximum 3 oxygen atoms, preferably R3 is either hydrogen or a saturated or unsaturated, aromatic or aliphatic, branched or unbranched, cyclic or linear, bridged or unbridged structural element with 1 to 15 carbon atoms, wherein the structural element optionally comprises 1 to maximum 3 oxygen atoms and the structural element is optionally substituted with 1 to maximum 3 alkyl and/or alkenyl groups and/or with one or two hydroxy groups,
and
R4 is an organic residue with 2 to 15 carbon atoms, wherein the organic residue optionally comprises 1 to maximum 3 oxygen atoms, preferably R4 is a saturated or unsaturated, aromatic or aliphatic, branched or unbranched, cyclic or linear, bridged or unbridged structural element with 2 to 15 carbon atoms, wherein the structural element optionally comprises 1 to maximum 3 oxygen atoms and the structural element is optionally substituted with 1 to maximum 3 alkyl and/or alkenyl groups and/or with one or two hydroxy groups,
and wherein at least one of the following conditions applies:
i. if R4 is a branched or unbranched alkyl residue with 4 to 15 carbon atoms with optionally one, two or more double bonds, there is a carbon-carbon single bond at position 3 to position 4 of R4
or
ii. if R4 is a branched or unbranched alkyl residue with 2 to 15 carbon atoms with optionally one, two or more double bonds, R3 is not hydrogen,
as fragrance and/or flavoring to convey, enhance and/or modify one, two or more fragrance or flavoring notes, wherein no fragrance or flavoring note pineapple is conveyed.

9. Use according to claim 8, wherein
R1 and R2 in each case independent of each other are either hydrogen or methyl, preferably hydrogen,
and
a) R3 and R4 together form a cyclic alkyl system with 5, 6, 7 or 8 carbon atoms, which is optionally substituted with one or more branched or unbranched alkyl residues selected from the group consisting of methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl, and wherein the alkyl system optionally comprises one or two double bonds,
or
b) R3 is either hydrogen or a branched or unbranched alkyl residue with 1 to 15 carbon atoms and optionally one, two or more double bonds or a cyclic alkyl system with 5, 6 or 7 carbon atoms with optionally one, two or more double bonds, wherein the alkyl residue is preferably selected from the group consisting of methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl,
and
1. R4 is an aromatic compound, wherein the aromatic compound is optionally substituted with one, two or more alkyl residues with 1 to 3 carbon atoms and/or one, two or more hydroxy residues and/or one, two or more alkoxy residues,
or
2. R4 is a mono-, bi- or tri-cyclic alkyl system, which is optionally substituted with one, two or more alkyl residues, hydroxy residues or alkoxy residues, wherein the alkyl residues are preferably selected from the group consisting of methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl,
or
3. R4 is a branched or unbranched alkyl residue with 2 to 15 carbon atoms with optionally one, two or more double bonds or a ring system with 5, 6 or 7 carbon atoms with optionally one, two or more double bonds,
and wherein at least one of the following conditions applies:
i. if R4 is a branched or unbranched alkyl residue with 4 to 15 carbon atoms with optionally one, two or more double bonds, there is a carbon-carbon single bond at position 3 to position 4 of R4
or
ii. if R4 is a branched or unbranched alkyl residue with 2 to 15 carbon atoms with optionally one, two or more double bonds, R3 is a branched or unbranched alkyl residue with 1 to 15 carbon atoms and optionally one, two or more double bonds,
preferably of a compound of formula (II) 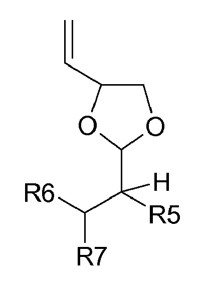 (II), including the stereoisomers,
wherein
a) R5 and R6 in each case independent of each other are either hydrogen or methyl,
and
b) R7 is a branched or unbranched alkyl residue with 1 to 10 carbon atoms, preferably selected from the group consisting of methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl,
or
R7 is a cycloalkyl residue with 5, 6 or 7 carbon atoms, which is optionally substituted with one or more alkyl residues selected from the group consisting of methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl, a hydroxy residue or an alkoxy residue,
wherein if R7 is a branched or unbranched alkyl residue with 1 to 10 carbon atoms, there is a carbon-carbon single bond at position 1 to position 2 of R7.

10. Use according to claim 8 or 9, wherein the compound is selected from the group consisting of:
- 2-isobutyl-4-vinyl-[1,3]dioxolane,
- 2-secbutyl-4-vinyl-[1,3]dioxolane,
- 2-isopropyl-4-vinyl-[1,3]dioxolane,
- 2-(3,5-dimethylcyclohex-3-enyl)-4-vinyl-[1,3]dioxolane,
- 2-(2,4-dimethylcyclohex-3-enyl)-4-vinyl-[1,3]dioxolane,
- 2-(2,6-dimethylhept-5-enyl)-4-vinyl-[1,3]dioxolane,
- 2-ethyl-2-(2-methylbutyl)-4-vinyl-[1,3]dioxolane,
- 2-(2,4,4-trimethylpentyl)-4-vinyl-[1,3]dioxolane,
- 2-(3,5-dimethylhex-4-enyl)-2-methyl-4-vinyl-[1,3]dioxolane,
- 2-methyl-2-(4-methylpent-3-enyl)-4-vinyl-[1,3]dioxolane,
- 8-tertnutyl-2-vinyl-1,4-dioxaspiro-[4.5]-decane,
- 2-(2,6-dimethylhepta-1,5-dienyl-4-vinyl-[1,3]dioxolane,
- 2-[2-(4-methylcyclohex-3-enyl)-propyl]-4-vinyl-[1,3]dioxolane,
- 2-phenyl-4-vinyl-[1,3]dioxolane,
- 2-cyclohexyl-4-vinyl-[1,3]dioxolane,
- 2-(2,2,3-trimethylcyclopent-3-enylmethyl)-4-vinyl-[1,3]dioxolane,
- 2-vinyl-1,4-dioxaspiro[4.5]decane,
- 7-methyl-2-vinyl-1,4-dioxaspiro[4.5]decane,
- 2-(2-methylpropenyl)-4-vinyl-[1,3]dioxolane, and
- 2-methyl-2-(3-methylbutyl)-4-vinyl-[1,3]dioxolane.

11. Use according to one of claims 8 to 10 to convey, enhance and/or modify one, two, three or more of the fragrance or flavoring notes flowery, fatty, green olive, leathery, fruit, green, blossom, rose, myrrh, pear, mango, grapefruit, melon, lemon, linalool, mirabelle, brook mint, phenolic, mushroom, metallic, chamomile, apple, carbinol, aromatic, watery, fresh, herb.

12. Perfumed and/or flavored article, comprising a fragrance and/or flavoring composition according to one of the claims 1 to 5 and/or a compound according to claim 6 or 7,
wherein the perfumed article is preferably selected from the group consisting of laundry soap, hygiene or care products, in the field of body and hair care, cosmetics and housekeeping and the flavored article is preferably selected from the group consisting of food stuff, semi-luxury products, beverages, mouth care products or pharmaceutic products.

13. Method to convey, modify and/or enhance a fragrance or flavor without conveying the fragrance or flavoring note of pineapple, wherein a sensory effective amount of a fragrance and/or flavoring composition according to one of the claims 1 to 6
or
- a compound according to claim 7 is contacted or mixed with a product, preferably to convey, modify and/or enhance one, two, three or more of the fragrance or flavoring notes flowery, fatty, green olive, leathery, fruit, green, blossom, rose, myrrh, pear, mango, grapefruit, melon, lemon, linalool, mirabelle, brook mint, phenolic, mushroom, metallic, chamomile, apple, carbinol, aromatic, watery, fresh, herb.

## Revendications

1. Composition de substance odoriférante et/ou de substance aromatisante, comprenant un, deux, trois ou plusieurs composés de la formule (I), 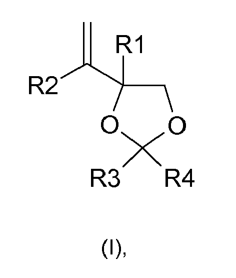 y compris les stéréoisomères, dans laquelle
R1 et R2 sont chacun, indépendamment l'un de l'autre, soit de l'hydrogène soit du méthyle, de préférence de l'hydrogène, et
a) R3 et R4 forment un cycle ayant, dans l'ensemble, 5, 6, 7 ou 8 atomes de carbone cycliques, ledit cycle contenant optionnellement une ou deux liaisons doubles et/ou optionnellement entre 1 et, au maximum, 3 atomes d'oxygène, et ledit cycle étant substitué optionnellement avec un ou plusieurs groupes alkyle, groupes alcényle, groupes cycloalkyle, groupes cycloalcényle, groupes aryle, groupes arylalkyle, groupes alcoxy alkyle ou groupes alcoxy aryle, ramifiés ou non ramifiés, pontés ou non pontés, et dans laquelle R3 et R4 comprennent ensemble 3 à 30 atomes de carbone dans l'ensemble,
ou
b) R3 est soit de l'hydrogène soit un radical organique ayant 1 à 15 atomes de carbone, le radical organique contenant optionnellement entre 1 et, au maximum, 3 atomes d'oxygène, de préférence R3 est soit de l'hydrogène soit un élément structurel saturé ou insaturé, aromatique ou aliphatique, ramifié ou non ramifié, cyclique ou linéaire, ponté ou non ponté, ayant 1 à 15 atomes de carbone, ledit élément structurel contenant optionnellement entre 1 et, au maximum, 3 atomes d'oxygène et ledit élément structurel étant substitué optionnellement avec 1 à, au maximum, 3 groupes alkyle et/ou alcényle et/ou avec un ou deux groupes hydroxyle,
et
c) R4 est un radical organique ayant 2 à 15 atomes de carbones, le radical organique contenant optionnellement entre 1 et, au maximum, 3 atomes d'oxygène, de préférence R4 est un élément structurel saturé ou insaturé, aromatique ou aliphatique, ramifié ou non ramifié, cyclique ou linéaire, ponté ou non ponté, ayant 2 à 15 atomes de carbone, ledit élément structurel contenant optionnellement entre 1 et, au maximum, 3 atomes d'oxygène et ledit élément structurel étant substitué optionnellement avec 1 à, au maximum, 3 groupes alkyle et/ou alcényle et/ou avec un ou deux groupes hydroxyle,
et dans laquelle l'une au moins des conditions suivantes s'applique :
i. si R4 est un radical alkyle ramifié ou non ramifié, ayant 4 à 15 atomes de carbone, avec, optionnellement, une, deux ou plusieurs liaisons doubles, il y a une liaison simple carbone-carbone en position 3 à la position 4 de R4
ou
ii. si R4 est un radical alkyle ramifié ou non ramifié, ayant 2 à 15 atomes de carbone, avec, optionnellement, une, deux ou plusieurs liaisons doubles, R3 n'est pas de l'hydrogène,
et ladite composition de substance odoriférante et/ou de substance aromatisante contenant au moins 3, 4, 5, 6, 7, 8 autres substances odoriférantes et/ou aromatisantes ou plus, dans le cas desquelles il ne s'agit respectivement pas d'un composé de la formule (I),
et ladite composition de substance odoriférante et/ou de substance aromatisante ne contenant pas de 2-hexyl-4-vinyl-1,3-dioxolane.

2. Composition de substance odoriférante et/ou de substance aromatisante selon la revendication 1, dans laquelle
R1 et R2 sont, respectivement indépendamment l'un de l'autre, soit de l'hydrogène soit du méthyle, chacun de préférence de l'hydrogène,
et
a) R3 et R4 forment ensemble un système alkyle cyclique ayant 5, 6, 7 ou 8 atomes de carbone qui est substitué optionnellement avec un ou plusieurs radiaux alkyle ramifiés ou non ramifiés, choisis dans le groupe comprenant le méthyle, l'éthyle, le propyle, le 1-méthyléthyle, le butyle, le 1-méthylpropyle, 2-méthylpropyle, le 1,1-diméthyléthyle, le pentyle, le 1-méthylbutyle, le 2-méthylbutyle, le 3-méthylbutyle, le 2,2-diméthylpropyle, le 1-éthylpropyle, le hexyl, le 1,1-diméthylpropyle, le 1,2-diméthylpropyle, le 1-méthylpentyle, le 2-méthylpentyle, le 3-méthylpentyle, le 4-méthylpentyle, le 1,1-diméthylbutyle, le 1,2-diméthylbutyle, le 1,3-diméthylbutyle, le 2,2-diméthylbutyle, le 2,3-diméthylbutyle, le 3,3-diméthylbutyle, le 1-éthylbutyle, le 2-éthylbutyle, le 1,1,2-triméthylpropyle, le 1,2,2-triméthylpropyle, le 1-éthyl-1-méthylpropyle et le 1-éthyl-2-méthylpropyle, et ledit système alkyle contenant optionnellement une ou deux liaisons doubles,
ou
b) R3 est soit de l'hydrogène soit un radical alkyle ramifié ou non ramifié ayant entre 1 et 15 atomes de carbone et, optionnellement, une, deux ou plusieurs liaisons doubles, ou un système alkyle cyclique ayant 5, 6 ou 7 atomes de carbone avec, optionnellement, une, deux ou plusieurs liaisons doubles, ledit radical alkyle étant choisi de préférence dans le groupe comprenant le méthyl, l'éthyle, le propyle, le 1-méthyléthyle, le butyle, le 1-méthylpropyle, le 2-méthylpropyle, le 1,1-diméthyléthyle, le pentyle, le 1-méthylbutyle, le 2-méthylbutyle, le 3-méthylbutyle, le 2,2-diméthylpropyle, le 1-éthylpropyle, le hexyl, le 1,1-diméthylpropyle, le 1,2-diméthylpropyle, le 1-méthylpentyle, le 2-méthylpentyle, le 3-méthylpentyle, le 4-méthylpentyle, le 1,1-diméthylbutyle, le 1,2-diméthylbutyle, le 1,3-diméthylbutyle, le 2,2-diméthylbutyle, le 2,3-diméthylbutyle, le 3,3-diméthylbutyle, le 1-éthylbutyle, le 2-éthylbutyle, le 1,1,2-triméthylpropyle, le 1,2,2-triméthylpropyle, le 1-éthyl-1-méthylpropyle et le 1-éthyl-2-méthylpropyle,
et
1. R4 est un aromate, ledit aromate étant substitué optionnellement avec un, deux ou plusieurs radicaux alkyle ayant entre 1 et 3 atomes de carbone et/ou un, deux ou plusieurs radicaux hydroxyle et/ou un, deux ou plusieurs radicaux alcoxy,
ou
2. R4 est un système alkyle mono-, bi- ou tricyclique qui est substitué optionnellement avec un, deux ou plusieurs radicaux alkyle, radicaux hydroxyle ou radicaux alcoxy, les radicaux alkyle étant choisis de préférence dans le groupe comprenant le méthyl, l'éthyle, le propyle, le 1-méthyléthyle, le butyle, le 1-méthylpropyle, le 2-méthylpropyle, le 1,1-diméthyléthyle, le pentyle, le 1-méthylbutyle, le 2-méthylbutyle, le 3-méthylbutyle, le 2,2-diméthylpropyle, le 1-éthylpropyle, le hexyl, le 1,1-diméthylpropyle, le 1,2-diméthylpropyle, le 1-méthylpentyle, le 2-méthylpentyle, le 3-méthylpentyle, le 4-méthylpentyle, le 1,1-diméthylbutyle, le 1,2-diméthylbutyle, le 1,3-diméthylbutyle, le 2,2-diméthylbutyle, le 2,3-diméthylbutyle, le 3,3-diméthylbutyle, le 1-éthylbutyle, le 2-éthylbutyle, le 1,1,2-triméthylpropyle, le 1,2,2-triméthylpropyle, le 1-éthyl-1-méthylpropyle et le 1-éthyl-2-méthylpropyle,
ou
3. R4 est un radical alkyle ramifié ou non ramifié ayant entre 2 à 15 atomes de carbone, avec, optionnellement, une, deux ou plusieurs liaisons doubles, ou un système cyclique ayant 5, 6 ou 7 atomes de carbone, avec, optionnellement, une, deux ou plusieurs liaisons doubles.

3. Composition de substance odoriférante et/ou de substance aromatisante selon la revenedication 1 ou 2, **caractérisée par le fait que** ladite composition de substance odoriférante et/ou de substance aromatisante comprend un, deux, trois ou plusieurs composés de la formule (II): 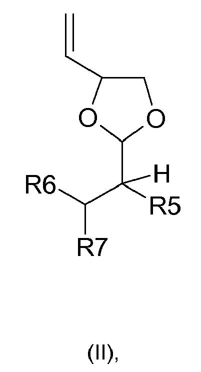 y compris les stéréoisomères, dans laquelle
a) R5 et R6 sont chacun, indépendamment l'un de l'autre, soit de l'hydrogène soit du méthyle,
et
b) R7 est un radical organique ayant 1 à 10 atomes de carbone, le radical organique contenant optionnellement entre 1 et, au maximum, 3 atomes d'oxygène, de préférence R7 est un élément structurel saturé ou insaturé, aromatique ou aliphatique, ramifié ou non ramifié, cyclique ou linéaire, ponté ou non ponté, ayant 1 à 10 atomes de carbone, ledit élément structurel contenant optionnellement entre 1 et, au maximum, 3 atomes d'oxygène et ledit élément structurel étant substitué optionnellement avec 1 à, au maximum, 3 groupes alkyle et/ou alcényle et/ou avec un groupe hydroxyle,
et
dans laquelle, si R7 est un radical alkyle ramifié ou non ramifié, ayant 1 à 10 atomes de carbone, il y a une liaison simple carbone-carbone en position 1 à la position 2 de R7.

4. Composition de substance odoriférante et/ou de substance aromatisante selon la revendication 3, **caractérisée par le fait que** ladite composition de substance odoriférante et/ou de substance aromatisante comprend un, deux, trois ou plusieurs composés de la formule (II), dans laquelle
a) R5 et R6 sont chacun, indépendamment l'un de l'autre, soit de l'hydrogène soit du méthyle,
et
b) R7 est un radical alkyle ramifié ou non ramifié, ayant 1 à 10 atomes de carbone, de préférence choisi dans le groupe comprenant le méthyl, l'éthyle, le propyle, le 1-méthyléthyle, le butyle, le 1-méthylpropyle, le 2-méthylpropyle, le 1,1-diméthyléthyle, le pentyle, le 1-méthylbutyle, le 2-méthylbutyle, le 3-méthylbutyle, le 2,2-diméthylpropyle, le 1-éthylpropyle, le hexyl, le 1,1-diméthylpropyle, le 1,2-diméthylpropyle, le 1-méthylpentyle, le 2-méthylpentyle, le 3-méthylpentyle, le 4-méthylpentyle, le 1,1-diméthylbutyle, le 1,2-diméthylbutyle, le 1,3-diméthylbutyle, le 2,2-diméthylbutyle, le 2,3-diméthylbutyle, le 3,3-diméthylbutyle, le 1-éthylbutyle, le 2-éthylbutyle, le 1,1,2-triméthylpropyle, le 1,2,2-triméthylpropyle, le 1-éthyl-1-méthylpropyle et le 1-éthyl-2-méthylpropyle
ou
R7 est un radical cycloalkyle ayant 5, 6 ou 7 atomes de carbone qui est substitué optionnellement avec un ou plusieurs radicaux alkyle choisis dans le groupe comprenant le méthyl, l'éthyle, le propyle, le 1-méthyléthyle, le butyle, le 1-méthylpropyle, le 2-méthylpropyle, le 1,1-diméthyléthyle, le pentyle, le 1-méthylbutyle, le 2-méthylbutyle, le 3-méthylbutyle, le 2,2-diméthylpropyle, le 1-éthylpropyle, le hexyl, le 1,1-diméthylpropyle, le 1,2-diméthylpropyle, le 1-méthylpentyle, le 2-méthylpentyle, le 3-méthylpentyle, le 4-méthylpentyle, le 1,1-diméthylbutyle, le 1,2-diméthylbutyle, le 1,3-diméthylbutyle, le 2,2-diméthylbutyle, le 2,3-diméthylbutyle, le 3,3-diméthylbutyle, le 1-éthylbutyle, le 2-éthylbutyle, le 1,1,2-triméthylpropyle, le 1,2,2-triméthylpropyle, le 1-éthyl-1-méthylpropyle et le 1-éthyl-2-méthylpropyle, un radical hydroxyle ou un radical alcoxy.

5. Composition de substance odoriférante et/ou de substance aromatisante selon l'une quelconque des revendications précédentes, **caractérisée par le fait que** ladite composition de substance odoriférante et/ou de substance aromatisante comprend un, deux, trois ou plusieurs composés choisis dans le groupe comprenant:
- le 2-isobutyl-4-vinyl-[1,3]dioxolane,
- le 2-secbutyl-4-vinyl-[1,3]dioxolane,
- le 2-isopropyl-4-vinyl-[1,3]dioxolane,
- le 2-(3,5-diméthylcyclohex-3-ényl)-4-vinyl-[1,3]dioxolane,
- le 2-(2,4-diméthylcyclohex-3-ényl)-4-vinyl-[1,3]dioxolane,
- le 2-(2,6-diméthylhept-5-ényl)-4-vinyl-[1,3]dioxolane,
- le 2-éthyl-2-(2-méthylbutyl)-4-vinyl-[1,3]dioxolane,
- le 2-(2,4,4-triméthylpentyl)-4-vinyl-[1,3]dioxolane,
- le 2-(3,5-diméthylhex-4-ényl)-2-méthyl-4-vinyl-[1,3]dioxolane,
- le 2-méthyl-2-(4-méthylpent-3-ényl)-4-vinyl-[1,3]dioxolane,
- le 8-*tert*butyl-2-vinyl-1,4-dioxaspiro-[4.5]-décane,
- le 2-(2,6-diméthylhepta-1,5-diényl-4-vinyl-[1,3]dioxolane,
- le 2-[2-(4-méthylcyclohex-3-ényl)-propyl]-4-vinyl-[1,3]dioxolane,
- le 2-phényl-4-vinyl-[1,3]dioxolane,
- le 2-cyclohexyl-4-vinyl-[1,3]dioxolane,
- le 2-(2,2,3-triméthylcyclopent-3-énylméthyl)-4-vinyl-[1,3]dioxolane,
- le 2-vinyl-1,4-dioxaspiro[4.5]décane,
- le 7-méthyl-2-vinyl-1,4-dioxaspiro[4.5]décane,
- le 2-(2-méthylpropenyl)-4-vinyl-[1,3]dioxolane et
- le 2-méthyl-2-(3-méthylbutyl)-4-vinyl-[1,3]dioxolane.

6. Composition de substance odoriférante et/ou de substance aromatisante selon l'une quelconque des revendications précédentes, **caractérisée par le fait que** ladite composition de substance odoriférante et/ou de substance aromatisante comprend une quantité totale de composés de la formule (I) et/ou (II) comprise entre 0,0001 et 70 % en poids, de préférence entre 0,001 et 50 % en poids et de manière particulièrement préférée entre 0,01 et 20 % en poids, respectivement par rapport à la quantité totale de substances odoriférantes et/ou aromatisantes, ladite composition de substance odoriférante et/ou de substance aromatisante comprenant de préférence une, deux, trois, quatre, cinq ou plusieurs substances odoriférantes et/ou aromatisantes qui confèrent une note olfactive ou gustative fleurie et/ou fruitée.

7. Le 2-(2,6-diméthylhept-5-ényl)-4-vinyl-[1,3]dioxolane.

8. Utilisation d'un composé de la formule (I) 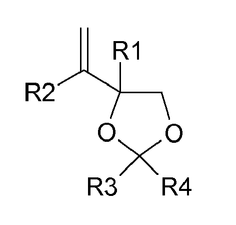 (I), y compris les stéréoisomères,
dans laquelle
R1 et R2 sont chacun, indépendamment l'un de l'autre, soit de l'hydrogène soit du méthyle, de préférence de l'hydrogène, et
a) R3 et R4 forment un cycle ayant, dans l'ensemble, 5, 6, 7 ou 8 atomes de carbone cycliques, ledit cycle contenant optionnellement une ou deux liaisons doubles et/ou optionnellement entre 1 et, au maximum, 3 atomes d'oxygène, et ledit cycle étant substitué optionnellement avec un ou plusieurs groupes alkyle, groupes alcényle, groupes cycloalkyle, groupes cycloalcényle, groupes aryle, groupes arylalkyle, groupes alcoxy alkyle ou groupes alcoxy aryle, ramifiés ou non ramifiés, pontés ou non pontés, et dans laquelle R3 et R4 comprennent ensemble 3 à 30 atomes de carbone dans l'ensemble,
ou
b) R3 est soit de l'hydrogène soit un radical organique ayant 1 à 15 atomes de carbone, le radical organique contenant optionnellement entre 1 et, au maximum, 3 atomes d'oxygène, de préférence R3 est soit de l'hydrogène soit un élément structurel saturé ou insaturé, aromatique ou aliphatique, ramifié ou non ramifié, cyclique ou linéaire, ponté ou non ponté, ayant 1 à 15 atomes de carbone, ledit élément structurel contenant optionnellement entre 1 et, au maximum, 3 atomes d'oxygène et ledit élément structurel étant substitué optionnellement avec 1 à, au maximum, 3 groupes alkyle et/ou alcényle et/ou avec un ou deux groupes hydroxyle,
et
R4 est un radical organique ayant 2 à 15 atomes de carbones, le radical organique contenant optionnellement entre 1 et, au maximum, 3 atomes d'oxygène, de préférence R4 est un élément structurel saturé ou insaturé, aromatique ou aliphatique, ramifié ou non ramifié, cyclique ou linéaire, ponté ou non ponté, ayant 2 à 15 atomes de carbone, ledit élément structurel contenant optionnellement entre 1 et, au maximum, 3 atomes d'oxygène et ledit élément structurel étant substitué optionnellement avec 1 à, au maximum, 3 groupes alkyle et/ou alcényle et/ou avec un ou deux groupes hydroxyle,
et dans laquelle l'une au moins des conditions suivantes s'applique :
i. si R4 est un radical alkyle ramifié ou non ramifié, ayant 4 à 15 atomes de carbone, avec, optionnellement, une, deux ou plusieurs liaisons doubles, il y a une liaison simple carbone-carbone en position 3 à la position 4 de R4
ou
ii. si R4 est un radical alkyle ramifié ou non ramifié, ayant 2 à 15 atomes de carbone, avec, optionnellement, une, deux ou plusieurs liaisons doubles, R3 n'est pas de l'hydrogène,
en tant que substance odoriférante et/ou aromatisante pour conférer, renforcer et/ou modifier une, deux ou plusieurs notes olfactives et/ou gustatives, aucune note olfactive et/ou gustative d'ananas n'étant conférée.

9. Utilisation selon la revendication 8, dans laquelle
R1 et R2 sont, respectivement indépendamment l'un de l'autre, soit de l'hydrogène soit du méthyle, de préférence de l'hydrogène,
et
a) R3 et R4 forment ensemble un système alkyle cyclique ayant 5, 6, 7 ou 8 atomes de carbone, qui est substitué optionnellement avec un ou plusieurs radiaux alkyle ramifiés ou non ramifiés, choisis dans le groupe comprenant le méthyle, l'éthyle, le propyle, le 1-méthyléthyle, le butyle, le 1-méthylpropyle, 2-méthylpropyle, le 1,1-diméthyléthyle, le pentyle, le 1-méthylbutyle, le 2-méthylbutyle, le 3-méthylbutyle, le 2,2-diméthylpropyle, le 1-éthylpropyle, le hexyl, le 1,1-diméthylpropyle, le 1,2-diméthylpropyle, le 1-méthylpentyle, le 2-méthylpentyle, le 3-méthylpentyle, le 4-méthylpentyle, le 1,1-diméthylbutyle, le 1,2-diméthylbutyle, le 1,3-diméthylbutyle, le 2,2-diméthylbutyle, le 2,3-diméthylbutyle, le 3,3-diméthylbutyle, le 1-éthylbutyle, le 2-éthylbutyle, le 1,1,2-triméthylpropyle, le 1,2,2-triméthylpropyle, le 1-éthyl-1-méthylpropyle et le 1-éthyl-2-méthylpropyle, et ledit système alkyle contenant optionnellement une ou deux liaisons doubles,
ou
b) R3 est soit de l'hydrogène soit un radical alkyle ramifié ou non ramifié ayant entre 1 et 15 atomes de carbone et, optionnellement, une, deux ou plusieurs liaisons doubles, ou un système alkyle cyclique ayant 5, 6 ou 7 atomes de carbone avec, optionnellement, une, deux ou plusieurs liaisons doubles, ledit radical alkyle étant choisi de préférence dans le groupe comprenant le méthyl, l'éthyle, le propyle, le 1-méthyléthyle, le butyle, le 1-méthylpropyle, le 2-méthylpropyle, le 1,1-diméthyléthyle, le pentyle, le 1-méthylbutyle, le 2-méthylbutyle, le 3-méthylbutyle, le 2,2-diméthylpropyle, le 1-éthylpropyle, le hexyl, le 1,1-diméthylpropyle, le 1,2-diméthylpropyle, le 1-méthylpentyle, le 2-méthylpentyle, le 3-méthylpentyle, le 4-méthylpentyle, le 1,1-diméthylbutyle, le 1,2-diméthylbutyle, le 1,3-diméthylbutyle, le 2,2-diméthylbutyle, le 2,3-diméthylbutyle, le 3,3-diméthylbutyle, le 1-éthylbutyle, le 2-éthylbutyle, le 1,1,2-triméthylpropyle, le 1,2,2-triméthylpropyle, le 1-éthyl-1-méthylpropyle et le 1-éthyl-2-méthylpropyle,
et
1. R4 est un aromate, ledit aromate étant substitué optionnellement avec un, deux ou plusieurs radicaux alkyle ayant entre 1 et 3 atomes de carbone et/ou un, deux ou plusieurs radicaux hydroxyle et/ou un, deux ou plusieurs radicaux alcoxy,
ou
2. R4 est un système alkyle mono-, bi- ou tricyclique qui est substitué optionnellement avec un, deux ou plusieurs radicaux alkyle, radicaux hydroxyle ou radicaux alcoxy, les radicaux alkyle étant choisis de préférence dans le groupe comprenant le méthyl, l'éthyle, le propyle, le 1-méthyléthyle, le butyle, le 1-méthylpropyle, le 2-méthylpropyle, le 1,1-diméthyléthyle, le pentyle, le 1-méthylbutyle, le 2-méthylbutyle, le 3-méthylbutyle, le 2,2-diméthylpropyle, le 1-éthylpropyle, le hexyl, le 1,1-diméthylpropyle, le 1,2-diméthylpropyle, le 1-méthylpentyle, le 2-méthylpentyle, le 3-méthylpentyle, le 4-méthylpentyle, le 1,1-diméthylbutyle, le 1,2-diméthylbutyle, le 1,3-diméthylbutyle, le 2,2-diméthylbutyle, le 2,3-diméthylbutyle, le 3,3-diméthylbutyle, le 1-éthylbutyle, le 2-éthylbutyle, le 1,1,2-triméthylpropyle, le 1,2,2-triméthylpropyle, le 1-éthyl-1-méthylpropyle et le 1-éthyl-2-méthylpropyle,
ou
3. R4 est un radical alkyle ramifié ou non ramifié, ayant entre 2 à 15 atomes de carbone, avec une, deux ou plusieurs liaisons doubles, ou un système cyclique ayant 5, 6 ou 7 atomes de carbone, avec une, deux ou plusieurs liaisons doubles,
et dans laquelle l'une au moins des conditions suivantes s'applique:
i. si R4 est un radical alkyle ramifié ou non ramifié, ayant 4 à 15 atomes de carbone, avec, optionnellement, une, deux ou plusieurs liaisons doubles, il y a une liaison simple carbone-carbone en position 3 à la position 4 de R4
ou
ii. si R4 est un radical alkyle ramifié ou non ramifié, ayant 2 à 15 atomes de carbone, avec, optionnellement, une, deux ou plusieurs liaisons doubles, R3 est un radical alkyle ramifié ou non ramifié ayant 1 à 15 atomes de carbone et, optionnellement, une, deux ou plusieurs liaisons doubles,
de préférence d'un composé selon la formule (II) 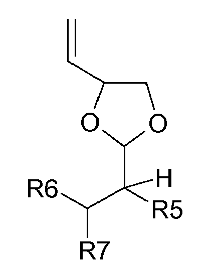 (II), y compris les stéréoisomères,
dans laquelle
a) R5 et R6 sont chacun, indépendamment l'un de l'autre, soit de l'hydrogène soit du méthyle,
et
b) R7 est un radical alkyle ramifié ou non ramifié, ayant 1 à 10 atomes de carbone, de préférence choisi dans le groupe comprenant le méthyl, l'éthyle, le propyle, le 1-méthyléthyle, le butyle, le 1-méthylpropyle, le 2-méthylpropyle, le 1,1-diméthyléthyle, le pentyle, le 1-méthylbutyle, le 2-méthylbutyle, le 3-méthylbutyle, le 2,2-diméthylpropyle, le 1-éthylpropyle, le hexyl, le 1,1-diméthylpropyle, le 1,2-diméthylpropyle, le 1-méthylpentyle, le 2-méthylpentyle, le 3-méthylpentyle, le 4-méthylpentyle, le 1,1-diméthylbutyle, le 1,2-diméthylbutyle, le 1,3-diméthylbutyle, le 2,2-diméthylbutyle, le 2,3-diméthylbutyle, le 3,3-diméthylbutyle, le 1-éthylbutyle, le 2-éthylbutyle, le 1,1,2-triméthylpropyle, le 1,2,2-triméthylpropyle, le 1-éthyl-1-méthylpropyle et le 1-éthyl-2-méthylpropyle,
ou
R7 est un radical cycloalkyle ayant 5, 6 ou 7 atomes de carbone qui est substitué optionnellement avec un ou plusieurs radicaux alkyle choisis dans le groupe comprenant le méthyl, l'éthyle, le propyle, le 1-méthyléthyle, le butyle, le 1-méthylpropyle, le 2-méthylpropyle, le 1,1-diméthyléthyle, le pentyle, le 1-méthylbutyle, le 2-méthylbutyle, le 3-méthylbutyle, le 2,2-diméthylpropyle, le 1-éthylpropyle, le hexyl, le 1,1-diméthylpropyle, le 1,2-diméthylpropyle, le 1-méthylpentyle, le 2-méthylpentyle, le 3-méthylpentyle, le 4-méthylpentyle, le 1,1-diméthylbutyle, le 1,2-diméthylbutyle, le 1,3-diméthylbutyle, le 2,2-diméthylbutyle, le 2,3-diméthylbutyle, le 3,3-diméthylbutyle, le 1-éthylbutyle, le 2-éthylbutyle, le 1,1,2-triméthylpropyle, le 1,2,2-triméthylpropyle, le 1-éthyl-1-méthylpropyle et le 1-éthyl-2-méthylpropyle, un radical hydroxyle ou un radical alcoxy,
dans laquelle, si R7 est un radical alkyle ramifié ou non ramifié, ayant 1 à 10 atomes de carbone, il y a une liaison simple carbone-carbone en position 1 à la position 2 de R7.

10. Utilisation selon la revendication 8 ou 9, dans laquelle le composé est choisi dans le groupe comprenant:
- le 2-isobutyl-4-vinyl-[1,3]dioxolane,
- le 2-secbutyl-4-vinyl-[1,3]dioxolane,
- le 2-isopropyl-4-vinyl-[1,3]dioxolane,
- le 2-(3,5-diméthylcyclohex-3-ényl)-4-vinyl-[1,3]dioxolane,
- le 2-(2,4-diméthylcyclohex-3-ényl)-4-vinyl-[1,3]dioxolane,
- le 2-(2,6-diméthylhept-5-ényl)-4-vinyl-[1,3]dioxolane,
- le 2-éthyl-2-(2-méthylbutyl)-4-vinyl-[1,3]dioxolane,
- le 2-(2,4,4-triméthylpentyl)-4-vinyl-[1,3]dioxolane,
- le 2-(3,5-diméthylhex-4-ényl)-2-méthyl-4-vinyl-[1,3]dioxolane,
- le 2-méthyl-2-(4-méthylpent-3-ényl)-4-vinyl-[1,3]dioxolane,
- le 8-*tert*butyl-2-vinyl-1,4-dioxaspiro-[4.5]-décane,
- le 2-(2,6-diméthylhepta-1,5-diényl-4-vinyl-[1,3]dioxolane,
- le 2-[2-(4-méthylcyclohex-3-ényl)-propyl]-4-vinyl-[1,3]dioxolane,
- le 2-phényl-4-vinyl-[1,3]dioxolane,
- le 2-cyclohexyl-4-vinyl-[1,3]dioxolane,
- le 2-(2,2,3-triméthylcyclopent-3-énylméthyl)-4-vinyl-[1,3]dioxolane,
- le 2-vinyl-1,4-dioxaspiro[4.5]décane,
- le 7-méthyl-2-vinyl-1,4-dioxaspiro[4.5]décane,
- le 2-(2-méthylpropenyl)-4-vinyl-[1,3]dioxolane et
- le 2-méthyl-2-(3-méthylbutyl)-4-vinyl-[1,3]dioxolane.

11. Utilisation selon l'une quelconque des revendications 8 à 10, pour conférer, renforcer et/ou modifier une, deux, trois ou plusieurs parmi les notes olfactives et/ou gustatives qui sont : fleurie, grasse, olive verte, de cuir, fruit, vert, fleur, rose, myrrhe, poire, mangue, pamplemousse, melon, citron, linalol, mirabelle, menthe crépue, phénolique, champignon, métallique, camomille, pomme, carbinol, aromatique, aqueuse, fraîche, chou.

12. Article parfumé et/ou aromatisé comprenant une composition de substance odoriférante et/ou de substance aromatisante selon l'une quelconque des revendications 1 à 5 et/ou un composé selon la revendication 6 ou 7,
ledit article parfumé étant choisi de préférence dans le groupe comprenant les lessives, les produits d'hygiène ou de soins, en particulier dans le domaine de l'hygiène corporelle et des soins capillaires, des soins de beauté et du ménage, et ledit article aromatisé étant choisi de préférence dans le groupe comprenant les denrées alimentaires, les produits de consommation de luxe, les boissons, les produits de l'hygiène buccale ou les produits pharmaceutiques.

13. Procédé pour conférer, modifier et/ou renforcer une odeur et/ou un goût sans conférer l'odeur et/ou le goût de la note d'ananas, une quantité sensoriellement efficace d'une composition de substance odoriférante et/ou de substance aromatisante selon
l'une quelconque des revendications 1 à 6
ou
- d'un composé selon la revendication 7 étant mise en contact avec ou mélangée à un produit,
de préférence pour conférer, modifier et/ou renforcer une, deux ou plusieurs parmi les notes olfactives et/ou gustatives qui sont : fleurie, grasse, olive verte, de cuir, fruit, vert, fleur, rose, myrrhe, poire, mangue, pamplemousse, melon, citron, linalol, mirabelle, menthe crépue, phénolique, champignon, métallique, camomille, pomme, carbinol, aromatique, aqueuse, fraîche, chou.
